(19)

Europäisches
Patentamt
European
Patent Office
Office européen
des brevets

(11) **EP 4 759 361 A2**

(12) **EUROPEAN PATENT APPLICATION**

(43) Date of publication:
**17.06.2026 Bulletin 2026/25**

(51) International Patent Classification (IPC):
***A61P 7/02*** (2006.01)

(21) Application number: 26160707.1

(22) Date of filing: **22.12.2017**

(52) Cooperative Patent Classification (CPC):
**C07K 16/36; A61P 7/02;** A61K 2039/505;
A61K 2039/545; C07K 2317/33; C07K 2317/76;
C07K 2317/92

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.12.2016 US 201662438654 P
22.11.2017 US 201762589851 P**

(62) Document number(s) of the earlier application(s) in
accordance with Art. 76 EPC:
**17832998.3 / 3 558 370**

(71) Applicant: **Novartis AG
4056 Basel (CH)**

(72) Inventors:
• **FRIEDMAN, Carola
East Hanover, NJ 07936 (US)**
• **KHDER, Yasser
4002 Basel (CH)**

• **LEFKOWITZ, Martin
East Hanover, NJ 07936 (US)**

(74) Representative: **Mathys & Squire
32 London Bridge Street
The Shard
London SE1 9SG (GB)**

Remarks:
• The complete document including Reference
Table(s) and the Sequence Listing(s) can be
downloaded from the EPO website
• This application was filed on 25-02-2026 as a
divisional application to the application mentioned
under INID code 62.
• Claims filed after the date of receipt of the divisional
application (Rule 68(4) EPC).

(54) **METHODS OF TREATMENT WITH ANTI-FACTOR XI/XIA ANTIBODIES**

(57) The present invention relates to methods of preventing, treating or managing, reducing the risk of stroke or thromboembolism in a subject afflicted with end stage renal disease comprising administering a pharmaceutical composition comprising a monoclonal antibody or an antigen binding fragment thereof that bind to human Factor XI and/or activated Factor XI ("Factor XIa").

**EP 4 759 361 A2**

## Description

This application claims the benefit of U.S. Provisional Application No. 62/438,654 filed on December 23, 2016 and U.S. Provisional Application No. 62/589,851 filed on November 22, 2017, each of which is hereby incorporated by reference in its entirety.

SEQUENCE LISTING

The instant application contains a Sequence Listing which has been submitted electronically in ASCII format and is hereby incorporated by reference in its entirety. Said ASCII copy, created on November 30, 2017, is named PAT057550-WO-PCT_SL.txt and is 45,591 bytes in size.

BACKGROUND

Thrombosis refers to thrombus formation inside blood vessels, subsequent to a combination of hereditary and acquired risk factors, known as thrombophilia or hypercoagulable states. Vessel wall damage, stasis, increased platelets reactivity and activation of clotting factors are some of the fundamental features of thrombosis. Thrombosis can occur in both venous and arterial circulation and can result in the development of deep vein thrombosis (DVT), pulmonary embolism, and stroke. If a thrombus occurs in the arterial system, down-stream ischemia can occur, leading to acute coronary syndromes (ACS), ischemic stroke, and acute limb ischemia. Thrombus formation in the venous system typically leads to deep venous thrombosis, pulmonary embolism and chronic thromboembolic pulmonary hypertension. Clots may also form in the left atrial appendage in patients with AF (AF), and dislodged thrombi may result in potentially devastating complications, *i.e.* thromboembolic stroke and systemic embolism. The currently available antithrombotic medications, including low molecular weight heparin (LMWH), thrombin inhibitors, and Factor Xa (FXa) inhibitors, are all associated with a significant risk of bleeding (Weitz J.I. (2010) Thromb. Haemost. 103, 62). The development of an antithrombotic agent that does not affect hemostasis, and therefore does not result in bleeding complications, would be highly desirable.

Current anticoagulants are either injected or taken orally. The injectable anticoagulant LMWH is widely used and offers an improved therapeutic profile over formerly applied unfractionated heparin. For the past few decades the most commonly used oral anticoagulant has been warfarin. Warfarin has a narrow therapeutic window that requires frequent monitoring of the coagulation status, and shows a variety of drug-drug interactions. More recently, orally available direct FXa and thrombin inhibitors entered the anticoagulant market and are increasingly applied.

LMWHs, FXa inhibitors, and thrombin inhibitors are all efficacious in the prevention of post-operative venous thromboembolic disease, in the treatment of spontaneous DVT and pulmonary embolism, and in the stroke prevention in atrial fibrillation. However, these anticoagulants are also associated with bleeding complications that were generally comparable to those observed with the older drugs warfarin and unfractionated heparin. In the ADVANCE-2 clinical trial, the FXa inhibitor apixaban (Eliquis) was compared to the LMWH enoxaparin in patients after total knee replacement. While acute apixaban therapy was more effective at preventing venous thromboembolic disease than enoxaparin, both agents were associated with a significant risk of bleeding. Clinically relevant bleeding occurred in 4% of patients receiving apixaban and in 5% of patients treated with enoxaparin (Lassen, M.R., et al. (2009) N. Engl. J. Med. 361, 594).

In the RE-LY trial, the direct thrombin inhibitor dabigatran (Pradaxa) was compared to warfarin in patients with AF and a risk of stroke (Connolly, S.J., et al. (2009) N. Engl. J. Med. 361, 1139). Chronic dabigatran therapy was associated with a significantly lower risk of stroke or systemic embolism. However, major bleeding complications occurred in 3.1% of patients receiving 150 mg per day of dabigatran and in 3.4% of patients receiving warfarin (p=0.31).

Atrial fibrillation (AF) remains the most common cardiac arrhythmia in clinical practice, accounting for approximately one third of hospitalizations for cardiac dysrhythmias. Currently, it is estimated to affect more than 6 million patients in Europe and approximately 2.3 million in the United States, and this number continues to grow rapidly because of the increasing proportion of the aging population. It is estimated that approximately 5% of the population over the age of 65 years, and 10% of people aged over 80 years, will develop AF, however, the prevalence of AF is increasing beyond what is explained by age alone. AF risk factors such as hypertension, congestive heart failure, left ventricular hypertrophy, coronary artery disease and diabetes mellitus, and obstructive sleep apnea are also on the rise. As such, the number of affected individuals with AF is expected to increase two to three times over the next three decades in western populations. (Kannel and Benjamin (2008) Med Clin North Am. 2008; 92:17-40; Bunch, et al. (2012) J Innovations of Card Rhythm Manag 2012; 3: 855-63).

The principal risk of AF is a four- to five fold increase in embolic stroke. The attributable risk for stroke associated with AF increases steeply with age to 23.5% at ages 80 to 89. AF is associated with a doubling of mortality in both genders (Kannel and Benjamin 2008). AF is also independently associated with cognitive decline and all forms of dementia (Marzona, et al. (2012) CMAJ 2012; 184: 329-36; Geita et al 2013; Bunch et al 2012).

Most patients with AF require life-long anticoagulation therapy to prevent cardioembolic stroke and systemic

embolism. The $CHA_2DS_2$-VASc risk score is a validated and widely used stratification tool to predict thromboembolic risk in AF patients and to identify patients who should benefit from anticoagulation therapy (LIP 2011; Camm, et al. (2012) Eur Heart J 2012; 33: 2719-2747); the accumulated evidence shows that $CHA_2DS_2$-VASc is at least as accurate as or possibly better than, scores such as CHADS2 in identifying patients who develop stroke and thromboembolism and definitively better at identifying 'truly low-risk' patients with AF. It is estimated that 85 to 90% of AF patients will require anticoagulation therapy.

In the recent years new oral anticoagulants (NOAC) also referred to as direct oral anticoagulants (DOAC) have been approved and introduced to clinical practice. These drugs are at least as effective or even better than warfarin for reducing thrombo-embolic disease (Connolly, et al. (2009) N Engl J Med; 361:1139-51; Connolly, et al. (2011) N Engl J Med; 364:806-17; Patel, et al. (2011) N Engl J Med 2011; 365:883-91). NOAC were also associated with large reductions in the most devastating complications of warfarin namely hemorrhagic stroke and intracranial hemorrhage. Major bleeding events were similar or slightly lower than well conducted warfarin therapy. In addition NOAC are associated with a lower potential for drug-drug interaction than warfarin and could be used without routine monitoring; this is expected to ease their use in everyday medical practice.

Despite recent improvements, bleeding risk continues to be high with the use of anticoagulants. For instance, the annual incidence of major and clinically relevant non major bleeding was 14.9% and the annual incidence of major bleeding events was 3.6% in patients treated with rivaroxaban in the ROCKET study (Patel et al 2011). The annual incidence of major bleeding was > 5% in patients at a high risk for bleeding defined as HAS Bled risk score $\geq$ 3 (Gallego, et al. (2012) Carc Arrhythm Electrophysiol.; 5:312-318). Major bleeding is a particularly relevant clinical outcome; for instance in the ROCKET study, once major bleeding has occurred, all-cause mortality rate was 20.4% in the rivaroxaban group and 26.1% in the warfarin group. Once major bleeding events have occurred stroke and systemic embolism occurred in 4.7% and 5.4% of patients in rivaroxaban and warfarin groups, respectively (Piccini, et al. (2014) Eur Heart J; 35:1873-80). Hospital stay, transfusion of blood products and resources utilization were also severely impacted by the occurrence of major bleeding. Bleeding risk is also a major reason for not receiving anticoagulants in eligible patients. In the Euro Heart Survey on AF comprising data from 182 hospitals in 35 countries and 5333 ambulant and hospitalized AF patients, only 67% of eligible patients received oral anticoagulant at discharge (Nieuwlaat, et al. (2005) Eur Heart J;26, 2422-2434). A high unmet medical need therefore exists for a safer therapy which can reduce AF thromboembolic complications such as stroke, systemic embolism, cognitive decline and mortality with comparable efficacy as existing therapy but with a lower bleeding liability.

In the setting of end-stage renal disease (ESRD), the incidence and risk for thrombotic events are increased and use of anticoagulants is common, in particular in ESRD patients undergoing hemodialysis. The incidence of bleeding, however, is also a frequent issue and creates additional challenges in the management of anticoagulation therapy.

Moreover, AF is a highly prevalent comorbidity in patients with ESRD (Zimmerman et al. (2012) Nephrol Dial Transplant, 27: 3816-3822). For example, AF is associated with a higher stroke risk and mortality in patients with ESRD (Zimmerman et al. (2012) Nephrol Dial Transplant, 27: 3816-3822)). Hence a high unmet medical need for a safe anticoagulant with a non-renal clearance exists in patients with ESRD, and in particular, AF and ESRD undergoing dialysis.


SUMMARY

The present disclosure relates to methods of preventing, treating or managing, or reducing the risk of thrombosis or thromboembolism associated with treatment of subjects afflicted with end stage renal disease (ESRD). The methods include administering to the subject a monoclonal antibody or antigen binding fragment thereof that binds to human coagulation Factor XI and XIa (activated Factor XI) (hereinafter, sometimes referred to as "FXI", "FXIa," and similar terms). The development of an anti-thrombotic agent that is efficacious in the prevention and treatment of thrombosis or thromboembolic disease/disorder associated with treatment of end stage renal disease (e.g., thrombic stroke, AF, stroke prevention in AF (SPAF), deep vein thrombosis, venous thromboembolism, pulmonary embolism, ischemic stroke, acute limb ischemia, chronic thromboembolic pulmonary hypertension, systemic embolism) but carries no or only minimal bleeding risk would meet a sizable unmet medical need.

In some embodiments, the subject is receiving dialysis. For example, the subject has end stage renal disease and AF (e.g., non-valvular AF) and is receiving dialysis.

In particular aspects, the present disclosure relates to methods of preventing, treating or managing, or reducing the risk of thrombosis or thromboembolism in subjects with end stage renal disease (ESRD) and AF (e.g., non-valvular AF) undergoing dialysis, said method comprises administering to subjects an anti-FXI/FXIa antibody (e.g., those described in Table 1).

In some embodiments, the subject is obese.

In specific aspects, the subject is administered an antibody or fragment (e.g., human, chimeric, humanized monoclonal antibody or fragment) provided herein that binds with similarly high affinity to the catalytic domain (CD) of human FXIa and FXI and induces an inactive protease domain conformation in FXIa.

In some embodiments, the antibody, or antigen binding fragments thereof, for use in the methods described herein, binds within the catalytic domain of FXI and/or FXIa, specifically to the surface of the active site region.

The methods described herein can comprise administering an antibody, or antigen binding fragment thereof, that binds FXI and/or FXIa and competes for binding with an antibody described in Table 1 (*e.g.,* NOV1401). As described here, "competition" between antibodies and/or antigen binding fragments thereof signifies that both antibodies (or binding fragments thereof) bind to the same, or overlapping, FXI and/or FXIa epitope (*e.g.,* as determined by a competitive binding assay, by any of the methods well known to those of skill in the art). As used herein, an antibody or antigen binding fragment thereof does not "compete" with an FXI and/or FXIa antibody or antigen binding fragment of the present disclosure (*e.g.,* NOV1401 or NOV1090) unless said competing antibody or antigen binding fragment thereof binds the same FXI and/or FXIa epitope, or an overlapping FXI and/or FXIa epitope, as an antibody or antigen binding fragment of the present disclosure. As used herein, a competing antibody or antigen binding fragment thereof does not include one which (i) sterically blocks an antibody or antigen binding fragment of the present disclosure from binding its target (*e.g.,* if said competing antibody binds to a nearby, non-overlapping FXI and/or FXIa epitope and physically prevents an antibody or antigen binding fragment of the present disclosure from binding its target); and/or (ii) binds to a different, non-overlapping FXI and/or FXIa epitope and induces a conformational change to the FXI and/or FXIa protein such that said protein can no longer be bound by an FXI and/or FXIa antibody or antigen binding fragment of the present disclosure in a way that would occur absent said conformational change.

In one embodiment, the method comrpises administering an antibody, or antigen binding fragment thereof, that binds to active FXI (FXIa) and leads upon binding to the active FXI (FXIa) catalytic domain to FXIa changing its conformation to an inactive conformation. In another embodiment, the antibody or antigen binding fragment thereof further induce a change in which the N-terminal 4 residues, loops 145, 188 and 220 of the inactive conformation are shifted and/or disordered compared to the active conformation.

In one embodiment, the method comprises administering an antibody, or antigen binding fragment thereof, that binds to FXI (*e.g.*, human FXI) and upon binding to FXI prevents the FXI catalytic domain from assuming an active conformation, in which loops 145, 188 and 220 are ordered as in the structure of the FXIa catalytic domain.

In one embodiment, the method comprises administering an antibody, or antigen binding fragment thereof, that binds to FXI and, upon binding to FXI, prevents the FXI catalytic domain from assuming an active conformation, in which the N-terminal 4 residues, loops 145, 188 and 220 are ordered as in the structure of the FXIa catalytic domain.

In one embodiment, the method comprises administering an antibody, or antigen binding fragment thereof, that binds to FXI and, upon binding to FXI, prevents the FXI catalytic domain from assuming an active conformation by inducing conformational changes in the zymogen structure, further leading to an inhibited FXI conformation closely related to that observed when binding to FXIa.

In one embodiment, the method comprises administering an antibody, or antigen binding fragment thereof, that binds to FXI and prevents the catalytic domain from assuming an active conformation by inducing a conformational changes in the zymogen structure, thereby leading to an inhibited FXI conformation closely related to that observed when binding to FXIa.

The methods described herein can comprise administering an antibody, or antigen binding fragment thereof, that binds the same epitope as an antibody as described in Table 1 (e.g., NOV1401).

The anti-FXI and/or FXIa antibody or antigen binding fragment used in the methods described can inhibit the direct or indirect activation of Factor IX (also known as FIX), Factor X (FX), and/or thrombin with an $IC_{50}$ of less than or equal to 100 nM, less than or equal to 50 nM, less than or equal to 35 nM, less than or equal to 25 nM, less than or equal to 10 nM, or less than or equal to 5.2 nM. More specifically, the antibody or antigen binding fragment thereof can inhibit the direct or indirect activation of Factor IX (also known as FIX), Factor X (FX), and/or thrombin with an $IC_{50}$ of less than or equal to 100 nM, less than or equal to 50 nM, less than or equal to 35 nM, less than or equal to 25 nM, less than or equal to 10 nM, or less than or equal to 5.2 nM. More specifically, the antibody or antigen binding fragment thereof can inhibit the direct or indirect activation of Factor IX (also known as FIX), Factor X (FX), and/or thrombin with an $IC_{50}$ of less than or equal to 100 nM, less than or equal to 50 nM, less than or equal to 35 nM, less than or equal to 25 nM, less than or equal to 20 nM, or less than or equal to 18 nM. More specifically, the antibody or antigen binding fragment thereof can inhibit the direct or indirect activation of Factor IX (also known as FIX), Factor X (FX), and/or thrombin with an $IC_{50}$ of less than or equal to 100 nM, less than or equal to 50 nM, less than or equal to 35 nM, less than or equal to 25 nM, less than or equal to 10 nM, or less than or equal to 5 nM. In a specific embodiment, the anti-FXI/FXIa antibody, or antigen binding fragment thereof, inhibits FXIa-mediated activation of its native substrate FIX with an $IC_{50}$ of less than or equal to 2 nM, *e.g.,* 1.8 nM.

In some embodiments, the method comprises administering an antibody, or antigen binding fragment thereof, that prolongs the clotting time (*e.g.,* time until a blood clot starts to form) of human plasma in a concentration-dependent manner as determined by an aPTT assay, for example as described in the Examples Section. In a specific embodiment, clotting time (aPTT) is doubled compared to baseline at a total anti-FXI antibody (*e.g.,* NOV1401) concentration in the range of 10 nM to 20 nM, for example approximately 14 nM or 15 nM, as determined by an aPTT assay. In particular embodiments, the antibody, or antigen binding fragment thereof, prolongs the clotting time of human plasma in a concentration-dependent manner with an IC50 in the range of 5 nM to 20 nM, for example approximately 13 nM, as determined by the aPTT assay, for example as described in the Examples Section.

In some embodiments, the method comprises administering an antibody, or antigen binding fragment thereof, that prolongs the clotting time (*e.g.,* time until a blood clot starts to form) of human plasma by at least 1.1 fold, 1.2 fold, 1.3 fold, 1.4 fold, 1.5 fold, 1.6 fold, 1.7 fold, 1.8 fold, 1.9 fold, or 2 fold, e.g., in a concentration-dependent manner, as determined by an aPTT assay, for example as described in the Examples Section. In specific embodiments, the antibody, or antigen binding fragment thereof, prolongs the clotting time (*e.g.,* time until a blood clot starts to form) of human plasma by at least 1.4 fold, 1.5 fold, 1.6 fold, or 1.7 fold, as determined by an aPTT assay, for example as described in the Examples Section.

In some embodiments, the method comprises administering an antibody, or antigen binding fragment thereof, that reduces the amount of thrombin, in a concentration-dependent manner, in a thrombin generation assay (TGA) in human plasma, which measures the effect of FXIa inhibition on the thrombin→FXIa feed-forward loop in the presence of very low tissue factor (TF) concentrations. In particular embodiments, the antibody, or antigen binding fragment thereof, reduces the amount of thrombin in a thrombin generation assay (TGA) in human plasma with an $IC_{50}$ value in the range of 10 nM to 30 nM, for example approximately 20 nM or 24 nM, and a residual thrombin concentration of approximately 159 nM.

In some embodiments, the method comprises administering an antibody (e.g., an antibody in Table 1 such as NOV1401 or an antibody comprising the HCDRs 1-3 and LCDRs 1-3 of NOV1401), or antigen binding fragment thereof, which specifically binds to the catalytic domain of human FXI and/or FXIa, and which has a terminal elimination half-life ($t_{1/2}$) of total antibodies in cynomolgus monkeys as approximately 14-15 days. In specific embodiments, the antibody or antigen binding fragment thereof exhibits an absolute subcutaneous (s.c.) bioavailability of approximately 61-66%.

In one embodiment, the method comprises administering an antibody or antigen binding fragment thereof (*e.g.,* an antibody described in Table 1, such as NOV1401), which specifically binds to human FXI and/or FXIa, and exhibits one or more (*e.g.,* two, or three, or four, or five, or six, or seven), or all, of the following characteristics:

(i) specifically binds to a catalytic domain (CD) of human FXI and FXIa, for example, with an apparent $K_D$ of approximately 1-2 pM and 4-5 pM respectively;

(ii) prolongs clotting time as evaluated by activated partial thromboplastin time (aPTT) assay;

(iii) inhibits thrombin generation in human plasma through inhibition of FXI activation by activated factor XII (FXIIa) and by thrombin, respectively;

(iv) shows antithrombotic and anticoagulant activity in FXI-/- mice reconstituted with human FXI;

(v) reduces or prolongs the reduction of free FXI ($FXI_f$) levels, for example, in cynomolgus monkeys;

(vi) has a terminal elimination half-life of total antibody of approximately 14-15 days, for example, in cynomolgus monkeys;

(vii) specifically binds to human and monkey FXI and/or FXIa but does not specifically bind to mouse or rat FXI and/or FXIa; and

(viii) contacts one or more (*e.g.,* two, three, four, five, six, or seven, or more), or some, or all, of the following residues of human FXI (Swissprot numbering): Pro410, Arg413, Leu415, Cys416, His431, Cys432, Tyr434, Gly435, Glu437, Tyr472-Glu476, Tyr521-Lys527, Arg548, His552, Ser575, Ser594-Glu597, and Arg602-Arg604.

The antibody, or antigen binding fragment thereof, used in the methods described herein can be a monoclonal antibody, a human or humanized antibody, a chimeric antibody, a single chain antibody, a Fab fragment, a Fv fragment, a F(ab')2 fragment, or a scFv fragment, and/or an IgG isotype (*e.g.,* IgG1 such as human IgG1). In specific embodiments, the antibody can be a recombinant human antibody. In specific embodiments, the antibody is a human IgG1/lambda (A) antibody. In specific embodiments, the antibody is a human IgG1/lambda (A) antibodies comprising an Fc domain engineered to reduce the potential for effector function (e.g., ADCC and/or CDC), for example a human Fc domain comprising D265A and/or P329A substitutions.

The antibody, or antigen binding fragment thereof, for use in the methods described herein can also include a framework in which an amino acid has been substituted into the antibody framework from the respective human VH or VL germline sequences.

The methods described herein can comrpise administering an antibody or antigen binding fragment thereof having the heavy and light chain sequences of an antibody described in Table 1. More specifically, the antibody or antigen binding fragment thereof can have the heavy and light chain sequences of NOV1090 or NOV1401.

The methods described herein can comrpise administering an antibody or antigen binding fragment thereof having the heavy and light chain variable domain sequences of Fabs described in Table 1. More specifically, the isolated antibody or antigen binding fragment thereof can have the heavy and light chain variable domain sequence of NOV1090 and NOV1401.

The methods described herein can comrpise administering an antibody or antigen binding fragment thereof comprising the heavy chain variable domain CDR (*i.e.,* HCDR1, HCDR2, and HCDR3) and light chain variable domain CDR (*i.e.,* LCDR1, LCDR2, and LCDR3) sequences of antibodies described in Table 1, such as Kabat CDRs, IMGT CDRs, Chothia CDRs, or combined CDRs. More specifically, the isolated antibody or antigen binding fragment thereof can have the HCDR1, HCDR2, HCDR3, LCDR1, LCDR2, and LCDR3 sequences of NOV1090 and NOV1401, for example as presented in Table 1, such as Kabat CDRs, IMGT CDRs, Chothia CDRs, or combined CDRs.

In specific aspects, the methods described herein can comrpise administering an antibody or antigen binding fragment thereof comprising HCDR1, HCDR2, and HCDR3 of the variable heavy chain of SEQ ID NOs: 9 and 29, and the LCDR1, LCDR2 and LCDR3 of the variable light chain of SEQ ID NOs: 19 and 39, as defined by Chothia. In another aspect of the present disclosure the antibody or antigen binding fragment may have the HCDR1, HCDR2, and HCDR3 of the heavy chain variable domain sequence of SEQ ID NOs: 9 and 29, and the LCDR1, LCDR2 and LCDR3 of the light chain variable domain sequence of SEQ ID NOs: 19 and 39, as defined by Kabat.

In specific aspects, the methods described herein can comrpise administering an antibody or antigen binding fragment thereof comprising HCDR1, HCDR2, and HCDR3 of the variable heavy chain of SEQ ID NOs: 9 and 29, and the LCDR1, LCDR2 and LCDR3 of the variable light chain of SEQ ID NOs: 19 and 39, as defined by IMGT. In another aspect of the present disclosure the antibody or antigen binding fragment may have the HCDR1, HCDR2, and HCDR3 of the heavy chain variable domain sequence of SEQ ID NOs: 9 and 29, and the LCDR1, LCDR2 and LCDR3 of the light chain variable domain sequence of SEQ ID NOs: 19 and 39, as defined by Combined.

In specific aspects, the methods described herein comrpise administering an antibody or antigen binding fragment thereof that includes a heavy chain variable domain sequence selected from the group consisting of SEQ ID NOs: 9 and 29. The isolated antibody or antigen binding fragment further can comprise a light chain variable domain sequence wherein the heavy chain variable domain and light chain variable domain combine to form an antigen binding site for FXIa. In particular the light chain variable domain sequence can be selected from SEQ ID NOs: 19 and 39 wherein said isolated antibody or antigen binding fragments thereof binds FXI and/or FXIa.

In specific aspects, the methods described herein comrpise administering an antibody or antigen binding fragment thereof that includes a light chain variable domain sequence selected from the group consisting of SEQ ID NOs: 19 and 39, wherein said isolated antibody or antigen binding fragments thereof binds to human FXI and/or FXIa. The isolated antibody or antigen binding fragment may further comprise a heavy chain variable domain sequence wherein the light chain variable domain and heavy chain variable domain combine to form and antigen binding site for FXI and/or FXIa.

In specific aspects, the methods described herein comrpise administering an antibody or antigen binding fragment thereof that binds FXI and/or FXIa, may have heavy and light chain variable domains comprising the sequences of SEQ ID NOs: 9 and 19; or 19 and 39, respectively.

In specific aspects, the methods described herein comrpise administering an antibody or antigen binding fragment thereof that includes a heavy chain variable domain having at least 80%, 85%, 90%, 95%, 97%, 98%, or 99% sequence identity to a sequence selected from the group consisting of SEQ ID NOs: 9 and 29, wherein said antibody binds to FXI and/or FXIa. In one aspect, the isolated antibody or antigen binding fragments thereof also includes a light chain variable domain having at least 80%, 85%, 90%, 95%, 97%, 98%, or 99% sequence identity to a sequence selected from the group consisting of SEQ ID NOs: 19 and 39. In a further aspect of the present disclosure, the isolated antibody or antigen binding fragment has an HCDR1, HCDR2, HCDR3, LCDR1, LCDR2 and LCDR3 as defined by Kabat and as described in Table 1. In a specific embodiment, the isolated antibody or antigen binding fragment has an HCDR1, HCDR2, HCDR3, LCDR1, LCDR2 and LCDR3 as defined by Chothia, IMGT, or Combined and as described in Table 1.

In specific aspects, the methods described herein comrpise administering an antibody or antigen binding fragment thereof having a light chain variable domain having at least 80%, 85%, 90%, 95%, 97%, 98%, or 99% sequence identity to a sequence selected from the group consisting of SEQ ID NOs: 19 and 39 , wherein said antibody binds FXI and/or FXIa.

In specific aspects, the methods described herein comrpise administering an antibody or antigen binding fragment thereof that bind to FXI and/or FXIa may have a heavy chain comprising the sequence of SEQ ID NOs: 11 or 31. The isolated antibody can also include a light chain that can combine with the heavy chain to form an antigen binding site to human FXI and/or FXIa. In particular, the light chain may have a sequence comprising SEQ ID NOs: 21 or 41. In particular, the isolated antibody or antigen binding fragments thereof that binds FXI and/or FXIa, may have a heavy chain and a light chain comprising the sequences of SEQ ID NOs: 11 and 21; or 31 and 41, respectively.

In specific aspects, the methods described herein comrpise administering an antibody or antigen binding fragment thereof that includes a heavy chain having at least 90% sequence identity to a sequence selected from the group consisting of SEQ ID NOs: 11 or 31, wherein said antibody binds to FXI and/or FXIa. In one aspect, the isolated antibody or antigen binding fragments thereof also includes a light chain having at least 80%, 85%, 90%, 95%, 97%, 98%, or 99% sequence identity to a sequence selected from the group consisting of SEQ ID NOs: 21 or 41.

In specific aspects, the methods described herein comrpise administering an antibody or antigen binding fragment thereof that includes a light chain having at least 80%, 85%, 90%, 95%, 97%, 98%, or 99% sequence identity to a sequence selected from the group consisting of SEQ ID NOs: 21 or 41, wherein said antibody binds FXI and/or FXIa.

In specific aspects, the methods described herein comrpise administering pharmaceutical compositions comprising an antibody or antigen binding fragments thereof of Table 1, such as, for example antibody NOV1090 and NOV1401. In specific aspects, the methods described herein comrpise administering pharmaceutical compositions comprising a combination of two or more of the isolated antibodies or antigen binding fragments thereof of Table 1.

The present disclosure also relates to a method of treating, improving, or preventing a thromboembolic disease in a subject, wherein the method includes the step of administering to the subject an effective amount of a composition comprising an antibody that binds FXI and/or FXIa or antigen binding fragments thereof described herein. In one aspect, the thromboembolic disease is a thrombotic disorder (*e.g.*, thrombosis, thrombic stroke, AF, stroke prevention in AF (SPAF), deep vein thrombosis, venous thromboembolism, and pulmonary embolism). It is contemplated that the subject is human.

Any of the foregoing isolated antibodies or antigen binding fragments thereof may be a monoclonal antibody or antigen binding fragments thereof.

Non-limiting embodiments of the present disclosure are described in the following aspects:

1. A method of preventing, treating or managing or reducing the risk of stroke or thromboembolism comprising administering to a subject afflicated with end stage renal disease, an effective amount of a pharmaceutical composition comprising an antibody or antigen-binding fragment that binds within the catalytic domain of FXI and/or FXIa.

2. The method of aspect 1, wherein the subject is receiving dialysis or extracorporeal membrane oxygenation.

3. The method of aspect 1 or 2, wherein the subject is afflicted with atrial fibrillation.

4. The method of aspect 3, wherein the subject is afflicted with non-valvular atrial fibrillation.

5. A method of preventing or reducing the risk of stroke or thromboembolism comprising administering to a subject afflicated with atrial fibrillation and end stage renal disease and who is undergoing dialysis or extracorporeal membrane oxygentation, an effective amount of a pharmaceutical composition comprising an antibody or antigen-binding fragment that binds within the catalytic domain of FXI and/or FXIa.

6. The method of aspect 5, wherein the subject is afflicted with non-valvular atrial fibrillation.

7. The method of 3, 4, 5, or 6, wherein the subject has a demonstrated high risk of bleeding.

8. The method of any one of aspects 1-7, wherein the antibody or fragment thereof binds to one or more epitopes of FXI and/or FXIa, wherein the epitope comprises two or more amino acid residues of Pro410, Arg413, Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Lys527, Arg548, His552, Ser575, Ser594, Trp595, Gly596, Glu597, Arg602, Glu603, and Arg604.

9. The method of aspect 8, wherein the antibody or fragment thereof binds to one or more epitopes of anti-FXI and/or FXIa and the epitope comprises four or more amino acid residues of Pro410, Arg413, Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Lys527, Arg548, His552, Ser575, Ser594, Trp595, Gly596, Glu597, Arg602, Glu603, and Arg604.

10. The method of aspect 8, wherein the antibody or fragment thereof binds to one or more epitopes of anti-FXI and/or FXIa and the the epitope comprises six or more amino acid residues of Pro410, Arg413, Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Lys527, Arg548, His552, Ser575, Ser594, Trp595, Gly596, Glu597, Arg602, Glu603, and Arg604.

11. The method of aspect 8, wherein the antibody or fragment thereof binds to one or more epitopes of anti-FXI and/or FXIa and the the epitope comprises eight or more amino acid residues of Pro410, Arg413, Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Lys527, Arg548, His552, Ser575, Ser594, Trp595, Gly596, Glu597, Arg602, Glu603, and Arg604.

12. The method of aspect 8, wherein the antibody or fragment thereof binds to one or more epitopes of anti-FXI and/or FXIa and the epitope comprises the residues of Pro410, Arg413, Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Lys527, Arg548, His552, Ser575, Ser594, Trp595, Gly596, Glu597, Arg602, Glu603, and Arg604.

13. The method of aspect 8, wherein the antibody or fragment thereof binds to one or more epitopes of anti-FXI and/or FXIa and the epitope comprises amino acid residues of Pro410, Arg413, Lys527 and one or more amino acid residues of Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Arg548, His552, Ser575, Ser594, Trp595, Gly596, Glu597, Arg602, Glu603, and Arg604.

14. The method of aspect 8, wherein the antibody or fragment thereof binds to one or more epitopes of anti-FXI and/or FXIa and the epitope comprises amino acid residues of Pro410, Arg413, Lys527 and four or more amino acid residues of Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Arg548, His552, Ser575, Ser594, Trp595, Gly596, Glu597, Arg602, Glu603, and Arg604.

15. The method of aspect 8, wherein the antibody or fragment thereof binds to one or more epitopes of anti-FXI and/or FXIa and the epitope comprises amino acid residues of Pro410, Arg413, Lys527 and six or more amino acid residues of Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Arg548, His552, Ser575, Ser594, Trp595, Gly596, Glu597, Arg602, Glu603, and Arg604.

16. The method of any one of aspects 1-15, wherein said antibody or fragment blocks FXI and/or FXIa binding to one or more of Factor IX, Factor XIIa, and thrombin.

17. The method of aspect 16, wherein the antibody or fragment blocks FXI and/or FXIa binding to one or more of Factor IX, Factor XIIa, or thrombin, and other components of the coagulation pathway.

18. The method of any one of aspects 1-15, wherein the antibody or fragment blocks one or more of FIX, FXI, and FXIa binding to platelet receptors.

19. The method of any one of aspects 1-15, wherein said antibody or fragment prevents activation of the intrinsic or common coagulation pathways.

20. The method of any one of aspects 1-19, wherein the antibody or fragment thereof binds to a human FXI and/or FXIa protein with a $K_D$ of less than or equal to 34 nM, as measured by BIACORE™ assay, or less than or equal to 4 pM, as measured by solution equilibrium titration assay (SET).

21. The method of any one of aspects 1-20, wherein the antibody or fragment comprises at least one complementarity determining region having at least 90% identity to at least one of the CDRs recited in Table 1.

22. The method of any one of aspects 1-20, wherein the antibody or fragment comprises a CDR1, CDR2, and CDR3 from Table 1.

23. The method of any one of aspects 1-20, wherein the antibody or fragment comprises a heavy chain CDR3 selected from the group consisting of SEQ ID NO: 5 and 25.

24. The method of any one of aspects 1-23, wherein the antibody or fragment comprises a VH selected from the group consisting of SEQ ID NO: 9 and 29 or an amino acid sequence with 90% identity thereof; and a VL selected from the group consisting of SEQ ID NO: 19 and 39 or an amino acid sequence with 90% identity thereof.

25. The method of aspect 24, wherein the antibody or fragment comprises a VH selected from the group consisting of SEQ ID NO: 9 and 29 or an amino acid sequence with 95% identity thereof; and a VL selected from the group consisting of SEQ ID NO: 19 and 39 or an amino acid sequence with 95% identity thereof.

26. The method of aspect 24, wherein the antibody or fragment comprises a VH selected from the group consisting of SEQ ID NO: 9 and 29 or an amino acid sequence with 97% identity thereof; and a VL selected from the group consisting of SEQ ID NO: 19 and 39 or an amino acid sequence with 97% identity thereof.

27. The method of aspect 24, wherein the antibody or fragment comprises a variable heavy chain sequence selected from the group consisting of SEQ ID NO: 9 and 29.

28. The method of aspect 24, wherein the antibody or fragment comprises a variable light chain sequence selected from the group consisting of SEQ ID NO: 19 and 39.

29. The method of aspect 24, wherein the antibody or fragment comprises a variable heavy chain selected from the group consisting of SEQ ID NO: 9 and 29; and variable light chain sequence selected from the group consisting of SEQ ID NO: 19 and 39.

30. The method of aspect 24, wherein the antibody or fragment is selected from the group consisting of: (i) an antibody or fragment comprising a variable heavy chain of SEQ ID NO: 9 and a variable light chain sequence of SEQ ID NO: 19; (ii) an antibody or fragment comprising a variable heavy chain of SEQ ID NO: 29 and a variable light chain sequence of SEQ ID NO: 39; (iii) an antibody comprising a heavy chain comprising the amino acid sequence of SEQ ID NO: 29 and a light chain comprising the amino acid sequence of SEQ ID NO: 39; and (iv) an antibody comprising a heavy chain comprising the amino acid sequence of SEQ ID NO: 9 and a light chain comprising the amino acid sequence of SEQ ID NO: 19.

31. The method of any one of aspects 1-30, wherein the antibody or fragment comprises (i) a heavy chain variable region CDR1 comprises the amino acid sequence of SEQ ID NO: 46; CDR2 comprises the amino acid sequence of SEQ ID NO: 4; CDR3 comprises the amino acid sequence of SEQ ID NO: 5; and (ii) a light chain variable region CDR1 comprising the amino acid sequence of SEQ ID NO: 33; CDR2 comprising the amino acid sequence of SEQ ID NO: 14; and CDR3 comprising the amino acid sequence of SEQ ID NO: 15.

32. The method of any one of aspects 1-30, wherein the antibody or fragment comprises (i) a heavy chain variable region CDR1 selected from the group consisting of SEQ ID NO: 3 and 23; CDR2 selected from the group consisting of SEQ ID NO: 4 and 24; CDR3 selected from the group consisting of 5 and 25; and (ii) a light chain variable region CDR1 selected from the group consisting of SEQ ID NO: 13 and 33; CDR2 selected from the group consisting of SEQ ID NO: 14 and 34; and CDR3 selected from the group consisting of SEQ ID NO: 15 and 35.

33. The method of any one of aspects 1-30, wherein the antibody or fragment comprises (i) a heavy chain variable region CDR1 selected from the group consisting of SEQ ID NO: 6 and 26; CDR2 selected from the group consisting of SEQ ID NO: 7 and 27; CDR3 selected from the group consisting of 8 and 28; and (ii) a light chain variable region CDR1 selected from the group consisting of SEQ ID NO: 16 and 36; CDR2 selected from the group consisting of SEQ ID NO: 17 and 37; and CDR3 selected from the group consisting of SEQ ID NO: 18 and 38.

34. The method of any one of aspects 1-30, wherein the antibody or fragment comprises a heavy chain variable region CDR1 of SEQ ID NO: 3; a heavy chain variable region CDR2 of SEQ ID NO: 4; a heavy chain variable region CDR3 of SEQ ID NO: 5; a light chain variable region CDR1 of SEQ ID NO: 13; a light chain variable region CDR2 of SEQ ID NO: 14; and a light chain variable region CDR3 of SEQ ID NO: 15.

35. The method of any one of aspects 1-30, wherein the antibody or fragment comprises a heavy chain variable region CDR1 of SEQ ID NO: 23; a heavy chain variable region CDR2 of SEQ ID NO: 24; a heavy chain variable region CDR3 of SEQ ID NO: 25; a light chain variable region CDR1 of SEQ ID NO: 33; a light chain variable region CDR2 of SEQ ID NO: 34; and a light chain variable region CDR3 of SEQ ID NO: 35.

36. The method of any one of aspects 1-30, wherein the antibody or fragment comprises a heavy chain variable region CDR1 of SEQ ID NO: 6; a heavy chain variable region CDR2 of SEQ ID NO: 7; a heavy chain variable region CDR3 of SEQ ID NO: 8; a light chain variable region CDR1 of SEQ ID NO: 16; a light chain variable region CDR2 of SEQ ID NO: 17; and a light chain variable region CDR3 of SEQ ID NO: 18.

37. The method of any one of aspects 1-30, wherein the antibody or fragment comprises a heavy chain variable region CDR1 of SEQ ID NO: 26; a heavy chain variable region CDR2 of SEQ ID NO: 27; a heavy chain variable region CDR3 of SEQ ID NO: 28; a light chain variable region CDR1 of SEQ ID NO: 36; a light chain variable region CDR2 of SEQ ID NO: 37; and a light chain variable region CDR3 of SEQ ID NO: 38.

38. The method of any one of aspects 1-30, wherein the antibody or fragment binds to the same epitope as an antibody comprising a variable heavy chain of SEQ ID NO: 9 and a variable light chain sequence of SEQ ID NO: 19 or an antibody comprising a variable heavy chain of SEQ ID NO: 29 and a variable light chain sequence of SEQ ID NO: 39.

39. The method of any one of aspects 1-30, wherein the antibody or fragment competes for binding to a human FXI and/or FXIa protein with an antibody comprising a variable heavy chain of SEQ ID NO: 9 and a variable light chain sequence of SEQ ID NO: 19 or an antibody comprising a variable heavy chain of SEQ ID NO: 29 and a variable light chain sequence of SEQ ID NO: 39.

40. The method of any one of aspects 1-30, wherein the antibody or fragment is selected from the group consisting of NOV1090 and NOV1401.

41. A method of managing or reducing bleeding or bleeding risk in a subject afflicted with end stage renal disease who has been treated or administered an anti-FXI/FXIa antibody or antigen-biniding fragment that binds within the catalytic domain of FXI and/or FXIa, comprising the step of administering to the subject in need thereof, an anti-idiotype antibody or fragment thereof that specifically binds to the anti-FXI/FXIa antibody and blocks the anti-FXI/FXIa antibody from binding to FXI and/or FXIa, and wherein the anti-idiotype antibody or fragment thereof reverses the anticoagulant activity of the anti-FXI/FXIa antibody.

42. The method of aspect 41, wherein the anti-idiotype antibody or fragment thereof is administered to the subject once or a few times (e.g. twice, three times or four times) to reverse the anticoagulant effect of the anti-FXI/FXIa antibody.

43. A method of managing or reducing bleeding or bleeding risk in a subject afflicted with end stage renal disease who has been treated or administered an anti-FXI/FXIa antibody or antigen-biniding fragment that binds within the catalytic domain of FXI and/or FXIa, said method comprises temporarily reversing of the anticoagulant effect for a sufficient time to manage the bleeding by one of the following: (i) fluid replacement using colloids, crystalloids, human plasma or plasma proteins such as albumin; (ii) transfusion with packed red blood or whole blood; or (iii) administration of fresh frozen plasma (FFP), prothrombin complex concentrates (PCC), activated PCC (APCC), such as, factor VIII inhibitor, and/or recombinant activated factor VII.

44. The method of any one of aspects 41-43, wherein the subject is receiving dialysis, such as hemodialysis.

45. The method of any one of aspects 41-43, wherein the subject is afflicted with atrial fibrillation.

46. The method of aspect 45, wherein the subject is afflicted with non-valvular atrial fibrillation.

47. The method of aspect 45 or 46, wherein the subject has a demonstrated high risk of bleeding.

48. The method of any one of aspects 41-47, wherein the anti-FXI/FXIa antibody or antigen-biniding fragment comprises a VH selected from the group consisting of SEQ ID NO: 9 and 29 or an amino acid sequence with 90% identity thereof; and a VL selected from the group consisting of SEQ ID NO: 19 and 39 or an amino acid sequence with 90% identity thereof.

49. The method of any one of aspects 41-47, wherein the anti-FXI/FXIa antibody or antigen-biniding fragment competes, in a concentration-dependent manner, for binding to a human FXI and/or FXIa protein with an antibody comprising a variable heavy chain of SEQ ID NO: 9 and a variable light chain sequence of SEQ ID NO: 19 or an antibody comprising a variable heavy chain of SEQ ID NO: 29 and a variable light chain sequence of SEQ ID NO: 39.

50. The method of any one of aspects 1-49, wherein the antibody is a recombinant human antibody or a humanized antibody.

51. An antibody or antigen-binding fragment thereof that binds within the catalytic domain of FXI and/or FXIa, or a pharmaceutical composition comprising the antibody or antigen-binding fragment thereof, for use in preventing, treating or managing or reducing the risk of stroke or thromboembolism in a subject afflicated with end stage renal disease, wherein the antibody comprises a heavy chain comprising the amino acid sequence of SEQ ID NO: 31 and a light chain comprising the amino acid sequence of SEQ ID NO: 41.

52. The antibody or antigen-binding fragment thereof or a pharmaceutical composition according to aspect 51, wherein the subject is receiving dialysis.

53. The antibody or antigen-binding fragment thereof or a pharmaceutical composition according to aspect 51 or 52, wherein the subject is receiving hemodialysis or other types of dialysis, such as, peritoneal dialysis.

54. The antibody or antigen-binding fragment thereof or a pharmaceutical composition according to any one of claims 51-53, wherein the subject is afflicted with atrial fibrillation, such as non-valvular atrial fibrillation.

55. An antibody or antigen-binding fragment thereof that binds within the catalytic domain of FXI and/or FXIa, or a pharmaceutical composition comprising the antibody or antigen-binding fragment thereof, for use in preventing or reducing the risk of stroke or thromboembolism in a subject afflicated with atrial fibrillation and end stage renal disease and who is undergoing dialysis, such as hemodialysis, wherein the antibody comprises a heavy chain comprising the amino acid sequence of SEQ ID NO: 31 and a light chain comprising the amino acid sequence of SEQ ID NO: 41.

56. The antibody or antigen-binding fragment thereof or a pharmaceutical composition according to any one of aspects 51-55, wherein the subject is afflicted with non-valvular atrial fibrillation.

57. An antibody or antigen-binding fragment thereof that binds within the catalytic domain of FXI and/or FXIa, or a pharmaceutical composition comprising the antibody or antigen-binding fragment thereof, for use in preventing, treating or managing or reducing the risk of catheter-related thrombosis in a subject afflicated with end stage renal disease and initiated on hemodialysis with a catheter, wherein the antibody comprises a heavy chain comprising the amino acid sequence of SEQ ID NO: 31 and a light chain comprising the amino acid sequence of SEQ ID NO: 41.

58. The antibody or antigen-binding fragment thereof or a pharmaceutical composition according to any one of aspects 51-58, wherein the subject has a demonstrated high risk of bleeding.

59. An antibody or antigen-binding fragment thereof that binds within the catalytic domain of FXI and/or FXIa, or a pharmaceutical composition comprising the antibody or antigen-binding fragment thereof, for use in preventing or reducing the risk of stroke or thromboembolism, such as systemic embolism, in a subject afflicated with atrial fibrillation, wherein the subject has a demonstrated high risk of bleeding which is characterized by a $CHA_2DS_2VASc$ risk score $\geq 2$, and wherein the antibody comprises a heavy chain comprising the amino acid sequence of SEQ ID NO: 31 and a light chain comprising the amino acid sequence of SEQ ID NO: 41.

60. The antibody or antigen-binding fragment thereof or a pharmaceutical composition according to any one of aspects 51-59, wherein the subject is at least 55 years old.

61. The antibody or antigen-binding fragment thereof or a pharmaceutical composition according to any one of aspects 51-60, wherein the subject has a demonstrated high risk of bleeding characterized as having (i) a $CHA_2DS_2VASc$ risk score $\geq 2$ and (ii) one, two or more of the following risk factors:

 (a) over 65 years old;

 (b) history of previous stroke or transient ischemic attack;

 (c) previous major bleed or clinically relevant bleed;

 (d) chronic kidney disease (CKD) stage 3 to 4;

 (e) treatment with single or dual antiplatelet therapy; and

 (f) history of falls associated with hematoma bruise or other manifestations.

62. The antibody or antigen-binding fragment thereof or a pharmaceutical composition according to any one of aspects 51-61, wherein the antibody or antigen-binding fragment thereof is administered to the subject subcutaneously.

63. The antibody or antigen-binding fragment thereof or a pharmaceutical composition according to any one of aspects 37-48, wherein the antibody or antigen-binding fragment thereof is administered at a dose of at least 90 mg, 120 mg, 150 mg, 180 mg, or 210 mg.

64. The antibody or antigen-binding fragment thereof or a pharmaceutical composition according to any one of aspects 51-63, wherein the antibody or antigen-binding fragment thereof is administered at a dose of 120 mg, 150 mg, or 180 mg.

65. The antibody or antigen-binding fragment thereof or a pharmaceutical composition according to any one of aspects 51-64, wherein the antibody or antigen-binding fragment thereof is administered once a month.

66. Use of antibody or antigen-binding fragment thereof that binds within the catalytic domain of FXI and/or FXIa for the manufacture of a medicament for preventing, treating or managing or reducing the risk of stroke or thromboembolism in a subject afflicated with end stage renal disease, wherein the antibody comprises a heavy chain comprising the amino acid sequence of SEQ ID NO: 31 and a light chain comprising the amino acid sequence of SEQ ID NO: 41.

67. The use according to aspect 66, wherein the subject is receiving dialysis.

68. The use according to aspect 66 or 67, wherein the subject is receiving hemodialysis or other types of dialysis, such as, peritoneal dialysis.

69. The use according to any one of aspects 66-68, wherein the subject is afflicted with atrial fibrillation, such as non-valvular atrial fibrillation.

70. Use of an antibody or antigen-binding fragment thereof that binds within the catalytic domain of FXI and/or FXIa for the manufacture of a medicament for preventing or reducing the risk of stroke or thromboembolism in a subject afflicated with atrial fibrillation and end stage renal disease and who is undergoing dialysis, such as hemodialysis, wherein the antibody comprises a heavy chain comprising the amino acid sequence of SEQ ID NO: 31 and a light chain comprising the amino acid sequence of SEQ ID NO: 41.

71. The use according to any one of aspects 66-70, wherein the subject is afflicted with non-valvular atrial fibrillation.

72. Use of an antibody or antigen-binding fragment thereof that binds within the catalytic domain of FXI and/or FXIa for the manufacture of a medicament for preventing, treating or managing or reducing the risk of catheter-related thrombosis in a subject afflicated with end stage renal disease and initiated on hemodialysis with a catheter, wherein the antibody comprises a heavy chain comprising the amino acid sequence of SEQ ID NO: 31 and a light chain comprising the amino acid sequence of SEQ ID NO: 41.

73. The use according to any one of claims 66-72, wherein the subject has a demonstrated high risk of bleeding.

74. Use of an antibody or antigen-binding fragment thereof that binds within the catalytic domain of FXI and/or FXIa for the manufacture of a medicament for preventing or reducing the risk of stroke or thromboembolism, such as systemic embolism, in a subject afflicated with atrial fibrillation, wherein the subject has a demonstrated high risk of bleeding which is characterized by a $CHA_2DS_2VASc$ risk score $\geq 2$, and wherein the antibody comprises a heavy chain comprising the amino acid sequence of SEQ ID NO: 31 and a light chain comprising the amino acid sequence of SEQ ID NO: 41.

75. The use according to any one of aspects 66-74, wherein the subject is at least 55 years old.

76. The use according to any one of aspects 66-75, wherein the subject has a demonstrated high risk of bleeding characterized as having (i) a $CHA_2DS_2VASc$ risk score $\geq 2$ and (ii) one, two or more of the following risk factors:

   (a) over 65 years old;

   (b) history of previous stroke or transient ischemic attack;

   (c) previous major bleed or clinically relevant bleed;

   (d) chronic kidney disease (CKD) stage 3 to 4;

   (e) treatment with single or dual antiplatelet therapy; and

   (f) history of falls associated with hematoma bruise or other manifestations.

77. The use according to any one of aspects 66-76, wherein the the medicament is suitable for administration to the subject subcutaneously.

78. The use according to any one of aspects 66-77, wherein the medicament is suitable for administration to the subject at a dose of at least 90 mg, 120 mg, 150 mg, 180 mg, or 210 mg.

79. The use according to any one of aspects 66-78, wherein the medicament is suitable for administration to the subject at a dose of 120 mg, 150 mg, or 180 mg.

80. The use according to any one of aspects 66-79, wherein the medicament is suitable for administration to the subject once a month.

81. The method of any one of aspects 1-40 further comprises administering to the subject one or more therapeutically active agents.

82. The method of aspect 81 wherein the one or more therapeutically active agents is selected from the group consisting of: thromboxane inhibitors (e.g., aspirin), adenosine diphosphate receptor antagonists (or P2Y12 inhibitors) such as the thienopyridines (e.g., clopidogrel and prasugrel) and the nonthienopyridines (e.g., ticagrelor and cangrelor), and protease-activated receptor-1 (PAR1) antagonists (e.g., Vorapaxar).

83. The method of aspect 81, wherein the one or more therapeutic active agents is a proton pump inhibitor (PPI) and wherein the subject has or has a history of a GI disorder, such as previous GI bleed or antecedent of peptic ulcer.

84. The antibody or antigen-binding fragment thereof or a pharmaceutical composition according to any one of aspects 51-64, which is for use in combination with one or more therapeutically active agents.

85. The antibody or antigen-binding fragment thereof or a pharmaceutical composition according to aspect 84, wherein the one or more therapeutically active agents is selected from the group consisting of: thromboxane inhibitors (e.g., aspirin), adenosine diphosphate receptor antagonists (or P2Y12 inhibitors) such as the thienopyridines (e.g., clopidogrel and prasugrel) and the nonthienopyridines (e.g., ticagrelor and cangrelor), and protease-activated receptor-1 (PAR1) antagonists (e.g., Vorapaxar).

86. The antibody or antigen-binding fragment thereof or a pharmaceutical composition according to aspect 84, wherein the subject has or has a history of a GI disorder, such as previous GI bleed or antecedent of peptic ulcer.

87. A method of preventing, treating or managing or reducing the risk of stroke or thromboembolism, such as systemic embolism, comprising administering to a subject in need thereof about 100 mg to about 250 mg of an anti-Factor XI ("FXI") antibody or antigen-binding fragment thereof that specifically binds within the catalytic domain of FXI, wherein the subject has one, two, or more, or all, of the following characteristics:

(i) Male and female subjects $\geq$ 55 and < 85 years old;

(ii) Body weight between 50 and 130 kg inclusive;

(iii) Atrial fibrillation (AF) or atrial flutter, as documented by Electrocardiography;

(iv) $CHA_2DS_2$-VASc risk score $\geq$ 2 for male and female subject;

(v) Male subject with $CHA_2DS_2VASc$ risk score of 1 and anticoagulation therapy is warranted; and

(vi) Either anticoagulant-naïve or receiving a stable treatment of a recommended dose of a new oral anticoagulant (NOAC) over the 8 weeks prior to treatment.

88. The method of aspect 87, wherein the subject is afflicted with non-valvular atrial fibrillation.

89. The method of aspect 87 or 88, wherein the anti-FXI antibody comprises a heavy chain comprising the amino acid sequence of SEQ ID NO: 31 and a light chain comprising the amino acid sequence of SEQ ID NO: 41.

90. The method of any one of the preceding aspects, wherein the anti-FXI antibody or antigen-binding fragment thereof is administered at a dose of 120 mg, 150 mg, or 180 mg, once a month.

TERMINOLOGY

Unless defined otherwise, all technical and scientific terms used herein have the same meaning as commonly understood by those of ordinary skill in the art to which this present disclosure pertains.

The terms "FXI protein," "FXI antigen," and "FXI" are used interchangeably, and refers to the Factor XI protein in different species. Factor XI is the mammalian plasma coagulation factor XI, a glycoprotein present in human plasma at a concentration of 25-30 nM as a zymogen that when converted by limited proteolysis to an active serine protease, participates in the intrinsic pathway of blood coagulation.

The terms "FXIa protein," "FXIa antigen," and "FXIa", are used interchangeably, and refers to the activated FXI protein in different species. The zymogen Factor XI is converted into its active form, the coagulation factor XIa (FXIa), either via the contact phase of blood coagulation or through thrombin-mediated activation on the platelet surface. During this activation of factor XI, an internal peptide bond is cleaved in each of the two chains, resulting in the activated factor XIa, a serine protease composed of two heavy and two light chains held together by disulfide bonds. This serine protease FXIa converts the coagulation Factor IX into IXa, which subsequently activates coagulation Factor X (Xa). Xa then can mediate coagulation Factor II/Thrombin activation. For example, human FXI has the sequence as set out in Table 1 (SEQ ID NO:1), and has been described in previous reports and literature (Mandle RJ Jr, et al. (1979) Blood;54(4):850; NCBI Reference Sequence: AAA51985).

In the context of this present disclosure, the terms "FXI" and "FXIa" (and the like) include mutants and variants of the natural FXI and FXIa protein, respectively, which have substantially the same amino acid sequence as that of the native primary structure (amino acid sequence) described in the above-mentioned reports.

The term "catalytic domain," "serine protease catalytic domain," and similar terms as used herein, means amino acids Ile370 to Val607, as counted from the Glu1 at the N-terminus of the mature protein that is in circulation. It can also be described as residues 388-625 at the C-terminus of FXI. As used herein, the term "active site" means the catalytic triad comprised of the amino acids His413, Asp462 and Se557. (Bane and Gailani (2014) Drug Disc. 19(9)).

The term "about" in relation to a numerical value x means, for example, $x \pm 10\%$.

The term "antibody" as used herein means a whole antibody and any antigen binding fragment (i.e., "antigen-binding portion") or single chain thereof. A whole antibody is a glycoprotein comprising at least two heavy (H) chains and two light (L) chains inter-connected by disulfide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. In some specific aspects, an antibody can be a monoclonal antibody, human antibody, humanized antibody, camelised antibody, or chimeric antibody. Antibodies can be of any isotype (e.g., IgG, IgE, IgM, IgD, IgA and IgY), class (e.g., IgG1, IgG2, IgG3, IgG4, IgA1 and IgA2) or subclass.

The term "antigen binding portion" or "antigen binding fragment" of an antibody, as used herein, refers to one or more fragments of an intact antibody that retain the ability to specifically bind to a given antigen (e.g., Factor XIa (FXIa)). Antigen binding functions of an antibody can be performed by fragments of an intact antibody. Examples of binding fragments encompassed within the term antigen binding portion or antigen binding fragment of an antibody include a Fab fragment, a monovalent fragment consisting of the VL, VH, CL and CH1 domains; a F(ab)$_2$ fragment, a bivalent fragment comprising two Fab fragments linked by a disulfide bridge at the hinge region; an Fd fragment consisting of the VH and CH1 domains; an Fv fragment consisting of the VL and VH domains of a single arm of an antibody; a single domain antibody (dAb) fragment (Ward et al., 1989 Nature 341:544-546), which consists of a VH domain or a VL domain; and an isolated complementarity determining region (CDR).

Furthermore, although the two domains of the Fv fragment, VL and VH, are coded for by separate genes, they can be joined, using recombinant methods, by an artificial peptide linker that enables them to be made as a single protein chain in which the VL and VH regions pair to form monovalent molecules (known as single chain Fv (scFv); see, e.g., Bird et al., 1988 Science 242:423-426; and Huston et al., 1988 Proc. Natl. Acad. Sci. 85:5879-5883). Such single chain antibodies include one or more antigen binding portions or fragments of an antibody. These antibody fragments are obtained using conventional techniques known to those of skill in the art, and the fragments are screened for utility in the same manner as are intact antibodies.

Antigen binding fragments can also be incorporated into single domain antibodies, maxibodies, minibodies, intrabodies, diabodies, triabodies, tetrabodies, v-NAR and bis-scFv (see, e.g., Hollinger and Hudson, 2005, Nature Biotechnology, 23, 9, 1126-1136). Antigen binding portions of antibodies can be grafted into scaffolds based on polypeptides such as Fibronectin type III (Fn3) (see U.S. Pat. No. 6,703,199, which describes fibronectin polypeptide monobodies).

Antigen binding fragments can be incorporated into single chain molecules comprising a pair of tandem Fv segments (VH-CH1-VH-CH1) which, together with complementary light chain polypeptides, form a pair of antigen binding regions (Zapata et al., 1995 Protein Eng. 8(10):1057-1062; and U.S. Pat. No. 5,641,870).

As used herein, the term "affinity" refers to the strength of interaction between antibody and antigen at single antigenic sites. Within each antigenic site, the variable region of the antibody "arm" interacts through weak non-covalent forces with antigen at numerous sites; the more interactions, the stronger the affinity. As used herein, the term "high affinity" for an antibody or antigen binding fragments thereof (*e.g.,* a Fab fragment) generally refers to an antibody, or antigen binding fragment, having a $K_D$ of $10^{-9}$ M or less (e.g., a $K_D$ of $10^{-10}$ M or less, a $K_D$ of $10^{-11}$ M or less, a $K_D$ of $10^{-12}$ M or less, a $K_D$ of $10^{-13}$ M or less, a $K_D$ of $10^{-14}$ M or less, etc.).

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refer to compounds that have the same basic chemical structure as a naturally occurring amino acid, *i.e.,* an alpha carbon that is bound to a hydrogen, a carboxyl group, an amino group, and an R group, e.g., homoserine, norleucine, methionine sulfoxide, methionine methyl sulfonium. Such analogs have modified R groups *(e.g.,* norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid.

The term "binding specificity" as used herein refers to the ability of an individual antibody combining site to react with only one antigenic determinant.

The phrase "specifically (or selectively) binds" to an antibody (e.g., a FXI and/or FXIa - binding antibody) refers to a binding reaction that is determinative of the presence of a cognate antigen (*e.g.,* a human FXI and/or FXIa or cynomolgus FXI and/or FXIa) in a heterogeneous population of proteins and other biologics. The phrases "an antibody recognizing an antigen" and "an antibody specific for an antigen" are used interchangeably herein with the term "an antibody which binds specifically to an antigen".

The term "FXI and/or FXIa mediated" refers to the fact that FXI and/or FXIa mediates the intrinsic and/or common coagulation pathways by directly or indirectly activating Factor IX (also known as FIX), Factor X (FX), and/or thrombin, and/or by binding to platelet receptors.

The term "hemostasis" represents the principal mechanisms for arresting the flow of blood at sites of injury and restoring vascular patency during wound healing, respectively. During normal hemostasis and pathological thrombosis, three mechanisms become activated simultaneously: primary hemostasis meaning the interactions of activated platelets with the vessel wall, the formation of fibrin, and a process termed as fibrinolysis.

The terms "coagulation and coagulation cascade," "cascade model of coagulation," and the like, refer to the protein based system which serves to stabilize a clot that has formed to seal up a wound. The coagulation pathway is a proteolytic cascade. Each enzyme of the pathway is present in the plasma as a Zymogen (in an inactive form), which on activation undergoes proteolytic cleavage to release the active factor from the precursor molecule. The coagulation cascade functions as a series of positive and negative feedback loops which control the activation process. The ultimate goal of the pathway is to produce thrombin, which can then convert soluble fibrinogen into fibrin that forms a clot.

The process of generation of thrombin can be divided into three phases: the intrinsic and extrinsic pathways, which provide alternative routes for the generation of an active clotting factor: FXa (Activated Factor-X), and the final common pathway, which results in thrombin formation (Hoffman M.M. and Monroe D.M. (2005) Curr Hematol Rep. 4:391 -396; Johne J, et al. (2006) Biol Chem. 387:173-178).

"Platelet aggregation" refers to the process whereby when a break in a blood vessel occurs, substances are exposed that normally are not in direct contact with the blood flow. These substances (primarily collagen and von Willebrand factor) allow the platelets to adhere to the broken surface. Once a platelet adheres to the surface, it releases chemicals that attract additional platelets to the damaged area, referred to as platelet aggregation. These two processes are the first responses to stop bleeding.

A "thromboembolic disorder," or similar terms as used herein, refer to any number of conditions or diseases in which the intrinsic and/or common coagulation pathways are aberrantly activated or are not naturally deactivated (e.g., without therapeutic means). These conditions include but are not limited to thromboemolic stroke and other types of stroke of ischemicorigin, atrial fibrillation, stroke prevention in atrial fibrillation (SPAF), deep vein thrombosis, venous thromboembolism, and pulmonary embolism. These can also include prevention and treatment of catheter-related thromobsis (e.g., Hickman catheter in oncology patients) in which catheters become thrombosed, and extracorporeal membrane oxygenation (ECMO), in which the tubing and oxygenation membrane develops clots.

A "thromboembolic," or similar terms as used herein, can also refer to any number of the following, which the anti-FXI and/or FXIa antibodies or antigen binding fragments thereof of the present disclosure can be used to prevent or treat:

- thromboembolism in subjects with suspected or confirmed cardiac arrhythmia such as paroxysmal, persistent or permanent atrial fibrillation or atrial flutter;
- stroke prevention in atrial fibrillation (SPAF), a subpopulation of which is AF patients undergoing percutaneous coronary interventions (PCI);
- acute venous thromboembolic events (VTE) treatment and extended secondary VTE prevention in patients at high risk for bleeding;
- cerebral and cardiovascular events in secondary prevention after transient ischemic attack (TIA) or non-disabling stroke and prevention of thromboembolic events in heart failure with sinus rhythm;
- clot formation in left atrium and thromboembolism in subjects undergoing cardioversion for cardiac arrhythmia;
- thrombosis before, during and after ablation procedure for cardiac arrhythmia;
- venous thrombosis, this includes but not exclusively, treatment and secondary prevention of deep or superficial veins thrombosis in the lower members or upper member, thrombosis in the abdominal and thoracic veins, sinus thrombosis and thrombosis of jugular veins;
- thrombosis on any artificial surface in the veins or arteries like catheter, pacemaker wires, synthetic arterial grafts; mechanical or biological heart valves or left ventricular assist device;
- pulmonary embolism in patients with or without venous thrombosis;
- Chronic Thromboembolic Pulmonary Hypertension (CTEPH);
- arterial thrombosis on ruptured atherosclerotic plaque, thrombosis on intra-arterial prosthesis or catheter and thrombosis in apparently normal arteries, this includes but not limited to acute coronary syndromes, ST elevation myocardial infarction, non ST elevation myocardial infarction, unstable angina, stent thrombosis, thrombosis of any artificial surface in the arterial system and thrombosis of pulmonary arteries in subjects with or without pulmonary hypertension;
- thrombosis and thromboembolism in patients undergoing percutaneous coronary interventions (PCI);
- cardioembolic and cryptogenic strokes;
- thrombosis in patients with invasive and non-invasive cancer malignancies;
- thrombosis over an indwelling catheter;
- thrombosis and thromboembolism in severely ill patients;
- cardiac thrombosis and thromboembolism, this includes but not exclusively cardiac thrombosis after myocardial infarction, cardiac thrombosis related to condition such as cardiac aneurysm, myocardial fibrosis, cardiac enlargement and insufficiency, myocarditis and artificial surface in the heart;
- thromboembolism in patients with valvular heart disease with or without atrial fibrillation;
- thromboembolism over valvular mechanic or biologic prostheses;
- thromboembolism in patients who had native or artificial cardiac patches, arterial or venous conduit tubes after heart repair of simple or complex cardiac malformations;
- venous thrombosis and thromboembolism after knee replacement surgery, hip replacement surgery, and orthopedic surgery, thoracic or abdominal surgery;
- arterial or venous thrombosis after neurosurgery including intracranial and spinal cord interventions;
- congenital or acquired thrombophilia including but not exclusively factor V Leiden, prothrombin mutation, antithrombin III, protein C and protein S deficiencies, factor XIII mutation, familial dysfibrinogenemia, congenital deficiency of plasminogen, increased levels of factor XI, sickle cell disease, antiphospholipid syndrome, autoimmune disease, chronic bowel disease, nephrotic syndrome, hemolytic uremia, myeloproliferative disease, disseminated intra vascular coagulation, paroxysmal nocturnal hemoglobinuria and heparin induced thrombopenia;
- thrombosis and thromboembolism in chronic kidney disease; and
- thrombosis and thromboembolism in patients undergoing hemodialysis and in patients undergoing extra-corporal membrane oxygenation.

The term "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity. For example, a mouse antibody can be modified by replacing its constant region with the constant region from a human immunoglobulin.

Due to the replacement with a human constant region, the chimeric antibody can retain its specificity in recognizing the antigen while having reduced antigenicity in human as compared to the original mouse antibody.

The term "conservatively modified variant" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

For polypeptide sequences, "conservatively modified variants" include individual substitutions, deletions or additions to a polypeptide sequence which result in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the present disclosure. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g., Creighton, Proteins (1984)). In some embodiments, the term "conservative sequence modifications" are used to refer to amino acid modifications that do not significantly affect or alter the binding characteristics of the antibody containing the amino acid sequence.

The term "epitope" means a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents. Two antibodies are said to "compete" if one antibody is shown to bind the same epitope as the second antibody in a competitive binding assay, by any of the methods well known to those of skill in the art.

The term "human antibody", as used herein, is intended to include antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences. The human antibodies of the present disclosure may include amino acid residues not encoded by human sequences (e.g., mutations introduced by random or site-specific mutagenesis *in vitro* or by somatic mutation *in vivo).*

The term "human monoclonal antibody" refers to antibodies displaying a single binding specificity which have variable regions in which both the framework and CDR regions are derived from human sequences. In one embodiment, the human monoclonal antibodies are prepared using phage display methods for screening libraries of human immunoglobulin genes.

A "humanized" antibody is an antibody that retains the reactivity of a non-human antibody while being less immunogenic in humans. This can be achieved, for instance, by retaining the non-human CDR regions and replacing the remaining parts of the antibody with their human counterparts (*i.e.,* the constant region as well as the framework portions of the variable region). See, *e.g.,* Morrison et al., Proc. Natl. Acad. Sci. USA, 81:6851-6855, 1984; Morrison and Oi, Adv. Immunol., 44:65-92, 1988; Verhoeyen et al., Science, 239:1534-1536, 1988; Padlan, Molec. Immun., 28:489-498, 1991; and Padlan, Molec. Immun., 31:169-217, 1994. Other examples of human engineering technology include, but are not limited to Xoma technology disclosed in US 5,766,886.

The terms "identical" or percent "identity," in the context of two or more nucleic acids or polypeptide sequences, refer to two or more sequences or subsequences that are the same. Two sequences are "substantially identical" if two sequences have a specified percentage of amino acid residues or nucleotides that are the same (*i.e.,* 60% identity, optionally 65%, 70%, 75%, 80%, 85%, 90%, 95%, or 99% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated.

Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g.,* by the local homology algorithm of Smith and Waterman (1970) Adv. Appl. Math. 2:482c, by the homology alignment algorithm of Needleman and Wunsch, J. Mol. Biol. 48:443, 1970, by the search for similarity method of Pearson and Lipman, Proc. Nat'l. Acad. Sci. USA 85:2444, 1988, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, *e.g.,* Brent et al., Current Protocols in Molecular Biology, John Wiley & Sons, Inc. (Ringbou ed., 2003)).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.,* supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sci. USA 89:10915, 1989) alignments (B) of 50, expectation (E) of 10, M=5, N=-4, and a comparison of both strands.

The BLAST algorithm also performs a statistical analysis of the similarity between two sequences (see, *e.g*., Karlin and Altschul, Proc. Natl. Acad. Sci. USA 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The percent identity between two amino acid sequences can also be determined using the algorithm of E. Meyers and W. Miller (Comput. Appl. Biosci., 4:11-17, 1988) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453, 1970) algorithm which has been incorporated into the GAP program in the GCG software package (available on the world wide web at gcg.com), using either a Blossom 62 matrix or a PAM250 matrix, and a gap weight of 16, 14, 12, 10, 8, 6, or 4 and a length weight of 1, 2, 3, 4, 5, or 6.

Other than percentage of sequence identity noted above, another indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequence.

The term "isolated antibody" refers to an antibody that is substantially free of other antibodies having different antigenic specificities (e.g., an isolated antibody that specifically binds FXI and/or FXIa is substantially free of antibodies that specifically bind antigens other than FXI and/or FXIa). An isolated antibody that specifically binds FXI and/or FXIa may, however, have cross-reactivity to other antigens. Moreover, an isolated antibody may be substantially free of other cellular material and/or chemicals.

The term "isotype" refers to the antibody class (e.g., IgM, IgE, IgG such as IgG1 or IgG4) that is provided by the heavy chain constant region genes. Isotype also includes modified versions of one of these classes, where modifications have been made to alter the Fc function, for example, to enhance or reduce effector functions or binding to Fc receptors.

The term "$k_{assoc}$" or "$k_a$", as used herein, is intended to refer to the association rate of a particular antibody-antigen interaction, whereas the term "$k_{dis}$" or "$k_d$," as used herein, is intended to refer to the dissociation rate of a particular antibody-antigen interaction. The term "$K_D$", as used herein, is intended to refer to the dissociation constant, which is obtained from the ratio of $k_d$ to $k_a$ (*i.e.* $k_d/k_a$) and is expressed as a molar concentration (M). $K_D$ values for antibodies can be determined using methods well established in the art. Methods for determining the $K_D$ of an antibody include measuring surface plasmon resonance using a biosensor system such as a BIACORE™ system, or measuring affinity in solution by solution equilibrium titration (SET).

The terms "monoclonal antibody" or "monoclonal antibody composition" as used herein refer to a preparation of antibody molecules of single molecular composition. A monoclonal antibody composition displays a single binding specificity and affinity for a particular epitope.

The term "nucleic acid" is used herein interchangeably with the term "polynucleotide" and refers to deoxyribonucleotides or ribonucleotides and polymers thereof in either single- or double-stranded form. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also implicitly encompasses conservatively modified variants thereof (*e.g.*, degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., Nucleic Acid Res. 19:5081, 1991; Ohtsuka et al., J. Biol. Chem. 260:2605-2608, 1985; and Rossolini et al., Mol. Cell. Probes 8:91-98, 1994).

The term "operably linked" refers to a functional relationship between two or more polynucleotide (*e.g.,* DNA) segments. Typically, the term refers to the functional relationship of a transcriptional regulatory sequence to a transcribed sequence. For example, a promoter or enhancer sequence is operably linked to a coding sequence if it stimulates or modulates the transcription of the coding sequence in an appropriate host cell or other expression system. Generally, promoter transcriptional regulatory sequences that are operably linked to a transcribed sequence are physically contiguous to the transcribed sequence, *i.e.,* they are cis-acting. However, some transcriptional regulatory sequences, such as enhancers, need not be physically contiguous or located in close proximity to the coding sequences whose transcription they enhance.

As used herein, the term, "optimized" means that a nucleotide sequence has been altered to encode an amino acid sequence using codons that are preferred in the production cell or organism, generally a eukaryotic cell, for example, a cell of Pichia, a Chinese Hamster Ovary cell (CHO) or a human cell. The optimized nucleotide sequence is engineered to retain completely or as much as possible the amino acid sequence originally encoded by the starting nucleotide sequence, which is also known as the "parental" sequence. The optimized sequences herein have been engineered to have codons that are preferred in mammalian cells. However, optimized expression of these sequences in other eukaryotic cells or prokaryotic cells is also envisioned herein. The amino acid sequences encoded by optimized nucleotide sequences are also referred to as optimized.

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

The term "recombinant human antibody", as used herein, includes all human antibodies that are prepared, expressed, created or isolated by recombinant means, such as antibodies isolated from an animal *(e.g.,* a mouse) that is transgenic or transchromosomal for human immunoglobulin genes or a hybridoma prepared therefrom, antibodies isolated from a host cell transformed to express the human antibody, *e.g.,* from a transfectoma, antibodies isolated from a recombinant, combinatorial human antibody library, and antibodies prepared, expressed, created or isolated by any other means that involve splicing of all or a portion of a human immunoglobulin gene, sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo*.

The term "recombinant host cell" (or simply "host cell") refers to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The term "subject" includes human and non-human animals. Non-human animals include all vertebrates (*e.g.*: mammals and non-mammals) such as, non-human primates (*e.g.*: cynomolgus monkey), sheep, rabbit, dog, cow, chickens, amphibians, and reptiles. Except when noted, the terms "patient" or "subject" are used herein interchangeably. As used herein, the terms "cyno" or "cynomolgus" refer to the cynomolgus monkey *(Macaca fascicularis).* In specific aspects, a patient or subject is a human.

As used herein, the term "treating" or "treatment" of any disease or disorder (*e.g.*, a thromboembolic disorder) refers in one embodiment, to ameliorating the disease or disorder (*i.e.*, slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (*e.g.*, stabilization of a discernible symptom), physiologically, (*e.g.*, stabilization of a physical parameter), or both. In yet another embodiment, "treating" or "treatment" refers to preventing or delaying the onset or development or progression of the disease or disorder.

"Prevention" as it relates to indications described herein, including, *e.g.*, a thromboembolic disorder, means any action that prevents or slows a worsening in *e.g.*, a thromboembolic disease parameters, as described below, in a patient at risk for said worsening.

The term "vector" is intended to refer to a polynucleotide molecule capable of transporting another polynucleotide to which it has been linked. One type of vector is a "plasmid", which refers to a circular double stranded DNA loop into which additional DNA segments may be ligated. Another type of vector is a viral vector, such as an adeno-associated viral vector (AAV, or AAV2), wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the present disclosure is intended to include such other forms of expression vectors, such as viral vectors (e.g., replication defective retroviruses, adenoviruses and adeno-associated viruses), which serve equivalent functions.

BRIEF DESCRIPTION OF THE DRAWINGS

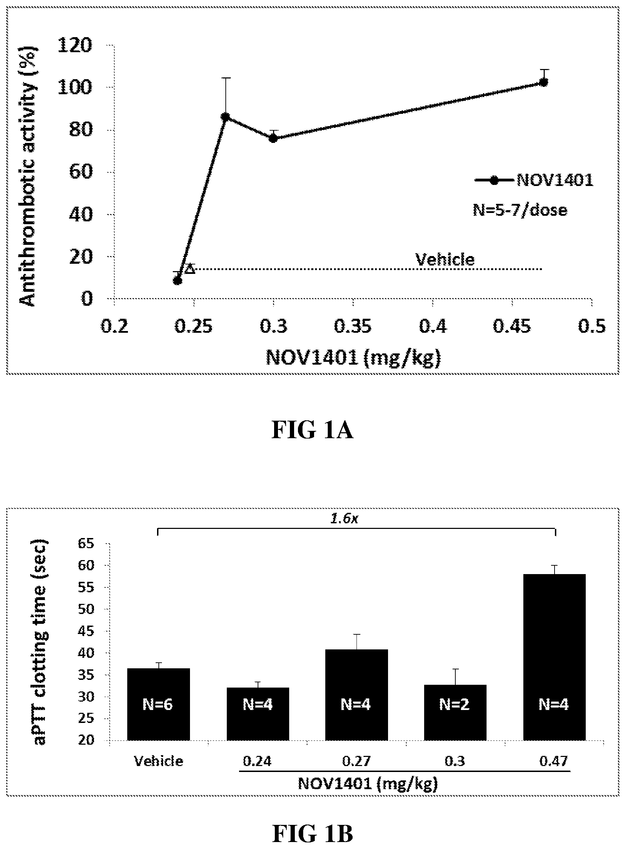

**Figures 1A-C** show the effect of NOV1401 on $FeCl_3$-induced thrombosis in $FXI^{-/-}$ mice reconstituted with human FXI protein. NOV1401 dose-dependently inhibited thrombosis. The antibody prolonged aPTT to the same extent as in untreated $FXI^{-/-}$ mice.

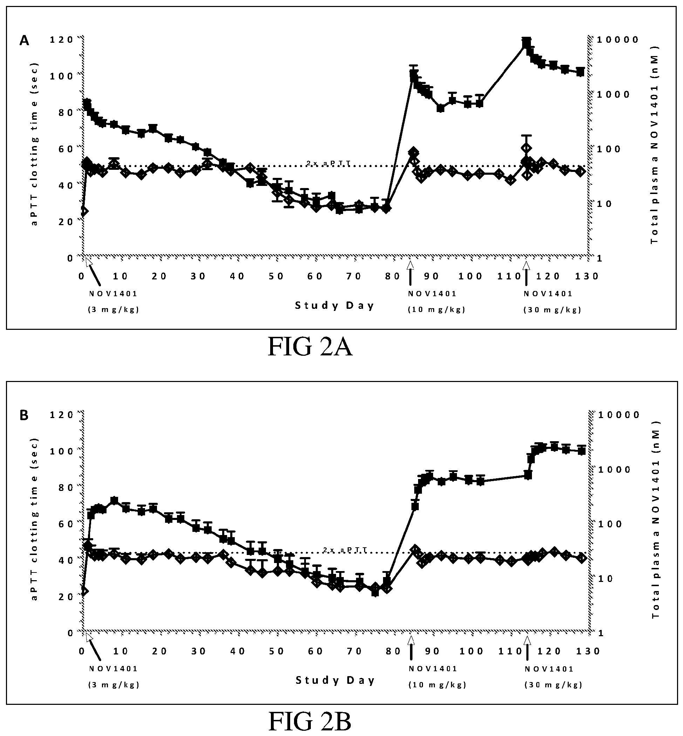

**Figures 2A-B** show the effect of multiple intravenous (i.v.) (A; N=2) or subcutaneous (s.c.) (B; N=2) doses of 3 mg/kg, 10 mg/kg and 30 mg/kg NOV1401 on aPTT (diamonds) and relationship to total plasma NOV1401 levels (squares) in cynomolgus monkeys. A single dose of 3 mg/kg led to ~2x aPTT that was maintained for 5-6 weeks. All doses tested prolonged aPTT to a similar extent, and the higher doses tested did not seem to increase the magnitude of aPTT prolongation observed at the 3 mg/kg dose.

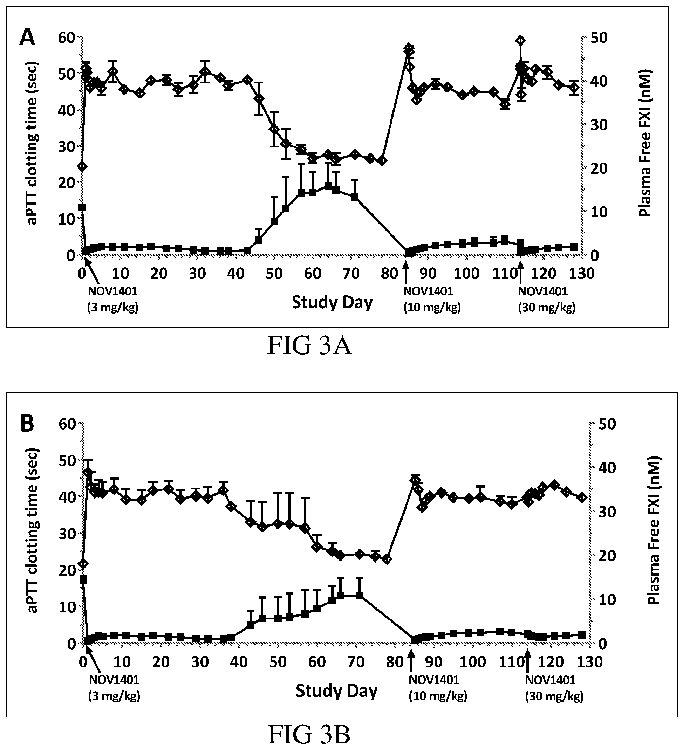

**Figures 3A-B** show the effect of multiple i.v. (A; N=2) or s.c. (B; N=2) doses of 3 mg/kg, 10 mg/kg and 30 mg/kg NOV1401 on plasma free FXI (squares) and relationship to aPTT (diamonds) in cynomolgus monkeys. A single dose of 3 mg/kg reduced free FXI by approximately 90% for 5-6 weeks. All doses tested reduced free FXI to a similar extent, and the higher doses tested did not seem to increase the magnitude of reduction of free FXI observed at the 3 mg/kg dose.

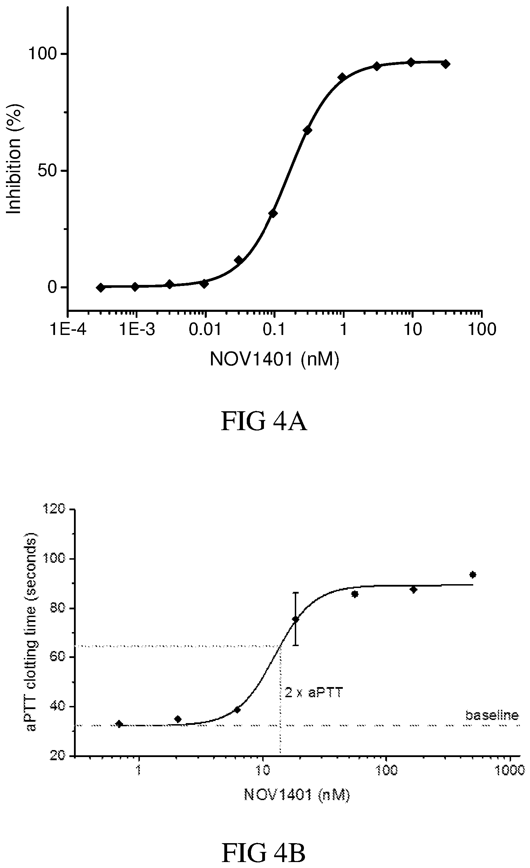

**Figures 4A-C** show compound response curves of an anti-FXI/FXIa antibody. **Figure 4A** shows inhibition of Factor XIa activity by NOV1401. Representative compound response curve of antibody NOV1401 inhibiting the enzymatic activity of full length human FXIa. The assay measures the cleavage of a fluorescently labelled peptide as is described in example 2. Using the non-linear curve fit with a logistic fit model [y = A2+ (A1- A2)/ (1+ (x/ IC50)^ p), where y is the %-inhibition at the inhibitor concentration, x. A1 is the lowest inhibition value, and A2 the maximum inhibition value.

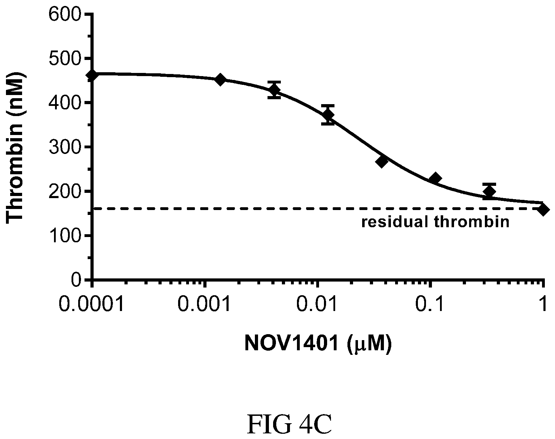

The exponent, p, is the Hill coefficient] on this representative data set leads to an $IC_{50}$ value of 160 pM. **Figure 4B** shows an aPTT compound response curve. Representative compound response curve of antibody NOV1401 prolonging coagulation time in the aPTT assay using pooled human plasma. The assay measures the time to coagulation after initiating the intrinsic clotting cascade in presence of different concentrations of NOV1401, as described in Example 3. The black line represents a fit using a logistics non-linear fit model. The dotted line represents the baseline coagulation time of pooled human plasma in absence of NOV1401. The baseline coagulation time is 32.3 seconds, and is indicated with a grey dashed line in the graph. The grey dotted line indicates the antibody concentration at which the clotting time is doubled compared to baseline, *i.e.* the 2 x aPTT value, which is 14 nM. **Figure 4C** shows a TGA response curve. A representative compound response curve of antibody NOV1401 inhibiting thrombin generation in the TGA with pooled human plasma is shown. The assay measures the effects of different concentrations of NOV1401 on FXI-dependent thrombin generation through the so-called thrombin→FXIa feed-forward loop that can be triggered by very low tissue factor (TF) concentrations as described in Example 3. The black line represents a fit using a four-parameter dose-response curve model. The dotted line represents the residual thrombin concentration due to thrombin generation induced by small amounts of TF. An $IC_{50}$ value of 24 nM and a residual thrombin concentration of 159 nM (dotted line) were calculated for this compound response curve.

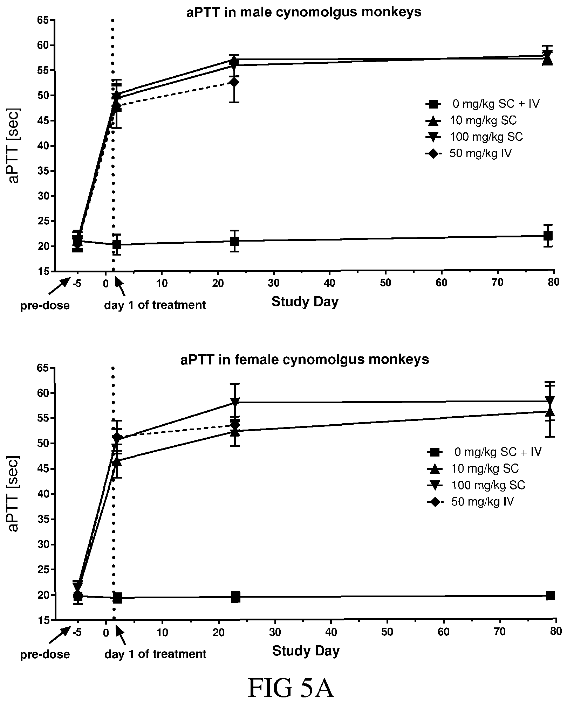

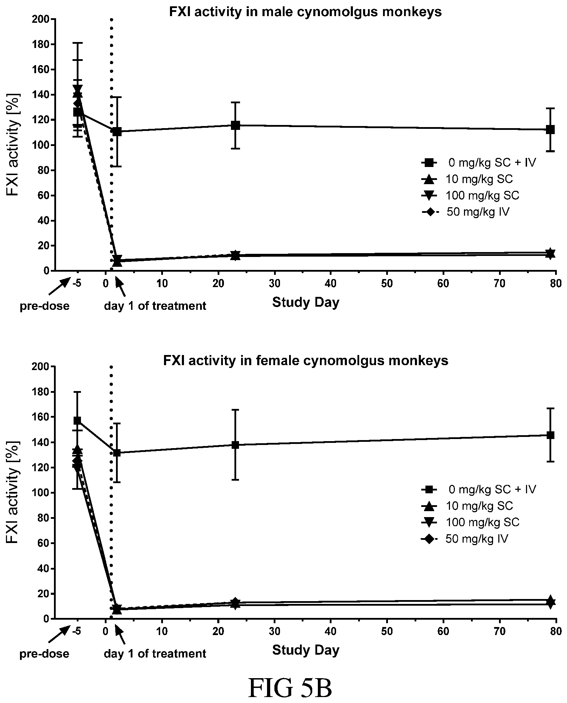

**Figures 5A-B** show the effect of weekly NOV1401 doses of 10 mg/kg (N=3) and 100 mg/kg (N=5) s.c. for 13 weeks (14 doses) or at 50 mg/kg (N=3) i.v. for 4 weeks (5 doses) on aPTT and FXI activity (FXI:C). **Figure 5A** shows the effect on aPTT, measured on study days 2, 23, and 79. aPTT increased by 2.1- to 3-fold in all animals receiving NOV1401 and remained elevated throughout the dosing phase of the study. No dose-dependency was observed and no gender-related differences were noted. **Figure 5B** shows the effect on FXI:C, measured on study days 2, 23 and 79 and depicted as percent of plasma FXI activity. FXI:C decreased in all animals receiving NOV1401 to levels of 5-12% and remained at these levels throughout the dosing phase of the study. No dose-dependency was observed and no gender-related differences were noted.

## DETAILED DESCRIPTION

The present disclosure is based, in part, on the discovery of antibody molecules (e.g., NOV1090 or NOV1401) that specifically bind to FXIa and inhibit its biological activities for use in preventing or reducing the risk of thrombosis or thromboembolism, such as stroke, in subjects with ESRD, such as subjects with ESRD undergoing hemodialysis. In particular aspects, provided herein are methods for preventing or reducing the risk of thrombosis or thromboembolism, such as stroke or systemic embolism, in subjects with AF and ESRD, such as subjects with ESRD undergoing hemodialysis. In specific aspects, such subjects are at high risk of bleeding.

The present disclosure relates to uses of both full IgG format antibodies (e.g., human antibodies) as well as antigen binding fragments thereof, such as Fab fragments (e.g., antibodies NOV1090 and NOV1401).

### Factor XI

FXI holds important roles in both intrinsic and extrinsic coagulation pathways and in bridging the initiation and amplification phases of plasmatic hemostasis. Both Factor XIIa and thrombin can activate FXI, resulting in a sustained thrombin generation and fibrinolysis inhibition. FXI plays a minor role in normal hemostasis in a high tissue factor environment "after vessel injury" whereas it appears to play a key role in thrombosis. Severe Factor XI deficiency is associated with a lower incidence of ischemic stroke and venous thromboembolic events (Salomon et al 2008; Salomon, et al. (2011) Thromb Haemost.; 105:269-73). Bleeding manifestations in subjects with severe factor XI deficiency are infrequent, often mild, injury-induced and affect preferably tissues with increased fibrinolytic activity such as the oral mucosa, nasal mucosa and urinary tract (Salomon et al 2011). Bleeding in critical organs is extremely rare or not existing.

Plasma coagulation is a sequential process by which coagulation factors in the blood interact and are activated, ultimately resulting in fibrin generation and clot formation. In the classical cascade model of coagulation, the process of fibrin generation can be initiated by two distinct pathways, *i.e.,* the intrinsic and the extrinsic pathway, respectively (Mackman, 2008).

In the extrinsic pathway, vessel injury allows extravascular tissue factor (TF) to interact with and activate factor VII (FVII), which sequentially leads to the activation of factor X and prothrombin. The active thrombin ultimately converts soluble fibrinogen into fibrin. The extrinsic pathway is central for hemostasis, interfering with coagulation factors in this pathway results in a risk of bleeding.

In the intrinsic pathway, factor XII may in some cases be activated by a process referred to as contact activation. Generation of activated factor XIIa leads to the sequential activations of factor XI and factor IX. As factor IXa activates factor X, the extrinsic and intrinsic pathways converge at this stage (at the common pathway). Thrombin activity is boosted by amplifying its own generation through a feed-forward loop in which thrombin activates factor XI independently of factor XII. This feed-forward loop contributes to sustained thrombus growth but is only minimally involved in hemostasis, as the strong activation by extravascular tissue factor is sufficient to clot formation. The intrinsic pathway therefore is not substantially involved in hemostasis (Gailani and Renné (2007) Arterioscler Thromb Vasc Biol. 2007, 27(12):2507-13, Müller, Gailiani, and Renné 2011).

Preclinical studies using a variety of approaches to inhibit FXI or FXIa across a variety of species have contributed to the validation of this target. FXI-/- mice are resistant to experimental venous (Wang, et al. (2006) J Thromb Haemost; 4:1982-8) and arterial (Wang, et al. (2005) J Thromb Haemost; 3:695-702) thrombosis. Treatment of mice with an antibody (Ab, 14E11) that blocks the activation of FXI by FXIIa resulted in inhibition of experimental thrombosis (Cheng, et al. (2010) Blood, 116:3981-9) and reduced cerebral infarct size in a mouse model of ischemic stroke (Leung, et al. (2012) Transl Stroke Res 2012; 3:381-9). In baboons administered an anti-FXI Ab that blocks binding and activation of FIX by FXIa, reduced growth of platelet-rich thrombi was observed on collagen-coated vascular grafts (Tucker, et al. (2009) Blood 2009; 113:936-44), and similar results were found with 14E11 in this model (Cheng 2010). Excessive bleeding was not noted in any of these studies.

Blocking FXI synthesis with antisense oligonucleotides in mice (Zhang, et al. (2010) Blood 2010; 116:4684-92), cynomolgus monkeys (Younis, et al. (2012) Blood 2012; 119:2401-8), and baboons (Crosby, et al. (2013) Arterioscler Thromb Vasc Biol 2013; 33:1670-8) resulted in antithrombotic and anticoagulant effects without excessive bleeding. Moreover, similar effects have been produced by blocking FXIa with low molecular weight inhibitors in venous and arterial models of thrombosis in rats (Schumacher, et al. (2007) Eur J Pharmacol 2007; 570:167-74) and rabbits (Wong, et al. (2011) J Thromb Thrombolysis 2011; 32:129-37).

Patients with severe FXI deficiency rarely bleed spontaneously and they show only mild trauma-induced bleeding, except in tissues with high fibrinolytic activity. The rarity of severe FXI deficiency necessitates the use of population studies for revealing the thrombotic profile of these patients relative to the general population. Notably, such studies report the incidence of ischemic stroke (Salomon 2008) and deep vein thrombosis (DVT) (Salomon, et al. (2011) Blood 2008; 111: 4113-17) to be reduced in these patients. Thus, the number of ischemic strokes (N = 1) observed in 115 patients with severe FXI deficiency was less (p < 0.003) than the expected incidence (N = 8.6) in the general population of Israel, while the incidence of DVT (N = 0) was lower (p < 0.019) in patients with severe FXI deficiency than expected in the control population (N = 4.7). Conversely, individuals with FXI levels above the 90th percentile had a two-fold risk of developing DVT (Meijers, et al. (2000) N Engl J Med. 2000; 342:696-701).

Recently, patients undergoing total knee replacement, a procedure that predisposes to DVT, were treated with FXI antisense therapy or standard of care (enoxaparin). The antisense group (300 mg) showed a 7-fold decreased incidence in venous thrombosis and fewer (not significant) bleeding events compared to standard of care (Büller et al, (2014) N Engl J Med. 372(3):232-40. doi: 10.1056/NEJMoa1405760. Epub 2014 Dec 7).

Taken together, the above studies strongly support FXI as a valid target for antithrombotic therapy.

**FXIa Antibodies & Antigen Binding Fragments**

The present disclosure provides antibodies that specifically bind to FXI and/or FXIa for use in treating, preventing or reducing the risk of thrombosis or thromboembolism, such as stroke. In some embodiments, the present disclosure provides antibodies that specifically bind to human, rabbit, and cynomolgus monkey FXI and/or FXIa. Antibodies of the present disclosure include, but are not limited to, the human monoclonal antibodies and Fabs, isolated as described in the Examples.

The present disclosure provides antibodies that specifically bind a FXI and/or FXIa protein (e.g., human, rabbit, and cynomolgus monkey FXI and/or FXIa), wherein the antibodies comprise a VH domain having an amino acid sequence of SEQ ID NOs: 9 and 29. The present disclosure also provides antibodies that specifically bind to a FXI and/or FXIa protein, wherein the antibodies comprise a VH CDR having an amino acid sequence of any one of the VH CDRs listed in Table *1, infra.* In particular, the present disclosure provides antibodies that specifically bind to an FXI and/or FXIa protein (e.g., human, rabbit, and cynomolgus monkey FXI and/or FXIa), wherein the antibodies comprise (or alternatively, consist of) one, two, three, or more VH CDRs having an amino acid sequence of any of the VH CDRs listed in Table 1, *infra.* (see PCT International Patent Application No. PCT/IB2016/053790 filed on June 24, 2016, and published as WO2016/207858, which is hereby incorporated by reference in its entirety).

The present disclosure provides antibodies that specifically bind to a FXIa protein, said antibodies comprising a VL domain having an amino acid sequence of SEQ ID NOs: 19 or 39, for use in the methods described herein (e.g., methods for reducing the risk of stroke and/or systemic embolism in subjects with ESRD and/or AF). The present disclosure also provides antibodies that specifically bind to an FXI and/or FXIa protein (e.g., human, rabbit, and cynomolgus monkey FXI and/or FXIa), said antibodies comprising a VL CDR having an amino acid sequence of any one of the VL CDRs listed in Table 1, *infra,* for use in the methods described herein (e.g., methods for reducing the risk of stroke and/or systemic embolism in subjects with ESRD and/or AF). In particular, the present disclosure provides antibodies that specifically bind to an FXIa protein *(e.g.,* human, rabbit, and cynomolgus monkey FXI and/or FXIa), said antibodies comprising (or alternatively, consisting of) one, two, three or more VL CDRs having an amino acid sequence of any of the VL CDRs listed in Table 1, *infra.*

Other antibodies for use in the methods described herein (e.g., methods for reducing the risk of stroke and/or systemic embolism in subjects with ESRD and/or AF) include amino acids that have been mutated, yet have at least 60, 70, 80, 85, 90 or 95 percent identity in the CDR regions with the CDR regions depicted in the sequences described in Table 1. In some embodiments, it includes mutant amino acid sequences wherein no more than 1, 2, 3, 4 or 5 amino acids have been mutated in the CDR regions when compared with the CDR regions depicted in the sequence described in Table 1.

**Table 1. Examples of FXIa Antibodies, Fabs and FXIa Proteins.**

| Sequence Description | Sequence Identifier (SEQ ID NO: ) | Amino acid or polynucleotide sequence |
|---|---|---|
| Human FXI full-length protein sequence (NCBI Reference Sequence: AAA51985) | 1 | MIFLYQVVHF ILFTSVSGEC VTQLLKDTCF EGGDITTVFT<br>PSAKYCQVVC TYHPRCLLFT FTAESPSEDP TRWFTCVLKD<br>SVTETLPRVN RTAAISGYSF KQCSHQISAC NKDIYVDLDM<br>KGINYNSSVA KSAQECQERC TDDVHCHFFT YATRQFPSLE<br>HRNICLLKHT QTGTPTRITK LDKVVSGFSL KSCALSNLAC<br>IRDIFPNTVF ADSNIDSVMA PDAFVSGRIC THHPGCLFFT<br>FFSQEWPKES QRNLCLLKTS ESGLPSTRIK KSKALSGFSL<br>QSCRHSIPVF CHSSFYHDTD FLGEELDIVA AKSHEACQKL<br>CTNAVRCQFF TYTPAQASCN EGKGKCYLKL SSNGSPTKIL<br>HGRGGISGYT LRLCKMDNEC TTKIKPRIVG GTASVRGEWP<br>WQVTLHTTSP TQRHLCGGSI IGNQWILTAA HCFYGVESPK<br>ILRVYSGILN QSEIKEDTSF FGVQEIIIHD QYKMAESGYD<br>IALLKLETTV NYTDSQRPIC LPSKGDRNVI YTDCWVTGWG<br>YRKLRDKIQN TLQKAKIPLV TNEECQKRYR GHKITHKMIC<br>AGYREGGKDA CKGDSGGPLS CKHNEVWHLV GITSWGEGCA<br>QRERPGVYTN VVEYVDWILE KTQAV |
| Human FXI full-length nucleotide sequence (NCBI | 2 | AGGCACACAG GCAAAATCAA GTTCTACATC TGTCCCTGTG<br>TATGTCACTT GTTTGAATAC GAAATAAAAT TAAAAAAATA<br>AATTCAGTGT ATTGAGAAAG CAAGCAATTC TCTCAAGGTA |

(continued)

| Sequence Description | Sequence Identifier (SEQ ID NO: ) | Amino acid or polynucleotide sequence |
|---|---|---|
| Reference Sequence: NM_000128.3) | | TATTTCTGAC ATACTAAGAT TTTAACGACT TTCACAAATA<br>TGCTGTACTG AGAGAGAATG TTACATAACA TTGAGAACTA<br>GTACAAGTAA ATATTAAAGT GAAGTGACCA TTTCCTACAC<br>AAGCTCATTC AGAGGAGGAT GAAGACCATT TTGGAGGAAG<br>AAAAGCACCC TTATTAAGAA TTGCAGCAAG TAAGCCAACA<br>AGGTCTTTTC AGGATGATTT TCTTATATCA AGTGGTACAT<br>TTCATTTTAT TTACTTCAGT TTCTGGTGAA TGTGTGACTC<br>AGTTGTTGAA GGACACCTGC TTTGAAGGAG GGGACATTAC<br>TACGGTCTTC ACACCAAGCG CCAAGTACTG CCAGGTAGTC<br>TGCACTTACC ACCCAAGATG TTTACTCTTC ACTTCACGG<br>CGGAATCACC ATCTGAGGAT CCCACCCGAT GGTTTACTTG<br>TGTCCTGAAA GACAGTGTTA CAGAAACACT GCCAAGAGTG<br>AATAGGACAG CAGCGATTTC TGGGTATTCT TTCAAGCAAT<br>GCTCACACCA AATAAGCGCT TGCAACAAAG ACATTTATGT<br>GGACCTAGAC ATGAAGGGCA TAAACTATAA CAGCTCAGTT<br>GCCAAGAGTG CTCAAGAATG CCAAGAAAGA TGCACGGATG<br>ACGTCCACTG CCACTTTTTC ACGTACGCCA CAAGGCAGTT<br>TCCCAGCCTG GAGCATCGTA ACATTTGTCT ACTGAAGCAC<br>ACCCAAACAG GGACACCAAC CAGAATAACG AAGCTCGATA<br>AAGTGGTGTC TGGATTTTCA CTGAAATCCT GTGCACTTTC<br>TAATCTGGCT TGTATTAGGG ACATTTTCCC TAATACGGTG<br>TTTGCAGACA GCAACATCGA CAGTGTCATG GCTCCCGATG<br>CTTTTGTCTG TGGCCGAATC TGCACTCATC ATCCCGGTTG<br>CTTGTTTTTT ACCTTCTTTT CCCAGGAATG GCCCAAAGAA<br>TCTCAAAGAA ATCTTTGTCT CCTTAAAACA TCTGAGAGTG<br>GATTGCCCAG TACACGCATT AAAAAGAGCA AAGCTCTTTC<br>TGGTTTCAGT CTACAAAGCT GCAGGCACAG CATCCCAGTG<br>TTCTGCCATT CTTCATTTTA CCATGACACT GATTTCTTGG<br>GAGAAGAACT GGATATTGTT GCTGCAAAAA GTCACGAGGC<br>CTGCCAGAAA CTGTGCACCA ATGCCGTCCG CTGCCAGTTT<br>TTTACCTATA CCCCAGCCCA AGCATCCTGC AACGAAGGGA<br>AGGGCAAGTG TTACTTAAAG CTTTCTTCAA ACGGATCTCC<br>AACTAAAATA CTTCACGGGA GAGGAGGCAT CTCTGGATAC<br>ACATTAAGGT TGTGTAAAAT GGATAATGAG TGTACCACCA<br>AAATCAAGCC CAGGATCGTT GGAGGAACTG CGTCTGTTCG<br>TGGTGAGTGG CCGTGGCAGG TGACCCTGCA CACAACCTCA<br>CCCACTCAGA GACACCTGTG TGGAGGCTCC ATCATTGGAA<br>ACCAGTGGAT ATTAACAGCC GCTCACTGTT TCTATGGGGT<br>AGAGTCACCT AAGATTTGC GTGTCTACAG TGGCATTTTA<br>AATCAATCTG AAATAAAAGA GGACACATCT TTCTTTGGGG<br>TTCAAGAAAT AATAATCCAT GATCAGTATA AAATGGCAGA |

(continued)

| Sequence Description | Sequence Identifier (SEQ ID NO: ) | Amino acid or polynucleotide sequence |
|---|---|---|
| | | AAGCGGGTAT GATATTGCCT TGTTGAAACT GGAAACCACA GTGAATTACA CAGATTCTCA ACGACCCATA TGCCTGCCTT CCAAAGGAGA TAGAAATGTA ATATACACTG ATTGCTGGGT GACTGGATGG GGGTACAGAA AACTAAGAGA CAAAATACAA AATACTCTCC AGAAAGCCAA GATACCCTTA GTGACCAACG AAGAGTGCCA GAAGAGATAC AGAGGACATA AAATAACCCA TAAGATGATC TGTGCCGGCT ACAGGGAAGG AGGGAAGGAC GCTTGCAAGG GAGATTCGGG AGGCCCTCTG TCCTGCAAAC ACAATGAGGT CTGGCATCTG GTAGGCATCA CGAGCTGGGG CGAAGGCTGT GCTCAAAGGG AGCGGCCAGG TGTTTACACC AACGTGGTCG AGTACGTGGA CTGGATTCTG GAGAAAACTC AAGCAGTGTG AATGGGTTCC CAGGGGCCAT TGGAGTCCCT GAAGGACCCA GGATTTGCTG GGAGAGGGTG TTGAGTTCAC TGTGCCAGCA TGCTTCCTCC ACAGTAACAC GCTGAAGGGG CTTGGTGTTT GTAAGAAAAT GCTAGAAGAA AACAAACTGT CACAAGTTGT TATGTCCAAA ACTCCCGTTC TATGATCGTT GTAGTTTGTT TGAGCATTCA GTCTCTTTGT TTTTGATCAC GCTTCTATGG AGTCCAAGAA TTACCATAAG GCAATATTTC TGAAGATTAC TATATAGGCA GATATAGCAG AAAATAACCA AGTAGTGGCA GTGGGGATCA GGCAGAAGAA CTGGTAAAAG AAGCCACCAT AAATAGATTT GTTCGATGAA AGATGAAAAC TGGAAGAAAG GAGAACAAAG ACAGTCTTCA CCATTTTGCA GGAATCTACA CTCTGCCTAT GTGAACACAT TTCTTTTGTA AAGAAAGAAA TTGATTGCAT TTAATGGCAG ATTTTCAGAA TAGTCAGGAA TTCTTGTCAT TTCCATTTTA AAATATATAT TAAAAAAAAT CAGTTCGAGT AGACACGAGC TAAGAGTGAA TGTGAAGATA ACAGAATTTC TGTGTGGAAG AGGATTACAA GCAGCAATTT ACCTGGAAGT GATACCTTAG GGGCAATCTT GAAGATACAC TTTCCTGAAA AATGATTTGT GATGGATTGT ATATTTATTT AAAATATCTT GGGAGGGGAG GCTGATGGAG ATAGGGAGCA TGCTCAAACC TCCCTAAGAC AAGCTGCTGC TGTGACTATG GGCTCCCAAA GAGCTAGATC GTATATTTAT TTGACAAAAA TCACCATAGA CTGCATCCAT ACTACAGAGA AAAACAATT AGGGCGCAAA TGGATAGTTA CAGTAAAGTC TTCAGCAAGC AGCTGCCTGT ATTCTAAGCA CTGGGATTTT CTGTTTCGTG CAAATATTTA TCTCATTATT GTTGTGATCT AGTTCAATAA CCTAGAATTT GAATTGTCAC CACATAGCTT TCAATCTGTG CCAACAACTA TACAATTCAT CAAGTGTG |
| **NOV1090** | | |

(continued)

| Sequence Description | | Sequence Identifier (SEQ ID NO: ) | Amino acid or polynucleotide sequence |
|---|---|---|---|
| | HCDR1 (Kabat) | 3 | TAAMS |
| | HCDR2 (Kabat) | 4 | GISGSGSSTYYADSVKG |
| | HCDR3 (Kabat) | 5 | ELSYLYSGYYFDY |
| | HCDR1 (Chothia) | 6 | GFTFSTA |
| | HCDR2 (Chothia) | 7 | SGSGSS |
| | HCDR3 (Chothia) | 8 | ELSYLYSGYYFDY |
| | HCDR1 (IMGT) | 43 | GFTFSTAA |
| | HCDR2 (IMGT) | 44 | ISGSGSST |
| | HCDR3 (IMGT) | 45 | ARELSYLYSGYYFDY |
| | HCDR1 (Combined) | 46 | GFTFSTAAMS |
| | HCDR2 (Combined) | 4 | GISGSGSSTYYADSVKG |
| | HCDR3 (Combined) | 5 | ELSYLYSGYYFDY |
| VH | | 9 | QVQLLESGGGLVQPGGSLRLSCAASGFTFSTAAMSWVRQAPGK GLEWVSGISGSGSSTYYADSVKGRFTISRDNSKNTLYLQMNSL RAEDTAVYYCARELSYLYSGYYFDYWGQGTLVTVSS |
| DNA encoding VH | | 10 | CAGGTGCAATTGCTGGAAAGCGGCGGTGGCCTGGTGCAGCCGG GTGGCAGCCTGCGTCTGAGCTGCGCGGCGTCCGGATTCACCTT TTCTACTGCTGCTATGTCTTGGGTGCGCCAGGCCCCGGGCAAA GGTCTCGAGTGGGTTTCCGGTATCTCTGGTTCTGGTTCTTCTA CCTACTATGCGGATAGCGTGAAAGGCCGCTTTACCATCAGCCG CGATAATTCGAAAAACACCCTGTATCTGCAAATGAACAGCCTG CGTGCGGAAGATACGGCCGTGTATTATTGCGCGCGTGAACTGT CTTACCTGTACTCTGGTTACTACTTCGATTACTGGGGCCAAGG CACCCTGGTGACTGTTAGCTCA |

(continued)

| Sequence Description | Sequence Identifier (SEQ ID NO: ) | Amino acid or polynucleotide sequence |
|---|---|---|
| Heavy Chain | 11 | QVQLLESGGGLVQPGGSLRLSCAASGFTFSTAAMSWVRQAPGK GLEWVSGISGSGSSTYYADSVKGRFTISRDNSKNTLYLQMNSL RAEDTAVYYCARELSYLYSGYYFDYWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTK VDKRVEPKSCDKTHTCPPCPAPEAAGGPSVFLFPPKPKDTLMI SRTPEVTCVVVDVSHEDPEVKFNWYVDGVEVHNAKTKPREEQY NSTYRVVSVLTVLHQDWLNGKEYKCKVSNKALPAPIEKTISKA KGQPREPQVYTLPPSREEMTKNQVSLTCLVKGFYPSDIAVEWE SNGQPENNYKTTPPVLDSDGSFFLYSKLTVDKSRWQQGNVFSC SVMHEALHNHYTQKSLSLSPGK |
| DNA encoding Heavy Chain | 12 | CAGGTGCAATTGCTGGAAAGCGGCGGTGGCCTGGTGCAGCCGG GTGGCAGCCTGCGTCTGAGCTGCGCGGCGTCCGGATTCACCTT TTCTACTGCTGCTATGTCTTGGGTGCGCCAGGCCCCGGGCAAA GGTCTCGAGTGGGTTTCCGGTATCTCTGGTTCTGGTTCTTCTA CCTACTATGCGGATAGCGTGAAAGGCCGCTTTACCATCAGCCG CGATAATTCGAAAAACACCCTGTATCTGCAAATGAACAGCCTG CGTGCGGAAGATACGGCCGTGTATTATTGCGCGCGTGAACTGT |

(continued)

| Sequence Description | Sequence Identifier (SEQ ID NO: ) | Amino acid or polynucleotide sequence |
|---|---|---|
| | | CTTACCTGTACTCTGGTTACTACTTCGATTACTGGGGCCAAGG CACCCTGGTGACTGTTAGCTCAGCCTCCACCAAGGGTCCATCG GTCTTCCCCCTGGCACCCTCCTCCAAGAGCACCTCTGGGGGCA CAGCGGCCCTGGGCTGCCTGGTCAAGGACTACTTCCCCGAACC GGTGACGGTGTCGTGGAACTCAGGCGCCCTGACCAGCGGCGTG CACACCTTCCCGGCTGTCCTACAGTCCTCAGGACTCTACTCCC TCAGCAGCGTGGTGACCGTGCCCTCCAGCAGCTTGGGCACCCA GACCTACATCTGCAACGTGAATCACAAGCCCAGCAACACCAAG GTGGACAAGAGAGTTGAGCCCAAATCTTGTGACAAAACTCACA CATGCCCACCGTGCCCAGCACCTGAAGCAGCGGGGGGGACCGTC AGTCTTCCTCTTCCCCCCAAAACCCAAGGACACCCTCATGATC TCCCGGACCCCTGAGGTCACATGCGTGGTGGTGGACGTGAGCC ACGAAGACCCTGAGGTCAAGTTCAACTGGTACGTGGACGGCGT GGAGGTGCATAATGCCAAGACAAAGCCGCGGGAGGAGCAGTAC AACAGCACGTACCGGGTGGTCAGCGTCCTCACCGTCCTGCACC AGGACTGGCTGAATGGCAAGGAGTACAAGTGCAAGGTCTCCAA CAAAGCCCTCCCAGCCCCCATCGAGAAAACCATCTCCAAAGCC AAAGGGCAGCCCCGAGAACCACAGGTGTACACCCTGCCCCCCAT CCCGGGAGGAGATGACCAAGAACCAGGTCAGCCTGACCTGCCT GGTCAAAGGCTTCTATCCCAGCGACATCGCCGTGGAGTGGGAG AGCAATGGGCAGCCGGAGAACAACTACAAGACCACGCCTCCCG TGCTGGACTCCGACGGCTCCTTCTTCCTCTACAGCAAGCTCAC CGTGGACAAGAGCAGGTGGCAGCAGGGGAACGTCTTCTCATGC TCCGTGATGCATGAGGCTCTGCACAACCACTACACGCAGAAGA GCCTCTCCCTGTCTCCGGGTAAA |
| LCDR1 (Kabat) | 13 | SGSSSNIGSNDVS |
| LCDR2 (Kabat) | 14 | KNYNRPS |
| LCDR3 (Kabat) | 15 | SAWDQRQFDVV |
| LCDR1 (Chothia) | 16 | SSSNIGSND |
| LCDR2 (Chothia) | 17 | KNY |
| LCDR3 (Chothia) | 18 | WDQRQFDV |
| LCDR1 (IMGT) | 47 | SSNIGSND |
| LCDR2 (IMGT) | 37 | KNY |

(continued)

| Sequence Description | Sequence Identifier (SEQ ID NO: ) | Amino acid or polynucleotide sequence |
|---|---|---|
| LCDR3 (IMGT) | 15 | SAWDQRQFDVV |
| LCDR1 (Combined) | 33 | SGSSSNIGSNDVS |
| LCDR2 (Combined) | 14 | KNYNRPS |
| LCDR3 (Combined) | 15 | SAWDQRQFDVV |
| VL | 19 | DIVLTQPPSVSGAPGQRVTISCSGSSSNIGSNDVSWYQQLPGT APKLLIYKNYNRPSGVPDRFSGSKSGTSASLAITGLQAEDEAD YYCSAWDQRQFDVVFGGGTKLTVL |
| DNA encoding VL | 20 | GATATCGTGCTGACCCAGCCGCCGAGCGTGAGCGGTGCACCGG GCCAGCGCGTGACCATTAGCTGTAGCGGCAGCAGCAGCAACAT TGGTTCTAACGACGTGTCTTGGTACCAGCAGCTGCCGGGCACG GCGCCGAAACTGCTGATCTACAAAAACTACAACCGCCCGAGCG GCGTGCCGGATCGCTTTAGCGGATCCAAAAGCGGCACCAGCGC CAGCCTGGCGATTACCGGCCTGCAAGCAGAAGACGAAGCGGAT TATTACTGCTCTGCTTGGGACCAGCGTCAGTTCGACGTTGTGT TTGGCGGCGGCACGAAGTTAACCGTCCTA |
| Light Chain | 21 | DIVLTQPPSVSGAPGQRVTISCSGSSSNIGSNDVSWYQQLPGT APKLLIYKNYNRPSGVPDRFSGSKSGTSASLAITGLQAEDEAD YYCSAWDQRQFDVVFGGGTKLTVLGQPKAAPSVTLFPPSSEEL QANKATLVCLISDFYPGAVTVAWKADSSPVKAGVETTTPSKQS NNKYAASSYLSLTPEQWKSHRSYSCQVTHEGSTVEKTVAPTEC S |

(continued)

| Sequence Description | Sequence Identifier (SEQ ID NO: ) | Amino acid or polynucleotide sequence |
|---|---|---|
| DNA encoding Light Chain | 22 | GATATCGTGCTGACCCAGCCGCCGAGCGTGAGCGGTGCACCGG GCCAGCGCGTGACCATTAGCTGTAGCGGCAGCAGCAGCAACAT TGGTTCTAACGACGTGTCTTGGTACCAGCAGCTGCCGGGCACG GCGCCGAAACTGCTGATCTACAAAAACTACAACCGCCCGAGCG GCGTGCCGGATCGCTTTAGCGGATCCAAAAGCGGCACCAGCGC CAGCCTGGCGATTACCGGCCTGCAAGCAGAAGACGAAGCGGAT TATTACTGCTCTGCTTGGGACCAGCGTCAGTTCGACGTTGTGT TTGGCGGCGGCACGAAGTTAACCGTCCTAGGTCAGCCCAAGGC TGCCCCCTCGGTCACTCTGTTCCCGCCCTCCTCTGAGGAGCTT CAAGCCAACAAGGCCACACTGGTGTGTCTCATAAGTGACTTCT ACCCGGGAGCCGTGACAGTGGCCTGGAAGGCAGATAGCAGCCC CGTCAAGGCGGGAGTGGAGACCACCACACCCTCCAAACAAAGC AACAACAAGTACGCGGCCAGCAGCTATCTGAGCCTGACGCCTG AGCAGTGGAAGTCCCACAGAAGCTACAGCTGCCAGGTCACGCA TGAAGGGAGCACCGTGGAGAAGACAGTGGCCCCTACAGAATGT TCA |
| **NOV1401** | | |
| | HCDR1 (Kabat) | 23 | TAAMS |
| | HCDR2 (Kabat) | 24 | GISGSGSSTYYADSVKG |
| | HCDR3 (Kabat) | 25 | ELSYLYSGYYFDY |
| | HCDR1 (Chothia) | 26 | GFTFSTA |
| | HCDR2 (Chothia) | 27 | SGSGSS |
| | HCDR3 (Chothia) | 28 | ELSYLYSGYYFDY |
| | HCDR1 (IMGT) | 43 | GFTFSTAA |
| | HCDR2 (IMGT) | 44 | ISGSGSST |
| | HCDR3 (IMGT) | 45 | ARELSYLYSGYYFDY |
| | HCDR1 (Combined) | 46 | GFTFSTAAMS |
| | HCDR2 (Combined) | 4 | GISGSGSSTYYADSVKG |
| | HCDR3 (Combined) | 5 | ELSYLYSGYYFDY |

(continued)

| Sequence Description | Sequence Identifier (SEQ ID NO: ) | Amino acid or polynucleotide sequence |
|---|---|---|
| VH | 29 | QVQLLESGGGLVQPGGSLRLSCAASGFTFSTAAMSWVRQAPGK GLEWVSGISGSGSSTYYADSVKGRFTISRDNSKNTLYLQMNSL RAEDTAVYYCARELSYLYSGYYFDYWGQGTLVTVSS |
| DNA encoding VH | 30 | CAGGTGCAGCTGCTGGAATCAGGCGGCGGACTGGTGCAGCCTG GCGGTAGCCTGAGACTGAGCTGCGCTGCTAGTGGCTTCACCTT TAGCACCGCCGCTATGAGCTGGGTTCGACAGGCCCCAGGGAAA GGCCTCGAGTGGGTCTCAGGGATTAGCGGTAGCGGCTCTAGCA CCTACTACGCCGATAGCGTGAAGGGCCGGTTCACTATCTCTAG GGATAACTCTAAGAACACCCTGTACCTGCAGATGAATAGCCTG AGAGCCGAGGACACCGCCGTCTACTACTGCGCTAGAGAGCTGA GCTACCTGTATAGCGGCTACTACTTCGACTACTGGGGTCAAGG CACCCTGGTCACCGTGTCTAGC |
| Heavy Chain | 31 | QVQLLESGGGLVQPGGSLRLSCAASGFTFSTAAMSWVRQAPGK GLEWVSGISGSGSSTYYADSVKGRFTISRDNSKNTLYLQMNSL RAEDTAVYYCARELSYLYSGYYFDYWGQGTLVTVSSASTKGPS VFPLAPSSKSTSGGTAALGCLVKDYFPEPVTVSWNSGALTSGV HTFPAVLQSSGLYSLSSVVTVPSSSLGTQTYICNVNHKPSNTK VDKRVEPKSCDKTHTCPPCPAPELLGGPSVFLFPPKPKDTLMI SRTPEVTCVVVAVSHEDPEVKFNWYVDGVEVHNAKTKPREEQY NSTYRVVSVLTVLHQDWLNGKEYKCKVSNKALAAPIEKTISKA KGQPREPQVYTLPPSREEMTKNQVSLTCLVKGFYPSDIAVEWE SNGQPENNYKTTPPVLDSDGSFFLYSKLTVDKSRWQQGNVFSC SVMHEALHNHYTQKSLSLSPGK |
| DNA encoding Heavy Chain | 32 | CAGGTGCAGCTGCTGGAATCAGGCGGCGGACTGGTGCAGCCTG GCGGTAGCCTGAGACTGAGCTGCGCTGCTAGTGGCTTCACCTT TAGCACCGCCGCTATGAGCTGGGTTCGACAGGCCCCAGGGAAA GGCCTCGAGTGGGTCTCAGGGATTAGCGGTAGCGGCTCTAGCA CCTACTACGCCGATAGCGTGAAGGGCCGGTTCACTATCTCTAG GGATAACTCTAAGAACACCCTGTACCTGCAGATGAATAGCCTG AGAGCCGAGGACACCGCCGTCTACTACTGCGCTAGAGAGCTGA GCTACCTGTATAGCGGCTACTACTTCGACTACTGGGGTCAAGG CACCCTGGTCACCGTGTCTAGCGCTAGCACTAAGGGCCCCTCC GTGTTCCCTCTGGCCCCTTCCAGCAAGTCTACCTCCGGCGGCA CAGCTGCTCTGGGCTGCCTGGTCAAGGACTACTTCCCTGAGCC TGTGACAGTGTCCTGGAACTCTGGCGCCCTGACCTCTGGCGTG CACACCTTCCCTGCCGTGCTGCAGTCCTCCGGCCTGTACTCCC TGTCCTCCGTGGTCACAGTGCCTTCAAGCAGCCTGGGCACCCA GACCTATATCTGCAACGTGAACCACAAGCCTTCCAACACCAAG GTGGACAAGCGGGTGGAGCCTAAGTCCTGCGACAAGACCCACA |

(continued)

| Sequence Description | Sequence Identifier (SEQ ID NO: ) | Amino acid or polynucleotide sequence |
|---|---|---|
| | | CCTGTCCTCCCTGCCCTGCTCCTGAACTGCTGGGCGGCCCTTC TGTGTTCCTGTTCCCTCCAAAGCCCAAGGACACCCTGATGATC TCCCGGACCCCTGAAGTGACCTGCGTGGTGGTGGCCGTGTCCC ACGAGGATCCTGAAGTGAAGTTCAATTGGTACGTGGACGGCGT GGAGGTGCACAACGCCAAGACCAAGCCTCGGGAGGAACAGTAC AACTCCACCTACCGGGTGGTGTCCGTGCTGACCGTGCTGCACC AGGACTGGCTGAACGGCAAAGAGTACAAGTGCAAAGTCTCCAA CAAGGCCCTGGCCGCCCCTATCGAAAAGACAATCTCCAAGGCC AAGGGCCAGCCTAGGGAACCCCAGGTGTACACCCTGCCACCCA GCCGGGAGGAAATGACCAAGAACCAGGTGTCCCTGACCTGTCT GGTCAAGGGCTTCTACCCTTCCGATATCGCCGTGGAGTGGGAG TCTAACGGCCAGCCTGAGAACAACTACAAGACCACCCCTCCTG TGCTGGACTCCGACGGCTCCTTCTTCCTGTACTCCAAACTGAC CGTGGACAAGTCCCGGTGGCAGCAGGGCAACGTGTTCTCCTGC TCCGTGATGCACGAGGCCCTGCACAACCACTACACCCAGAAGT CCCTGTCCCTGTCTCCCGGCAAG |
| LCDR1 (Kabat) | 33 | SGSSSNIGSNDVS |
| LCDR2 (Kabat) | 34 | KNYNRPS |
| LCDR3 (Kabat) | 35 | SAWDQRQFDVV |
| LCDR1 (Chothia) | 36 | SSSNIGSND |
| LCDR2 (Chothia) | 37 | KNY |
| LCDR3 (Chothia) | 38 | WDQRQFDV |
| LCDR1 (IMGT) | 47 | SSNIGSND |
| LCDR2 (IMGT) | 37 | KNY |
| LCDR3 (IMGT) | 15 | SAWDQRQFDVV |
| LCDR1 (Combined) | 33 | SGSSSNIGSNDVS |
| LCDR2 (Combined) | 14 | KNYNRPS |
| LCDR3 (Combined) | 15 | SAWDQRQFDVV |

(continued)

| Sequence Description | Sequence Identifier (SEQ ID NO: ) | Amino acid or polynucleotide sequence |
|---|---|---|
| VL | 39 | QSVLTQPPSASGTPGQRVTISCSGSSSNIGSNDVSWYQQLPGT APKLLIYKNYNRPSGVPDRFSGSKSGTSASLAISGLQSEDEAD YYCSAWDQRQFDVVFGGGTKLTVL |
| DNA encoding VL | 40 | CAGTCAGTCCTGACTCAGCCCCCTAGCGCTAGTGGCACCCCTG GTCAAAGAGTGACTATTAGCTGTAGCGGCTCTAGCTCTAATAT CGGCTCTAACGACGTCAGCTGGTATCAGCAGCTGCCCGGCACC GCCCCTAAGCTGCTGATCTATAAGAACTATAATAGGCCTAGCG GCGTGCCCGATAGGTTTAGCGGATCTAAATCAGGGACTTCTGC TAGTCTGGCTATTAGCGGCCTGCAGTCAGAGGACGAGGCCGAC TACTACTGTAGCGCCTGGGATCAGCGTCAGTTCGACGTGGTGT TCGGCGGAGGCACTAAGCTGACCGTGCTG |
| Light Chain | 41 | QSVLTQPPSASGTPGQRVTISCSGSSSNIGSNDVSWYQQLPGT APKLLIYKNYNRPSGVPDRFSGSKSGTSASLAISGLQSEDEAD YYCSAWDQRQFDVVFGGGTKLTVLGQPKAAPSVTLFPPSSEEL QANKATLVCLISDFYPGAVTVAWKADSSPVKAGVETTTPSKQS NNKYAASSYLSLTPEQWKSHRSYSCQVTHEGSTVEKTVAPTEC S |
| DNA encoding Light Chain | 42 | CAGTCAGTCCTGACTCAGCCCCCTAGCGCTAGTGGCACCCCTG GTCAAAGAGTGACTATTAGCTGTAGCGGCTCTAGCTCTAATAT CGGCTCTAACGACGTCAGCTGGTATCAGCAGCTGCCCGGCACC GCCCCTAAGCTGCTGATCTATAAGAACTATAATAGGCCTAGCG GCGTGCCCGATAGGTTTAGCGGATCTAAATCAGGGACTTCTGC TAGTCTGGCTATTAGCGGCCTGCAGTCAGAGGACGAGGCCGAC TACTACTGTAGCGCCTGGGATCAGCGTCAGTTCGACGTGGTGT TCGGCGGAGGCACTAAGCTGACCGTGCTGGGTCAACCTAAGGC TGCCCCCAGCGTGACCCTGTTCCCCCCCAGCAGCGAGGAGCTG CAGGCCAACAAGGCCACCCTGGTGTGCCTGATCAGCGACTTCT ACCCAGGCGCCGTGACCGTGGCCTGGAAGGCCGACAGCAGCCC CGTGAAGGCCGGCGTGGAGACCACCACCCCCAGCAAGCAGAGC AACAACAAGTACGCCGCCAGCAGCTACCTGAGCCTGACCCCCG AGCAGTGGAAGAGCCACAGGTCCTACAGCTGCCAGGTGACCCA CGAGGGCAGCACCGTGGAAAAGACCGTGGCCCCCAACCGAGTGC AGC |

Other antibodies for use in the methods described herein (e.g., methods for reducing the risk of stroke and/or systemic embolism in subjects with ESRD and/or AF) include those where the amino acids or nucleic acids encoding the amino acids have been mutated, yet have at least 60, 65, 70, 75, 80, 85, 90, or 95 percent identity to the sequences described in Table 1. Some embodiments include mutant amino acid sequences wherein no more than 1, 2, 3, 4 or 5 amino acids have been mutated in the variable regions when compared with the variable regions depicted in the sequence described in Table 1, while retaining substantially the same antigen binding activity.

Since each of these antibodies can bind to FXI and/or FXIa, the VH, VL, full length light chain, and full length heavy chain sequences (amino acid sequences and the nucleotide sequences encoding the amino acid sequences) can be "mixed and matched" to create other FXI and/or FXIa-binding antibodies of the present disclosure. Such "mixed and matched" FXI and/or FXIa-binding antibodies can be tested using the binding assays known in the art *(e.g.,* ELISAs, and other assays described in the Example section). When these chains are mixed and matched, a VH sequence from a particular VH/VL pairing should be replaced with a structurally similar VH sequence. Likewise a full length heavy chain sequence from a particular full length heavy chain / full length light chain pairing should be replaced with a structurally similar full length heavy chain sequence. Likewise, a VL sequence from a particular VH/VL pairing should be replaced with a structurally similar VL sequence. Likewise a full length light chain sequence from a particular full length heavy chain / full length light chain pairing should be replaced with a structurally similar full length light chain sequence.

Accordingly, in one aspect, for use in the methods described herein (e.g., methods for reducing the risk of stroke and/or systemic embolism in subjects with ESRD and/or AF), the present disclosure provides an isolated antibody or antigen binding region thereof having: a heavy chain variable domain comprising an amino acid sequence selected from the group consisting of SEQ ID NOs: 9 and 29, and a light chain variable domain comprising an amino acid sequence selected from the group consisting of SEQ ID NOs: 19 and 39, wherein the antibody specifically binds to FXI and/or FXIa (e.g., human, rabbit, and cynomolgus monkey FXIa).

More specifically, in certain aspects, the present disclosure provides an isolated antibody or antigen binding region thereof having a heavy chain variable domain and a light chain variable domain comprising amino acid sequences selected from SEQ ID NOs: 9 and 29; or 19 and 39, respectively.

In a specific embodiment for use in the methods described herein (e.g., methods for reducing the risk of stroke and/or systemic embolism in subjects with ESRD and/or AF), an antibody or antigen binding fragment thereof provided herein which specifically binds to human FXI and/or FXIa, comprises a heavy chain variable region comprising the amino acid sequence of SEQ ID NO: 9, and a light chain variable region comprising the amino acid sequence of SEQ ID NO: 19.

In a specific embodiment for use in the methods described herein (e.g., methods for reducing the risk of stroke and/or systemic embolism in subjects with ESRD and/or AF), an antibody or antigen binding fragment thereof provided herein which specifically binds to human FXI and/or FXIa, comprises a heavy chain variable region comprising the amino acid sequence of SEQ ID NO: 29, and a light chain variable region comprising the amino acid sequence of SEQ ID NO: 39.

In another aspect for use in the methods described herein, the present disclosure provides (i) an isolated antibody having: a full length heavy chain comprising an amino acid sequence that has been optimized for expression in a mammalian cell selected from the group consisting of SEQ ID NOs: 11 or 31, and a full length light chain comprising an amino acid sequence that has been optimized for expression in a mammalian cell selected from the group consisting of SEQ ID NOs: 21 or 41; or (ii) a functional protein comprising an antigen binding portion thereof. More specifically, in certain aspects, the present disclosure provides an isolated antibody or antigen binding region thereof having a heavy chain and a light chain comprising amino acid sequences selected from SEQ ID NOs: 11 and 31; or 19 and 39, respectively.

In a specific embodiment for use in the methods described herein, an antibody or antigen binding fragment thereof provided herein which specifically binds to human FXI and/or FXIa, comprises a heavy chain comprising the amino acid sequence of SEQ ID NO: 11, and a light chain comprising the amino acid sequence of SEQ ID NO: 21.

In a specific embodiment for use in the methods described herein, an antibody or antigen binding fragment thereof provided herein which specifically binds to human FXI and/or FXIa, comprises a heavy chain variable region comprising the amino acid sequence of SEQ ID NO: 31, and a light chain variable region comprising the amino acid sequence of SEQ ID NO: 41.

The terms "complementarity determining region," and "CDR," as used herein refer to the sequences of amino acids within antibody variable regions which confer antigen specificity and binding affinity. In general, there are three CDRs in each heavy chain variable region (HCDR1, HCDR2, HCDR3) and three CDRs in each light chain variable region (LCDR1, LCDR2, LCDR3).

The precise amino acid sequence boundaries of a given CDR can be readily determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), Lefranc et al., (2003) Dev. Comp. Immunol., 27, 55-77 ("IMGT" numbering scheme), or the "Combined" system.

For example, under Kabat, the CDR amino acid residues of antibody NOV1090 in the heavy chain variable domain (VH) are numbered 31-35 (HCDR1), 50-66 (HCDR2), and 99-111 (HCDR3); and the CDR amino acid residues in the light chain variable domain (VL) are numbered 22-35 (LCDR1), 51-57 (LCDR2), and 90-100 (LCDR3). Under Chothia the CDR amino acids in the VH are numbered 26-32 (HCDR1), 52-57 (HCDR2), and 99-111 (HCDR3); and the amino acid residues in VL are numbered 25-33 (LCDR1), 51-53 (LCDR2), and 92-99 (LCDR3). By combining the CDR definitions of both Kabat and Chothia, the CDRs consist of amino acid residues 26-35 (HCDR1), 50-66 (HCDR2), and 99-111 (HCDR3) in human VH and amino acid residues 22-35 (LCDR1), 51-57 (LCDR2), and 90-100 (LCDR3) in human VL. By combining the CDR definitions of both Kabat and Chothia, the "Combined" CDRs consist of amino acid residues 26-35 (HCDR1), 50-66 (HCDR2), and 99-108 (HCDR3) in human VH and amino acid residues 24-38 (LCDR1), 54-60 (LCDR2), and 93-101 (LCDR3) in human VL. As another example, under IMGT, the CDR amino acid residues in the heavy chain variable domain (VH) are numbered 26-33 (HCDR1), 51-58 (HCDR2), and 97-108 (HCDR3); and the CDR amino acid residues in the light chain variable domain (VL) are numbered 27-36 (LCDR1), 54-56 (LCDR2), and 93-101 (LCDR3). Table 1 provides exemplary Kabat, Chothia, Combined, and IMGT HCDR1, HCDR2, HCDR3, LCDR1, LCDR2, and LCDR3 for anti-FXI/FXIa antibodies, *e.g.,* NOV1090 and NOV1401.In another aspect, the present disclosure provides FXIa binding antibodies that comprise the heavy chain and light chain CDR1s, CDR2s, and CDR3s as described in Table 1, or combinations thereof. The amino acid sequences of the VH CDR1s of the antibodies are shown in SEQ ID NOs: 3 and 23. The amino acid sequences of the VH CDR2s of the antibodies and are shown in SEQ ID NOs: 4 and 24. The amino acid sequences of the VH CDR3s of the antibodies are shown in SEQ ID NOs: 5 and 25. The amino acid sequences of the VL CDR1s of the antibodies are shown in SEQ ID NOs: 13 and 33. The amino acid sequences of the VL CDR2s of the antibodies are shown in SEQ ID NOs: 14 and 34. The amino acid sequences of the VL CDR3s of the antibodies are shown in SEQ ID NOs: 15 and 35. These CDR regions are delineated using the Kabat system.

Alternatively, as defined using the Chothia system (Al-Lazikani et al., (1997) JMB 273,927-948), the amino acid sequences of the VH CDR1s of the antibodies are shown in SEQ ID NOs: 6 and 26. The amino acid sequences of the VH CDR2s of the antibodies and are shown in SEQ ID NOs: 7 and 27. The amino acid sequences of the VH CDR3s of the antibodies are shown in SEQ ID NOs: 8 and 28. The amino acid sequences of the VL CDR1s of the antibodies are shown in SEQ ID NOs: 16 and 36. The amino acid sequences of the VL CDR2s of the antibodies are shown in SEQ ID NOs: 17 and 37. The amino acid sequences of the VL CDR3s of the antibodies are shown in SEQ ID NOs: 18 and 38.

Alternatively, as defined using the Combined system, the amino acid sequences of the VH CDR1 of the antibodies are shown in SEQ ID NO: 46. The amino acid sequences of the VH CDR2 of the antibodies and are shown in SEQ ID NO: 4. The amino acid sequences of the VH CDR3 of the antibodies are shown in SEQ ID NO: 5. The amino acid sequences of the VL CDR1 of the antibodies are shown in SEQ ID NO: 33. The amino acid sequences of the VL CDR2 of the antibodies are shown in SEQ ID NO: 14. The amino acid sequences of the VL CDR3 of the antibodies are shown in SEQ ID NO: 15.

Alternatively, as defined using the IMGT numbering scheme, the amino acid sequences of the VH CDR1 of the antibodies are shown in SEQ ID NO: 43. The amino acid sequences of the VH CDR2 of the antibodies and are shown in SEQ ID NO: 44. The amino acid sequences of the VH CDR3 of the antibodies are shown in SEQ ID NO: 45. The amino acid sequences of the VL CDR1 of the antibodies are shown in SEQ ID NO: 47. The amino acid sequences of the VL CDR2 of the antibodies are shown in SEQ ID NO: 37. The amino acid sequences of the VL CDR3 of the antibodies are shown in SEQ ID NO: 15.

Given that each of these antibodies can bind to FXI and/or FXIa and that antigen-binding specificity is provided primarily by the CDR1, 2 and 3 regions, the VH CDR1, 2 and 3 sequences and VL CDR1, 2 and 3 sequences can be "mixed and matched" *(i.e.,* CDRs from different antibodies can be mixed and matched, although each antibody preferably contains a VH CDR1, 2 and 3 and a VL CDR1, 2 and 3 to create other FXI and/or FXIa binding molecules of the present disclosure. Such "mixed and matched" FXI and/or FXIa binding antibodies can be tested using the binding assays known in the art and those described in the Examples (*e.g.*, ELISAs, SET, BIACORE™ assays). When VH CDR sequences are mixed and matched, the CDR1, CDR2 and/or CDR3 sequence from a particular VH sequence should be replaced with a structurally similar CDR sequence(s). Likewise, when VL CDR sequences are mixed and matched, the CDR1, CDR2 and/or CDR3 sequence from a particular VL sequence should be replaced with a structurally similar CDR sequence(s). It will be readily apparent to the ordinarily skilled artisan that novel VH and VL sequences can be created by substituting one or more VH and/or VL CDR region sequences with structurally similar sequences from the CDR sequences shown herein for monoclonal antibodies of the present disclosure. In addition to the foregoing, in one embodiment, the antigen binding fragments of the antibodies described herein can comprise a VH CDR1, 2, and 3, or a VL CDR 1, 2, and 3, wherein the fragment binds to FXI and/or FXIa as a single variable domain.

In certain embodiments of the present disclosure, the antibodies or antigen binding fragments thereof may have the heavy and light chain sequences of the Fabs described in Table 1. More specifically, the antibody or antigen binding fragments thereof may have the heavy and light sequence of NOV1090 and NOV1401.

In other embodiments of the present disclosure the antibody or antigen binding fragment in that specifically binds FXI and/or FXIa comprises a heavy chain variable region CDR1, a heavy chain variable region CDR2, a heavy chain variable region CDR3, a light chain variable region CDR1, a light chain variable region CDR2, and a light chain variable region CDR3 as defined by Kabat and described in Table 1. In still other embodiments of the present disclosure the antibody or antigen binding fragment in that specifically binds FXI and/or FXIa comprises a heavy chain variable region CDR1, a heavy chain variable region CDR2, a heavy chain variable region CDR3, a light chain variable region CDR1, a light chain variable region CDR2, and a light chain variable region CDR3 as defined by Chothia and described in Table 1. In other embodiments, the antibody or antigen binding fragment in that specifically binds FXI and/or FXIa comprises a heavy chain variable region CDR1, a heavy chain variable region CDR2, a heavy chain variable region CDR3, a light chain variable region CDR1, a light chain variable region CDR2, and a light chain variable region CDR3 as defined by the Combined system and described in Table 1. In still other embodiments of the present disclosure the antibody or antigen binding fragment in that specifically binds FXI and/or FXIa comprises a heavy chain variable region CDR1, a heavy chain variable region CDR2, a heavy chain variable region CDR3, a light chain variable region CDR1, a light chain variable region CDR2, and a light chain variable region CDR3 as defined by IMGT and described in Table 1.

In a specific embodiment for use in the methods described herein, the present disclosure includes an antibody that specifically binds to FXI and/or FXIa comprising a heavy chain variable region CDR1 of SEQ ID NO: 3; a heavy chain variable region CDR2 of SEQ ID NO: 4; a heavy chain variable region CDR3 of SEQ ID NO: 5; a light chain variable region CDR1 of SEQ ID NO: 13; a light chain variable region CDR2 of SEQ ID NO: 14; and a light chain variable region CDR3 of SEQ ID NO: 15.

In a specific embodiment, the present disclosure includes an antibody that specifically binds to FXI and/or FXIa comprising a heavy chain variable region CDR1 of SEQ ID NO: 23; a heavy chain variable region CDR2 of SEQ ID NO: 24; a heavy chain variable region CDR3 of SEQ ID NO: 25; a light chain variable region CDR1 of SEQ ID NO: 33; a light chain variable region CDR2 of SEQ ID NO: 34; and a light chain variable region CDR3 of SEQ ID NO: 35.

In a specific embodiment, the present disclosure includes an antibody that specifically binds to FXI and/or FXIa comprising a heavy chain variable region CDR1 of SEQ ID NO: 6; a heavy chain variable region CDR2 of SEQ ID NO: 7; a heavy chain variable region CDR3 of SEQ ID NO: 8; a light chain variable region CDR1 of SEQ ID NO: 16; a light chain variable region CDR2 of SEQ ID NO: 17; and a light chain variable region CDR3 of SEQ ID NO: 18.

In a specific embodiment, the present disclosure includes an antibody that specifically binds to FXI and/or FXIa comprising a heavy chain variable region CDR1 of SEQ ID NO: 26; a heavy chain variable region CDR2 of SEQ ID NO: 27; a heavy chain variable region CDR3 of SEQ ID NO: 28; a light chain variable region CDR1 of SEQ ID NO: 36; a light chain variable region CDR2 of SEQ ID NO: 37; and a light chain variable region CDR3 of SEQ ID NO: 38.

In a specific embodiment, provided herein is an antibody that specifically binds to FXI and/or FXIa comprising a heavy chain variable region CDR1 of SEQ ID NO: 43; a heavy chain variable region CDR2 of SEQ ID NO: 44; a heavy chain variable region CDR3 of SEQ ID NO: 45; a light chain variable region CDR1 of SEQ ID NO: 47; a light chain variable region CDR2 of SEQ ID NO: 37 and a light chain variable region CDR3 of SEQ ID NO: 15.

In a specific embodiment, provided herein is an antibody that specifically binds to FXI and/or FXIa comprising a heavy chain variable region CDR1 of SEQ ID NO: 46; a heavy chain variable region CDR2 of SEQ ID NO: 4; a heavy chain variable region CDR3 of SEQ ID NO: 5; a light chain variable region CDR1 of SEQ ID NO: 33; a light chain variable region CDR2 of SEQ ID NO: 14 and a light chain variable region CDR3 of SEQ ID NO: 15.

In certain embodiments, the present disclosure includes antibodies or antigen binding fragments that specifically bind to FXI and/or FXIa as described in Table 1. In a specific embodiment for use in the methods described herein, the antibody, or antigen binding fragment, that binds FXI and/or FXIa is NOV1090 and NOV1401.

As used herein, a human antibody comprises heavy or light chain variable regions or full length heavy or light chains that are "the product of" or "derived from" a particular germline sequence if the variable regions or full length chains of the antibody are obtained from a system that uses human germline immunoglobulin genes. Such systems include immunizing a transgenic mouse carrying human immunoglobulin genes with the antigen of interest or screening a human immunoglobulin gene library displayed on phage with the antigen of interest. A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (*i.e.,* greatest % identity) to the sequence of the human antibody.

A human antibody that is "the product of" or "derived from" a particular human germline immunoglobulin sequence may contain amino acid differences as compared to the germline sequence, due to, for example, naturally occurring somatic mutations or intentional introduction of site-directed mutations. However, in the VH or VL framework regions, a selected human antibody typically is at least 90% identical in amino acids sequence to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 60%, 70%, 80%, 90%, or at least 95%, or even at least 96%, 97%, 98%, or 99% identical in amino acid sequence to the amino acid sequence encoded by the germline immunoglobulin gene.

Typically, a recombinant human antibody will display no more than 10 amino acid differences from the amino acid sequence encoded by the human germline immunoglobulin gene in the VH or VL framework regions. In certain cases, the human antibody may display no more than 5, or even no more than 4, 3, 2, or 1 amino acid difference from the amino acid sequence encoded by the germline immunoglobulin gene. Examples of human germline immunoglobulin genes include, but are not limited to the variable domain germline fragments described below, as well as DP47 and DPK9.

**Homologous antibodies**

In yet another embodiment for use in the methods described herein (e.g., methods for reducing or preventing the risk of stroke and/or system embolism in subjects with ESRD and/or AF), the present disclosure provides an antibody, or an antigen binding fragment thereof, comprising amino acid sequences that are homologous to the sequences described in Table 1 (*e.g.,* SEQ ID NOs: 29, 31, 39, or 41), and the antibody binds to an FXI and/or FXIa protein (*e.g.,* human, rabbit, and cynomolgus monkey FXIa), and retains the desired functional properties of those antibodies described in Table 1 such as NOV1090 and NOV1401. In specific aspects, such homologous antibodies retain the CDR amino acid sequences described in Table 1 (*e.g.,* Kabat CDRs, Chothia CDRs, IMGT CDRs, or Combined CDRs).

For example, the present disclosure provides an isolated antibody, or a functional antigen binding fragment thereof, comprising a heavy chain variable domain and a light chain variable domain, wherein the heavy chain variable domain comprises an amino acid sequence that is at least 80%, at least 90%, or at least 95% identical to an amino acid sequence selected from the group consisting of SEQ ID NOs: 9 and 29; the light chain variable domain comprises an amino acid sequence that is at least 80%, at least 90%, or at least 95% identical to an amino acid sequence selected from the group consisting of SEQ ID NOs: 19 and 39; and the antibody specifically binds to FXI and/or FXIa (*e.g.,* human, rabbit, and cynomolgus monkey FXIa). In one embodiment, an isolated antibody, or a functional antigen binding fragment thereof, comprises a heavy chain variable domain and a light chain variable domain, wherein the heavy chain variable domain comprises an amino acid sequence that is at least 80%, at least 90%, or at least 95% identical to the amino acid sequence of SEQ ID NO: 9; the light chain variable domain comprises an amino acid sequence that is at least 80%, at least 90%, or at least 95% identical to the amino acid sequence of SEQ ID NO: 19; and the antibody specifically binds to FXI and/or FXIa (*e.g.,* human, rabbit, and cynomolgus monkey FXIa). In one embodiment, an isolated antibody, or a functional antigen binding fragment thereof, comprises a heavy chain variable domain and a light chain variable domain, wherein the heavy chain variable domain comprises an amino acid sequence that is at least 80%, at least 90%, or at least 95% identical to the amino acid sequence of SEQ ID NO: 29; the light chain variable domain comprises an amino acid sequence that is at least 80%, at least 90%, or at least 95% identical to the amino acid sequence of SEQ ID NO: 39; and the antibody specifically binds to FXI and/or FXIa (*e.g.,* human, rabbit, and cynomolgus monkey FXIa). In certain aspects of the present disclosure the heavy and light chain sequences further comprise HCDR1, HCDR2, HCDR3, LCDR1, LCDR2, and LCDR3 sequences as defined by Kabat, for example SEQ ID NOs: 3, 4, 5, 13, 14, and 15, respectively. In certain other aspects of the present disclosure the heavy and light chain sequences further comprise HCDR1, HCDR2, HCDR3, LCDR1, LCDR2, and LCDR3 sequences as defined by Chothia, for example SEQ ID NOs: 6, 7, 8, 16, 17, and 18, respectively. In certain other aspects, the heavy and light chain sequences further comprise HCDR1, HCDR2, HCDR3, LCDR1, LCDR2, and LCDR3 sequences as defined by the Combined system, for example SEQ ID NOs: 46, 4, 5, 33, 14, and 15, respectively. In certain other aspects, the heavy and light chain sequences further comprise HCDR1, HCDR2, HCDR3, LCDR1, LCDR2, and LCDR3 sequences as defined by IMGT, for example SEQ ID NOs: 43, 44, 45, 47, 37, and 15, respectively.

In other embodiments for use in the methods described herein, the VH and/or VL amino acid sequences may be 50%, 60%, 70%, 80%, 90%, 95%, 96%, 97%, 98% or 99% identical to the sequences set forth in Table 1. In other embodiments, the VH and/or VL amino acid sequences may be identical except for an amino acid substitution in no more than 1,2,3,4 or 5 amino acid positions. An antibody having VH and VL regions having high (*i. e.,* 80% or greater) identity to the VH and VL regions of those described in Table 1 can be obtained by mutagenesis (*e.g.,* site-directed or PCR-mediated mutagenesis) of nucleic acid molecules encoding SEQ ID NOs: 10 or 30 and SEQ ID NOs: 20 and 40, respectively, followed by testing of the encoded altered antibody for retained function using the functional assays described herein.

In other embodiments for use in the methods described herein, the full length heavy chain and/or full length light chain amino acid sequences may be 50% 60%, 70%, 80%, 90%, 95%, 96%, 97%, 98% or 99% identical to the sequences set forth in Table 1 (*e.g.,* SEQ ID NOs: 11 and/or 21, or 31 and/or 41). An antibody having a full length heavy chain and full length light chain having high (*i.e.,* 80% or greater) identity to the full length heavy chains of any of SEQ ID NOs : 11 or 31, and full length light chains of any of SEQ ID NOs: 21 or 41, can be obtained by mutagenesis (*e.g.,* site-directed or PCR-mediated mutagenesis) of nucleic acid molecules encoding such polypeptides, followed by testing of the encoded altered antibody for retained function using the functional assays described herein.

In one aspect, provided herein is an isolated antibody, or a functional antigen binding fragment thereof, comprising a heavy chain and a light chain, wherein the heavy chain comprises an amino acid sequence that is at least 80%, at least 90%, or at least 95% identical to an amino acid sequence selected from the group consisting of SEQ ID NOs: 11 and 31; the light chain comprises an amino acid sequence that is at least 80%, at least 90%, or at least 95% identical to an amino acid sequence selected from the group consisting of SEQ ID NOs: 21 and 41; and the antibody specifically binds to FXI and/or FXIa (*e.g*., human, rabbit, and cynomolgus monkey FXIa). In one embodiment, an isolated antibody, or a functional antigen binding fragment thereof, comprises a heavy chain and a light chain, wherein the heavy chain comprises an amino acid sequence that is at least 80%, at least 90%, or at least 95% identical to the amino acid sequence of SEQ ID NO: 11; the light chain comprises an amino acid sequence that is at least 80%, at least 90%, or at least 95% identical to the amino acid sequence of SEQ ID NO: 21; and the antibody specifically binds to FXI and/or FXIa (*e.g.,* human, rabbit, and

cynomolgus monkey FXIa). In one embodiment, an isolated antibody, or a functional antigen binding fragment thereof, comprises a heavy chain and a light chain, wherein the heavy chain comprises an amino acid sequence that is at least 80%, at least 90%, or at least 95% identical to the amino acid sequence of SEQ ID NO: 31; the light chain comprises an amino acid sequence that is at least 80%, at least 90%, or at least 95% identical to the amino acid sequence of SEQ ID NO: 41; and the antibody specifically binds to FXI and/or FXIa (*e.g.,* human, rabbit, and cynomolgus monkey FXIa). In certain aspects of the present disclosure the heavy and light chain sequences further comprise HCDR1, HCDR2, HCDR3, LCDR1, LCDR2, and LCDR3 sequences as defined by Kabat, for example SEQ ID NOs: 3, 4, 5, 13, 14, and 15, respectively. In certain other aspects of the present disclosure the heavy and light chain sequences further comprise HCDR1, HCDR2, HCDR3, LCDR1, LCDR2, and LCDR3 sequences as defined by Chothia, for example SEQ ID NOs: 6, 7, 8, 16, 17, and 18, respectively. In certain other aspects, the heavy and light chain sequences further comprise HCDR1, HCDR2, HCDR3, LCDR1, LCDR2, and LCDR3 sequences as defined by the Combined system, for example SEQ ID NOs: 46, 4, 5, 33, 14, and 15, respectively. In certain other aspects, the heavy and light chain sequences further comprise HCDR1, HCDR2, HCDR3, LCDR1, LCDR2, and LCDR3 sequences as defined by IMGT, for example SEQ ID NOs: 43, 44, 45, 47, 37, and 15, respectively.

In other embodiments for use in the methods described herein, the full length heavy chain and/or full length light chain nucleotide sequences may be 60%, 70%, 80%, 90%, 95%, 96%, 97%, 98% or 99% identical to the sequences set forth in Table 1 (e.g., SEQ ID NOs: 12 and/or 22, or 32 and/or 42).

In other embodiments for use in the methods described herein, the variable regions of heavy chain and/or the variable regions of light chain nucleotide sequences may be 60%, 70%, 80%, 90%, 95%, 96%, 97%, 98% or 99% identical to the sequences set forth in Table 1 (e.g., SEQ ID NOs: 10 and/or 20, or 30 and/or 40).

As used herein, the percent identity between the two sequences is a function of the number of identical positions shared by the sequences (*i.e.,* % identity equals number of identical positions/total number of positions x 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

Additionally or alternatively, the protein sequences of the present disclosure can further be used as a "query sequence" to perform a search against public databases to, for example, identify related sequences. For example, such searches can be performed using the BLAST program (version 2.0) of Altschul, et al., 1990 J. Mol. Biol. 215:403-10.

**Antibodies with Conservative Modifications**

In certain embodiments, an antibody of the present disclosure for use in the methods described herein (e.g., methods for reducing or preventing the risk of stroke and/or system embolism in subjects with ESRD and/or AF) has a heavy chain variable region comprising CDR1, CDR2, and CDR3 sequences and a light chain variable region comprising CDR1, CDR2, and CDR3 sequences, wherein one or more of these CDR sequences have specified amino acid sequences based on the antibodies described herein or conservative modifications thereof, and wherein the antibodies retain the desired functional properties of the FXIa-binding antibodies of the present disclosure.

Accordingly, for use in the methods described herein, the present disclosure provides an isolated antibody, or an antigen binding fragment thereof, consisting of a heavy chain variable region comprising CDR1, CDR2, and CDR3 sequences and a light chain variable region comprising CDR1, CDR2, and CDR3 sequences, wherein: the heavy chain variable region CDR1 amino acid sequences are selected from the group consisting of SEQ ID NOs: 3 and 23, and conservative modifications thereof; the heavy chain variable region CDR2 amino acid sequences are selected from the group consisting of SEQ ID NOs: 4 and 24, and conservative modifications thereof; the heavy chain variable region CDR3 amino acid sequences are selected from the group consisting of SEQ ID NOs: 5 and 25, and conservative modifications thereof; the light chain variable regions CDR1 amino acid sequences are selected from the group consisting of SEQ ID NOs: 13 and 33, and conservative modifications thereof; the light chain variable regions CDR2 amino acid sequences are selected from the group consisting of SEQ ID NOs: 14 and 34, and conservative modifications thereof; the light chain variable regions of CDR3 amino acid sequences are selected from the group consisting of SEQ ID NOs: 15 and 35, and conservative modifications thereof; and the antibody or antigen binding fragments thereof specifically binds to FXIa.

In one aspect, provided herein is an isolated antibody, or an antigen binding fragment thereof, consisting of a heavy chain variable region comprising CDR1, CDR2, and CDR3 sequences and a light chain variable region comprising CDR1, CDR2, and CDR3 sequences, wherein: the heavy chain variable region CDR1 amino acid sequences are selected from the group consisting of those described in Table 1, and conservative modifications thereof; the heavy chain variable region CDR2 amino acid sequences are selected from the group consisting of those described in Table 1, and conservative modifications thereof; the heavy chain variable region CDR3 amino acid sequences are selected from the group consisting of those described in Table 1, and conservative modifications thereof; the light chain variable regions CDR1 amino acid sequences are selected from the group consisting of those described in Table 1, and conservative modifications thereof; the light chain variable regions CDR2 amino acid sequences are selected from the group consisting of those described in Table 1, and conservative modifications thereof; the light chain variable regions of CDR3 amino acid sequences are selected from the group consisting of those described in Table 1, and conservative modifications thereof; and the antibody or antigen binding fragments thereof specifically binds to FXIa.

In other embodiments for use in the methods described herein, the antibody of the present disclosure is optimized for expression in a mammalian cell has a full length heavy chain sequence and a full length light chain sequence, wherein one or more of these sequences have specified amino acid sequences based on the antibodies described herein or conservative modifications thereof, and wherein the antibodies retain the desired functional properties of the FXIa binding antibodies of the present disclosure. Accordingly, the present disclosure provides an isolated antibody optimized for expression in a mammalian cell consisting of a full length heavy chain and a full length light chain wherein the full length heavy chain has amino acid sequences selected from the group of SEQ ID NOs: 11 or 31, and conservative modifications thereof; and the full length light chain has amino acid sequences selected from the group of SEQ ID NOs: 21 or 41, and conservative modifications thereof; and the antibody specifically binds to FXI and/or FXIa (e.g., human, rabbit, and cynomolgus monkey FXIa).

**Antibodies That Bind to the Same Epitope**

The present disclosure provides antibodies that bind to the same epitope as the FXI and/or FXIa binding antibodies described in Table 1, for use in the methods described herein (e.g., methods for reducing or preventing the risk of stroke and/or system embolism in subjects with ESRD and/or AF). Additional antibodies can therefore be identified based on their ability to compete (e.g., to competitively inhibit the binding of, in a statistically significant manner, by binding to the same or overlapping epitope) with other antibodies of the present disclosure in FXI and/or FXIa binding assays (such as those described in the Examples Section). The ability of a test antibody to inhibit the binding of antibodies of the present disclosure to a FXI and/or FXIa protein demonstrates that the test antibody can compete with that antibody for binding to FXI and/or FXIa; such an antibody may, according to non-limiting theory, bind to the same or a related (e.g., a structurally similar or spatially proximal) epitope on the FXI and/or FXIa protein as the antibody with which it competes. In a certain embodiment, the antibody that binds to the same epitope on FXI and/or FXIa as the antibodies of the present disclosure is a human monoclonal antibody. Such human monoclonal antibodies can be prepared and isolated as described herein.

As used herein, an antibody "competes" for binding when the competing antibody binds to the same FXI and/or FXIa epitope as an antibody or antigen binding fragment of the present disclosure (e.g., NOV1401 or NOV1090) and inhibits FXI and/or FXIa binding of an antibody or antigen binding fragment of the present disclosure by more than 50% (for example, 80%, 85%, 90%, 95%, 98% or 99%) in the presence of an equimolar concentration of competing antibody. This may be determined, for instance, in a competitive binding assay, by any of the methods well known to those of skill in the art.

As used herein, an antibody or antigen binding fragment thereof does not "compete" with an FXI and/or FXIa antibody or antigen binding fragment of the present disclosure (e.g., NOV1401 or NOV1090) unless said competing antibody or antigen binding fragment thereof binds the same FXI and/or FXIa epitope, or an overlapping FXI and/or FXIa epitope, as an antibody or antigen binding fragment of the present disclosure. As used herein, a competing antibody or antigen binding fragment thereof does not include one which (i) sterically blocks an antibody or antigen binding fragment of the present disclosure from binding its target (e.g., if said competing antibody binds to a nearby, non-overlapping FXI and/or FXIa epitope and physically prevents an antibody or antigen binding fragment of the present disclosure from binding its target); and/or (ii) binds to a different, non-overlapping FXI and/or FXIa epitope and induces a conformational change to the FXI and/or FXIa protein such that said protein can no longer be bound by an FXI and/or FXIa antibody or antigen binding fragment of the present disclosure in a way that would occur absent said conformational change.

**Engineered and Modified Antibodies**

An antibody of the present disclosure, for use in the methods described herein, further can be prepared using an antibody having one or more of the VH and/or VL sequences shown herein as starting material to engineer a modified antibody, which modified antibody may have altered properties from the starting antibody. An antibody can be engineered by modifying one or more residues within one or both variable regions (i. e., VH and/or VL), for example within one or more CDR regions and/or within one or more framework regions. Additionally or alternatively, an antibody can be engineered by modifying residues within the constant region(s), for example to alter the effector function(s) of the antibody.

One type of variable region engineering that can be performed is CDR grafting. Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al., 1998 Nature 332:323-327; Jones, P. et al., 1986 Nature 321:522-525; Queen, C. et al., 1989 Proc. Natl. Acad., U.S.A. 86:10029-10033; U.S. Patent No. 5,225,539 to Winter, and U.S. Patent Nos. 5,530,101; 5,585,089; 5,693,762 and 6,180,370 to Queen et al.)

Accordingly, another embodiment of the present disclosure pertains to an isolated antibody, or an antigen binding fragment thereof, comprising a heavy chain variable region comprising CDR1 sequences having an amino acid sequence selected from the group consisting of SEQ ID NOs: 3 and 23; CDR2 sequences having an amino acid sequence selected from the group consisting of SEQ ID NOs: 4 and 24; CDR3 sequences having an amino acid sequence selected from the group consisting of SEQ ID NOs: 5 and 25, respectively; and a light chain variable region having CDR1 sequences having an amino acid sequence selected from the group consisting of SEQ ID NOs: 13 and 33; CDR2 sequences having an amino acid sequence selected from the group consisting of SEQ ID NOs: 14 and 34; and CDR3 sequences consisting of an amino acid sequence selected from the group consisting of SEQ ID NOs: 15 and 35, respectively. Thus, such antibodies contain the VH and VL CDR sequences of monoclonal antibodies, yet may contain different framework sequences from these antibodies.

Such framework sequences can be obtained from public DNA databases or published references that include germline antibody gene sequences. For example, germline DNA sequences for human heavy and light chain variable region genes can be found in the "VBase" human germline sequence database (available on the world wide web at mrc-cpe.cam.ac.uk/vbase), as well as in Kabat, E. A., et al., 1991 Sequences of Proteins of Immunological Interest, Fifth Edition, U.S. Department of Health and Human Services, NIH Publication No. 91-3242; Tomlinson, I. M., et al., 1992 J. Mol. Biol. 227:776-798; and Cox, J. P. L. et al., 1994 Eur. J Immunol. 24:827-836; the contents of each of which are expressly incorporated herein by reference.

An example of framework sequences for use in the antibodies of the present disclosure are those that are structurally similar to the framework sequences used by selected antibodies of the present disclosure, e.g., consensus sequences and/or framework sequences used by monoclonal antibodies of the present disclosure. The VH CDR1, 2 and 3 sequences, and the VL CDR1, 2 and 3 sequences, can be grafted onto framework regions that have the identical sequence as that found in the germline immunoglobulin gene from which the framework sequence derive, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see *e.g.,* U.S. Patent Nos. 5,530,101; 5,585,089; 5,693,762 and 6,180,370 to Queen et al). Frameworks that can be utilized as scaffolds on which to build the antibodies and antigen binding fragments described herein include, but are not limited to VH1A, VH1B, VH3, Vk1, VI2, and Vk2. Additional frameworks are known in the art and may be found, for example, in the vBase data base on the world wide web at vbase.mrc-cpe.cam.ac.uk/index.php?&MMN_position=1:1.

Accordingly, for use in the methods described herein, an embodiment of the present disclosure relates to isolated FXIa binding antibodies, or antigen binding fragments thereof, comprising a heavy chain variable region comprising an amino acid sequence selected from the group consisting of SEQ ID NOs: 9 and 29, or an amino acid sequence having one, two, three, four or five amino acid substitutions, deletions or additions in the framework region of such sequences, and further comprising a light chain variable region having an amino acid sequence selected from the group consisting of SEQ ID NOs: 19 or 39, or an amino acid sequence having one, two, three, four or five amino acid substitutions, deletions or additions in the framework region of such sequences.

Another type of variable region modification is to mutate amino acid residues within the VH and/or VL CDR1, CDR2 and/or CDR3 regions to thereby improve one or more binding properties (*e.g.,* affinity) of the antibody of interest, known as "affinity maturation." Site-directed mutagenesis or PCR-mediated mutagenesis can be performed to introduce the mutation(s) and the effect on antibody binding, or other functional property of interest, can be evaluated in *in vitro* or *in vivo* assays as described herein and provided in the Examples Section. Conservative modifications (as discussed above) can be introduced. The mutations may be amino acid substitutions, additions or deletions. Moreover, typically no more than one, two, three, four or five residues within a CDR region are altered.

Accordingly, in another embodiment for use in the methods described herein, the present disclosure provides isolated FXIa-binding antibodies, or antigen binding fragments thereof, consisting of a heavy chain variable region having a VH CDR1 region consisting of an amino acid sequence selected from the group having SEQ ID NOs: 3 and 23 or an amino acid sequence having one, two, three, four or five amino acid substitutions, deletions or additions as compared to SEQ ID NOs: 3 and 23; a VH CDR2 region having an amino acid sequence selected from the group consisting of SEQ ID NOs: 4 and 24 or an amino acid sequence having one, two, three, four or five amino acid substitutions, deletions or additions as compared to SEQ ID NOs: 4 and 24; a VH CDR3 region having an amino acid sequence selected from the group consisting of SEQ ID NOs: 5 and 25, or an amino acid sequence having one, two, three, four or five amino acid substitutions, deletions or additions as compared to SEQ ID NOs: 5 and 25; a VL CDR1 region having an amino acid sequence selected from the group consisting of SEQ ID NOs: 13 and 33, or an amino acid sequence having one, two, three, four or five amino acid substitutions, deletions or additions as compared to SEQ ID NOs: 13 and 33; a VL CDR2 region having an amino acid sequence selected from the group consisting of SEQ ID NOs: 14 and 34, or an amino acid sequence having one, two, three, four or five amino acid substitutions, deletions or additions as compared to SEQ ID NOs: 14 and 34; and a VL CDR3 region having an amino acid sequence selected from the group consisting of SEQ ID NOs: 15 and 35, or an amino acid sequence having one, two, three, four or five amino acid substitutions, deletions or additions as compared to SEQ ID NOs: 15 and 35.

Accordingly, in another embodiment for use in the methods described herein, the present disclosure provides isolated FXIa-binding antibodies, or antigen binding fragments thereof, consisting of a heavy chain variable region having a VH CDR1 region consisting of an amino acid sequence selected from the group having SEQ ID NOs: 6 and 26 or an amino acid sequence having one, two, three, four or five amino acid substitutions, deletions or additions as compared to SEQ ID NOs: 6 and 26; a VH CDR2 region having an amino acid sequence selected from the group consisting of SEQ ID NOs: 7 and 27 or an amino acid sequence having one, two, three, four or five amino acid substitutions, deletions or additions as compared to SEQ ID NOs: 7 and 27; a VH CDR3 region having an amino acid sequence selected from the group consisting of SEQ ID NOs: 8 and 28, or an amino acid sequence having one, two, three, four or five amino acid substitutions, deletions or additions as compared to SEQ ID NOs: 8 and 28; a VL CDR1 region having an amino acid sequence selected from the group consisting of SEQ ID NOs: 16 and 36, or an amino acid sequence having one, two, three, four or five amino acid substitutions, deletions or additions as compared to SEQ ID NOs: 16 and 36; a VL CDR2 region having an amino acid sequence selected from the group consisting of SEQ ID NOs: 17 and 37, or an amino acid sequence having one, two, three, four or five amino acid substitutions, deletions or additions as compared to SEQ ID NOs: 17 and 37; and a VL CDR3 region having an amino acid sequence selected from the group consisting of SEQ ID NOs: 18 and 38, or an amino acid sequence having one, two, three, four or five amino acid substitutions, deletions or additions as compared to SEQ ID NOs: 18 and 38.

**Antibodies with Extended Half Life**

The present disclosure provides for antibodies that specifically bind to FXIa protein which have an extended half-life *in vivo,* for use in the methods described herein.

Many factors may affect a protein's half-life *in vivo.* For examples, kidney filtration, metabolism in the liver, degradation by proteolytic enzymes (proteases), and immunogenic responses (*e.g.,* protein neutralization by antibodies and uptake by macrophages and dendritic cells). A variety of strategies can be used to extend the half-life of the antibodies of the present disclosure. For example, by chemical linkage to polyethyleneglycol (PEG), reCODE PEG, antibody scaffold, polysialic acid (PSA), hydroxyethyl starch (HES), albumin-binding ligands, and carbohydrate shields; by genetic fusion to proteins binding to serum proteins, such as albumin, IgG, FcRn, and transferring; by coupling (genetically or chemically) to other binding moieties that bind to serum proteins, such as nanobodies, Fabs, DARPins, avimers, affibodies, and anticalins; by genetic fusion to rPEG, albumin, domain of albumin, albumin-binding proteins, and Fc; or by incorporation into nanocarriers, slow release formulations, or medical devices.

To prolong the serum circulation of antibodies *in vivo,* inert polymer molecules such as high molecular weight PEG can be attached to the antibodies or a fragment thereof with or without a multifunctional linker either through site-specific conjugation of the PEG to the N- or C-terminus of the antibodies or via epsilon-amino groups present on lysine residues. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Linear or branched polymer derivatization that results in minimal loss of biological activity will be used. The degree of conjugation can be closely monitored by SDS-PAGE and mass spectrometry to ensure proper conjugation of PEG molecules to the antibodies. Unreacted PEG can be separated from antibody-PEG conjugates by size-exclusion or by ion-exchange chromatography. PEG-derivatized antibodies can be tested for binding activity as well as for *in vivo* efficacy using methods well-known to those of skill in the art, for example, by immunoassays described herein. Methods for pegylating proteins are known in the art and can be applied to the antibodies of the present disclosure. See for example, EP 0 154 316 by Nishimura et al. and EP 0 401 384 by Ishikawa et al.

Other modified pegylation technologies include reconstituting chemically orthogonal directed engineering technology (ReCODE PEG), which incorporates chemically specified side chains into biosynthetic proteins via a reconstituted system that includes tRNA synthetase and tRNA. This technology enables incorporation of more than 30 new amino acids into biosynthetic proteins in E.coli, yeast, and mammalian cells. The tRNA incorporates a nonnative amino acid any place an amber codon is positioned, converting the amber from a stop codon to one that signals incorporation of the chemically specified amino acid.

Recombinant pegylation technology (rPEG) can also be used for serum halflife extension. This technology involves genetically fusing a 300-600 amino acid unstructured protein tail to an existing pharmaceutical protein. Because the apparent molecular weight of such an unstructured protein chain is about 15-fold larger than its actual molecular weight, the serum half-life of the protein is greatly increased. In contrast to traditional PEGylation, which requires chemical conjugation and repurification, the manufacturing process is greatly simplified and the product is homogeneous.

Polysialytion is another technology, which uses the natural polymer polysialic acid (PSA) to prolong the active life and improve the stability of therapeutic peptides and proteins. PSA is a polymer of sialic acid (a sugar). When used for protein and therapeutic peptide drug delivery, polysialic acid provides a protective microenvironment on conjugation. This increases the active life of the therapeutic protein in the circulation and prevents it from being recognized by the immune system. The PSA polymer is naturally found in the human body.

It was adopted by certain bacteria which evolved over millions of years to coat their walls with it. These naturally polysialylated bacteria were then able, by virtue of molecular mimicry, to foil the body's defense system. PSA, nature's ultimate stealth technology, can be easily produced from such bacteria in large quantities and with predetermined physical characteristics. Bacterial PSA is completely non-immunogenic, even when coupled to proteins, as it is chemically identical to PSA in the human body.

Another technology includes the use of hydroxyethyl starch ("HES") derivatives linked to antibodies. HES is a modified natural polymer derived from waxy maize starch and can be metabolized by the body's enzymes. HES solutions are usually administered to substitute deficient blood volume and to improve the rheological properties of the blood. Hesylation of an antibody enables the prolongation of the circulation half-life by increasing the stability of the molecule, as well as by reducing renal clearance, resulting in an increased biological activity. By varying different parameters, such as the molecular weight of HES, a wide range of HES antibody conjugates can be customized.

Antibodies having an increased half-life *in vivo* can also be generated introducing one or more amino acid modifications (*i.e.,* substitutions, insertions or deletions) into an IgG constant domain, or FcRn binding fragment thereof (preferably a Fc or hinge Fc domain fragment). See, e.g., International Publication No. WO 98/23289; International Publication No. WO 97/34631; and U.S. Patent No. 6,277,375.

Further, antibodies can be conjugated to albumin (e.g., human serum albumin; HSA) in order to make the antibody or antibody fragment more stable *in vivo* or have a longer half life *in vivo.* The techniques are well-known in the art, see, *e.g.,* International Publication Nos. WO 93/15199, WO 93/15200, and WO 01/77137; and European Patent No. EP 413,622. In addition, in the context of a bispecific antibody as described above, the specificities of the antibody can be designed such that one binding domain of the antibody binds to FXIa while a second binding domain of the antibody binds to serum albumin, preferably HSA.

The strategies for increasing half-life is especially useful in nanobodies, fibronectin-based binders, and other antibodies or proteins for which increased *in vivo* half-life is desired.

**Antibody Conjugates**

The present disclosure provides antibodies or fragments thereof, for use in the methods described herein, that specifically bind to a FXIa protein recombinantly fused or chemically conjugated (including both covalent and non-covalent conjugations) to a heterologous protein or polypeptide (or fragment thereof, preferably to a polypeptide of at least 10, at least 20, at least 30, at least 40, at least 50, at least 60, at least 70, at least 80, at least 90 or at least 100 amino acids) to generate fusion proteins. In particular, the present disclosure provides fusion proteins comprising an antigen-binding fragment of an antibody described herein (*e.g.,* a Fab fragment, Fd fragment, Fv fragment, F(ab)2 fragment, a VH domain, a VH CDR, a VL domain or a VL CDR) and a heterologous protein, polypeptide, or peptide. Methods for fusing or conjugating proteins, polypeptides, or peptides to an antibody or an antibody fragment are known in the art. See, *e.g.,* U.S. Patent Nos. 5,336,603, 5,622,929, 5,359,046, 5,349,053, 5,447,851, and 5,112,946; European Patent Nos. EP 307,434 and EP 367,166; International Publication Nos. WO 96/04388 and WO 91/06570; Ashkenazi et al., 1991, Proc. Natl. Acad. Sci. USA 88: 10535-10539; Zheng et al., 1995, J. Immunol. 154:5590-5600; and Vil et al., 1992, Proc. Natl. Acad. Sci. USA 89:11337- 11341.

Additional fusion proteins may be generated through the techniques of gene-shuffling, motif-shuffling, exon-shuffling, and/or codon-shuffling (collectively referred to as "DNA shuffling"). DNA shuffling may be employed to alter the activities of antibodies of the present disclosure or fragments thereof (*e.g.*, antibodies or fragments thereof with higher affinities and lower dissociation rates). See, generally, U.S. Patent Nos. 5,605,793, 5,811,238, 5,830,721, 5,834,252, and 5,837,458; Patten et al., 1997, Curr. Opinion Biotechnol. 8:724-33; Harayama, 1998, Trends Biotechnol. 16(2):76-82; Hansson, et al., 1999, J. Mol. Biol. 287:265-76; and Lorenzo and Blasco, 1998, Biotechniques 24(2):308- 313 (each of these patents and publications are hereby incorporated by reference in its entirety). Antibodies or fragments thereof, or the encoded antibodies or fragments thereof, may be altered by being subjected to random mutagenesis by error-prone PCR, random nucleotide insertion or other methods prior to recombination. A polynucleotide encoding an antibody or fragment thereof that specifically binds to a FXIa protein may be recombined with one or more components, motifs, sections, parts, domains, fragments, etc. of one or more heterologous molecules.

Moreover, the antibodies or fragments thereof can be fused to marker sequences, such as a peptide to facilitate purification. In preferred embodiments, the marker amino acid sequence is a hexa-histidine peptide (SEQ ID NO: 48), such as the tag provided in a pQE vector (QIAGEN, Inc., 9259 Eton Avenue, Chatsworth, CA, 91311), among others, many of which are commercially available. As described in Gentz et al., 1989, Proc. Natl. Acad. Sci. USA 86:821-824, for instance, hexa-histidine (SEQ ID NO: 48) provides for convenient purification of the fusion protein. Other peptide tags useful for purification include, but are not limited to, the hemagglutinin ("HA") tag, which corresponds to an epitope derived from the influenza hemagglutinin protein (Wilson et al., 1984, Cell 37:767), and the "flag" tag.

In other embodiments, antibodies of the present disclosure or fragments thereof conjugated to a diagnostic or detectable agent. Such antibodies can be useful for monitoring or prognosing the onset, development, progression and/or severity of a disease or disorder as part of a clinical testing procedure, such as determining the efficacy of a particular therapy. Such diagnosis and detection can accomplished by coupling the antibody to detectable substances including, but not limited to, various enzymes, such as, but not limited to, horseradish peroxidase, alkaline phosphatase, beta-galactosidase, or acetylcholinesterase; prosthetic groups, such as, but not limited to, streptavidinlbiotin and avidin/biotin; fluorescent materials, such as, but not limited to, umbelliferone, fluorescein, fluorescein isothiocynate, rhodamine, dichlorotriazinylamine fluorescein, dansyl chloride or phycoerythrin; luminescent materials, such as, but not limited to, luminol; bioluminescent materials, such as but not limited to, luciferase, luciferin, and aequorin; radioactive materials, such as, but not limited to, iodine (1311, 125I, 123I, and 1211,), carbon (14C), sulfur (35S), tritium (3H), indium (115In, 113In, 112In, and 111In,), technetium (99Tc), thallium (201Ti), gallium (68Ga, 67Ga), palladium (103Pd), molybdenum (99Mo), xenon (133Xe), fluorine (18F), 153Sm, 177Lu, 159Gd, 149Pm, 140La, 175Yb, 166Ho, 90Y, 47Sc, 186Re, 188Re,142 Pr, 105Rh, 97Ru, 68Ge, 57Co, 65Zn, 85Sr, 32P, 153Gd, 169Yb, 51Cr, 54Mn, 75Se, 113Sn, and 117Tin; and positron emitting metals using various positron emission tomographies, and noradioactive paramagnetic metal ions.

The present disclosure further encompasses uses of antibodies or fragments thereof conjugated to a therapeutic moiety. An antibody or fragment thereof may be conjugated to a therapeutic moiety such as a cytotoxin, e.g., a cytostatic or cytocidal agent, a therapeutic agent or a radioactive metal ion, e.g., alpha-emitters. A cytotoxin or cytotoxic agent includes any agent that is detrimental to cells.

Further, an antibody or fragment thereof may be conjugated to a therapeutic moiety or drug moiety that modifies a given biological response. Therapeutic moieties or drug moieties are not to be construed as limited to classical chemical therapeutic agents. For example, the drug moiety may be a protein, peptide, or polypeptide possessing a desired biological activity. Such proteins may include, for example, a toxin such as abrin, ricin A, pseudomonas exotoxin, cholera toxin, or diphtheria toxin; a protein such as tumor necrosis factor, $\alpha$-interferon, $\beta$-interferon, nerve growth factor, platelet derived growth factor, tissue plasminogen activator, an apoptotic agent, an anti-angiogenic agent; or, a biological response modifier such as, for example, a lymphokine.

Moreover, an antibody can be conjugated to therapeutic moieties such as a radioactive metal ion, such as alph-emiters such as 213Bi or macrocyclic chelators useful for conjugating radiometal ions, including but not limited to, 131In, 131LU, 131Y, 131Ho, 131Sm, to polypeptides. In certain embodiments, the macrocyclic chelator is 1,4,7,10-tetraazacyclododecane-N,N',N",N'''-tetraacetic acid (DOTA) which can be attached to the antibody via a linker molecule. Such linker molecules are commonly known in the art and described in Denardo et al., 1998, Clin Cancer Res. 4(10):2483-90; Peterson et al., 1999, Bioconjug. Chem. 10(4):553-7; and Zimmerman et al., 1999, Nucl. Med. Biol. 26(8):943-50, each incorporated by reference in their entireties.

Techniques for conjugating therapeutic moieties to antibodies are well known, see, *e.g.,* Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies 84: Biological And Clinical Applications, Pinchera et al. (eds.), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., 1982, Immunol. Rev. 62:119-58.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

**Methods of Producing Antibodies**

Nucleic Acids Encoding the Antibodies

The present disclosure provides substantially purified nucleic acid molecules which encode polypeptides comprising segments or domains of the FXIa-binding antibody chains described above, as well as vectors and host cells for producing antibodies. Some of the nucleic acids of the present disclosure comprise the nucleotide sequence encoding the heavy chain variable region shown in SEQ ID NO: 10 or 30, and/or the nucleotide sequence encoding the light chain variable region shown in SEQ ID NO: 20 or 40. In a specific embodiment, the nucleic acid molecules are those identified in Table 1. Some other nucleic acid molecules of the present disclosure comprise nucleotide sequences that are substantially identical (e.g., at least 65, 80%, 95%, or 99%) to the nucleotide sequences of those identified in Table 1. When expressed from appropriate expression vectors, polypeptides encoded by these polynucleotides are capable of exhibiting FXI and/or FXIa antigen binding capacity. In specific aspects, because of the degeneracy of the code, a variety of nucleic acid sequences will encode each of the immunoglobulin amino acid sequences.

The nucleic acid molecules can encode both a variable region and a constant region of the antibody. Some of nucleic acid sequences of the present disclosure comprise nucleotides encoding a heavy chain sequence that is substantially identical (e.g., at least 80%, 90%, or 99%) to the heavy chain sequence set forth in SEQ ID NO: 11 or 31. Some other nucleic acid sequences comprising nucleotide encoding a light chain sequence that is substantially identical (e.g., at least 80%, 90%, or 99%) to the light chain sequence set forth in SEQ ID NO: 21 or 41.

Polynucleotide sequences can be produced by de novo solid-phase DNA synthesis or by PCR mutagenesis of an existing sequence (e.g., sequences as described in the Examples below) encoding a FXIa-binding antibody or its binding fragment. Direct chemical synthesis of nucleic acids can be accomplished by methods known in the art, such as the phosphotriester method of Narang et al., 1979, Meth. Enzymol. 68:90; the phosphodiester method of Brown et al., Meth. Enzymol. 68:109, 1979; the diethylphosphoramidite method of Beaucage et al., Tetra. Lett., 22:1859, 1981; and the solid support method of U.S. Patent No. 4,458,066. Introducing mutations to a polynucleotide sequence by PCR can be performed as described in, e.g., PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al., Nucleic Acids Res. 19:967, 1991; and Eckert et al., PCR Methods and Applications 1:17, 1991.

Various expression vectors can be employed to express the polynucleotides encoding the FXIa-binding antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., Nat Genet 15:345, 1997). For example, nonviral vectors useful for expression of the FXIa-binding polynucleotides and polypeptides in mammalian (e.g., human) cells include pThioHis A, B & C, pcDNA3.1/His, pEBVHis A, B & C, (Invitrogen, San Diego, CA), MPSV vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., supra; Smith, Annu. Rev. Microbiol. 49:807, 1995; and Rosenfeld et al., Cell 68:143, 1992.

The expression vectors may also provide a secretion signal sequence position to form a fusion protein with polypeptides encoded by inserted FXIa-binding antibody sequences. More often, the inserted FXI and/or FXIa-binding antibody sequences are linked to a signal sequences before inclusion in the vector. Vectors to be used to receive sequences encoding FXI and/or FXIa-binding antibody light and heavy chain variable domains sometimes also encode constant regions or parts thereof. Such vectors allow expression of the variable regions as fusion proteins with the constant regions thereby leading to production of intact antibodies or fragments thereof. Typically, such constant regions are human.

The host cells for harboring and expressing the FXI and/or FXIa-binding antibody chains can be either prokaryotic or eukaryotic. E. coli is one prokaryotic host useful for cloning and expressing the polynucleotides of the present disclosure. Other microbial hosts suitable for use include bacilli, such as Bacillus subtilis, and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express FXIa-binding polypeptides of the present disclosure. Insect cells in combination with baculovirus vectors can also be used.

In specific aspects, mammalian host cells are used to express and produce the FXI and/or FXIa-binding polypeptides of the present disclosure. These include any normal mortal or normal or abnormal immortal animal or human cell. For example, a number of suitable host cell lines capable of secreting intact immunoglobulins have been developed including the CHO cell lines, various Cos cell lines, HeLa cells, myeloma cell lines, and transformed B-cells. The use of mammalian tissue cell culture to express polypeptides is discussed generally in, e.g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e.g., Queen, et al., Immunol. Rev. 89:49-68, 1986), and necessary processing information sites, such as ribosome binding sites, RNA splice sites, polyadenylation sites, and transcriptional terminator sequences.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, *et al.,* supra). Other methods include, e.g., electroporation, calcium phosphate treatment, liposome-mediated transformation, injection and microinjection, ballistic methods, virosomes, immunoliposomes, polycation:nucleic acid conjugates, naked DNA, artificial virions, fusion to the herpes virus structural protein VP22 (Elliot and O'Hare, Cell 88:223, 1997), agent-enhanced uptake of DNA, and ex vivo transduction. For long-term, high-yield production of recombinant proteins, stable expression will often be desired. For example, cell lines which stably express FXIa-binding antibody chains or binding fragments can be prepared using expression vectors of the invention which contain viral origins of replication or endogenous expression elements and a selectable marker gene. Following the introduction of the vector, cells may be allowed to grow for 1-2 days in an enriched media before they are switched to selective media. The purpose of the selectable marker is to confer resistance to selection, and its presence allows growth of cells which successfully express the introduced sequences in selective media. Resistant, stably transfected cells can be proliferated using tissue culture techniques appropriate to the cell type.

## Prophylactic and Therapeutic Uses

Antibodies that bind FXI and/or FXIa as described herein (e.g., antibodies described in Table 1, such as, anti-FXI/FXIa antibodies comprising VL CDRs and VHCDRs of NOV1401), can be used at a therapeutically useful concentration for the treatment of a thromboembolic disease or disorder, such as, for reduce the risk of stroke and/or systemic embolism in patients with ESRD and/or AF, undergoing dialysis. The present disclosure provides a method of treating thromboembolic disorders by administering to a subject in need thereof an effective amount of antibodies of the present disclosure. The present disclosure provides a method of treating thromboembolic disorders (*e.g.,* ischemic stroke (cardioembolic, thrombotic) or systemic embolism, AF, stroke prevention in AF (SPAF), deep vein thrombosis, venous thromboembolism, pulmonary embolism, acute coronary syndromes (ACS), acute limb ischemia, chronic thromboembolic pulmonary hypertension, or systemic embolism) by administering to a subject in need thereof an effective amount of an anti-FXI/FXIa antibody of the present disclosure. Also provided herein are combination therapies.

The antibodies described herein (*e.g.,* antibodies described in Table 1, such as, NOV1401 or NOV1090 or anti-FXI/FXIa antibodies comprising VL CDRs and VHCDRs of NOV1401 or NOV1090) can be used, *inter alia,* to prevent treat, prevent, and improve thromboembolic conditions or disorders, including but not limited to thrombotic disorders, as described in greater detail herein.

The antibodies provided herein (*e.g.,* antibodies described in Table 1, such as, anti-FXI/FXIa antibodies comprising VL CDRs and VHCDRs of NOV1401 or NOV1090) can also be used in combination with other agents for the prevention, treatment, or improvement of thromboembolic disorders. For example, statin therapies may be used in combination with the FXIa antibodies and antigen binding fragments of the present disclosure for the treatment of patients with thrombotic and/or thromboembolic disorders.

In a specific embodiment, provided herein is a method of treating or preventing stroke in a patient with AF, comprising administering to the patient in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of managing or preventing risks or conditions associated with atrial fibrillation (AF), such as cardioembolic stroke and systemic embolism in a patient with AF, comprising administering to the patient in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of treating, managing or preventing conditions associated with atrial fibrillation (AF), such as cardioembolic stroke and systemic embolism in a patient with AF, comprising administering to the patient in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401. In particular embodiments, an AF patient has a high bleeding risk. In a particular aspect, an AF patient has a high bleeding risk as determined by a $CHA_2DS_2VASc$ risk score $\geq 2$. In specific aspects, an AF patient has non-valvular AF and a high bleeding risk as determined by a $CHA_2DS_2VASc$ risk score $\geq 2$. In specific aspects, an AF patient is age 55 or older, and has non-valvular AF and a high bleeding risk as determined by a $CHA_2DS_2VASc$ risk score $\geq 2$.

In a specific embodiment, provided herein is a method of treating, managing or preventing deep vein thrombosis or conditions associated therewith, in a subject (e.g., a subject with, or at risk of developing, deep vein thrombosis), comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL

CDRs and VHCDRs of NOV1401.

In a specific embodiment, provided herein is a method of treating, managing or preventing venous thromboembolism (VTE) or conditions associated therewith, in a subject (*e.g.,* a subject with, or at risk of developing, venous thromboembolism), comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401. In particular embodiments, subjects being treated with an anti-FXI/FXIa antibody provided herein have experienced 1) a first provoked or unprovoked VTE with low risk for bleeding, 2) recurrence of unprovoked VTE, or 3) VTE associated with thrombophilia including cancer patients. In a particular aspect, the subject being treated for VTE is between the age of 0 and 18 years old. In a particular aspect, the subject being treated for VTE is between the age of 1 and 18 years old. In a particular aspect, the subject being treated for VTE is between the age of 2 and 18 years old. In a particular aspect, the subject being treated for VTE is between the age of 0 and 12 years old. In a particular aspect, the subject being treated for VTE is between the age of 1 and 12 years old. In a particular aspect, the subject being treated for VTE is between the age of 2 and 12 years old. In a particular aspect, the subject being treated for VTE is between the age of 12 and 18 years old. In a particular aspect, the subject being treated for VTE is a child at least 2 years old and under 18 years old. In a particular aspect, the subject being treated for VTE is a child at least 12 years old and under 18 years old.

In a specific embodiment, provided herein is a method for secondary prevention (e.g., secondary prevention for a period in the range of 28 days to 18 years) of venous thromboembolism, comprising administering to a subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401. In a particular aspect of methods for secondary prevention (e.g., secondary prevention for a period in the range of 28 days to 18 years) of venous thromboembolism provided herein, the subject is between the age of 0 and 18 years old. In a particular aspect of methods for secondary prevention (e.g., secondary prevention for a period in the range of 28 days to 18 years) of venous thromboembolism provided herein, the subject is between the age of 0 and 12 years old. In a particular aspect of methods for secondary prevention of venous thromboembolism provided herein, the subject is between the age of 12 and 18 years old. In a particular aspect of methods for secondary prevention of venous thromboembolism provided herein, the subject is a child at least 2 years old and under 18 years old. In a particular aspect of methods for secondary prevention of venous thromboembolism provided herein, the subject is a child at least 12 years old and under 18 years old.

In a specific embodiment, provided herein is a method of treating, managing or preventing pulmonary embolism or conditions associated therewith, in a subject (e.g., a subject with, or at risk of developing, pulmonary embolism), comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of treating, managing or preventing acute coronary syndromes (ACS) or conditions associated therewith, in a subject, comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of treating, managing or preventing ischemic stroke, in a subject *(e.g.,* a subject with, or at risk of developing, ischemic stroke), comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of treating, managing or preventing acute limb ischemia, in a subject, comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of treating, managing or preventing chronic thromboembolic pulmonary hypertension, in a subject, comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a specific embodiment, provided herein is a method of treating, managing or preventing systemic embolism, in a subject (e.g., a subject with, or at risk of developing, systemic embolism), comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In a certain embodiment, provided herein is a method of treating, managing, or preventing thromboembolic conditions that are catheter-related conditions (e.g., Hickman catheter in cancer patients) in which catheters become thrombosed, or extracorporeal membrane oxygenation (ECMO), in which the tubing develops clots, comprising administering to the subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VH CDRs of NOV1401.

In specific aspects, provided herein is a method of preventing, treating or managing or reducing the risk of catheter-related thrombosis comprising administering to a subject in need thereof which is afflicated with ESRD initiated on hemodialysis with a catheter (e.g., inserted tunneled catheter), an effective amount of a pharmaceutical composition comprising an anti-FXI/FXIa antibody described herein, for example, an anti-FXI/FXIa antibody described in Table 1, such as, NOV1401 or anti-FXI/FXIa antibodies comprising VL CDRs and VH CDRs of NOV1401 (e.g., VH CDRs of SEQ ID NO: 31 and VL CDRs of SEQ ID NO: 41). In particular aspects of the methods provided herein, the subject has a demonstrated high risk of bleeding. In particular aspects of the methods provided herein, the subject afflicted with ESRD has AF, such as non-valvular AF, and a high bleeding risk as determined by a $CHA_2DS_2VASc$ risk score $\geq 2$. In specific aspects, In particular aspects of the methods provided herein, the subject afflicted with ESRD has AF, such as non-valvular AF, and a high bleeding risk as determined by a $CHA_2DS_2VASc$ risk score $\geq 2$, and is 55 years old or older.

In specific aspects, provided herein are methods and uses to reduce the incidence of thrombosis and improves the vascular access survival (e.g., tunneled catheter, arteriovenous graft and arteriovenous fistula) in patients undergoing hemodialysis for ESRD and prevents extra-corporal thrombosis when used alone or in combination with other anticoagulants such as unfractionated heparin or low molecular weight heparin, wherein said method or uses involve administering to patients an effective amount of an anti-FXI/FXIa antibody described herein, such as NOV1401 (e.g., antibody comprising SEQ ID NOs: 31 and 41).

In other embodiments, provided herein are methods for acute VTE treatment, primary and extended secondary prevention of VTE, prevention of major adverse thromboembolic events in patient undergoing dialysis (with or without AF), prevention of major cardiovascular and cerebral events (MACCE) in patients with CAD undergoing PCI and receiving single or dual antiplatelet therapy, post-acute coronary syndromes (ACS) patients, heparin induced thrombocytopenia (HIT), prevention of thromboembolic events in heart failure patients and secondary stroke prevention, wherein said methods comprise administering to a subject in need hereof an effective amount of an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401.

In particular embodiments, subjects in need of treatment with an anti-FXI/FXIa antibody described herein, for example, an anti-FXI/FXIa antibody described in Table 1, such as, NOV1401 (e.g., antibody comprising a heavy chain comprising the amino acid sequence of SEQ ID NO: 31 and a light chain comprising the amino acid sequence of SEQ ID NO: 41) or anti-FXI/FXIa antibodies comprising VL CDRs and VHCDRs of NOV1401, may include:

- Subjects with indications for chronic anticoagulation therapy (e.g., AF, left ventricular thrombus, prior cardioembolic stroke);
- subjects at intermediate-to-high risk for major bleeding;
- subjects undergoing elective or primary percutaneous coronary intervention (PCI) with stenting which may be require to receive single or dual antiplatelet therapy (e.g., aspirin and/or P2Y12 receptor antagonists) to prevent stent thrombosis.

In particular embodiments, one of the following conditions can be treated or managed with an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401:

- thromboembolism in subjects with suspected or confirmed cardiac arrhythmia such as paroxysmal, persistent or permanent atrial fibrillation or atrial flutter;
- stroke prevention in atrial fibrillation (SPAF), a subpopulation of which is AF patients undergoing percutaneous coronary interventions (PCI);
- acute venous thromboembolic events (VTE) treatment and extended secondary VTE prevention in patients at high risk for bleeding;
- cerebral and cardiovascular events in secondary prevention after transient ischemic attack (TIA) or non-disabling stroke and prevention of thromboembolic events in heart failure with sinus rhythm;
- clot formation in left atrium and thromboembolism in subjects undergoing cardioversion for cardiac arrhythmia;
- thrombosis before, during and after ablation procedure for cardiac arrhythmia;
- venous thrombosis, this includes but not exclusively, treatment and secondary prevention of deep or superficial veins thrombosis in the lower members or upper member, thrombosis in the abdominal and thoracic veins, sinus thrombosis and thrombosis of jugular veins;
- thrombosis on any artificial surface in the veins like catheter or pacemaker wires;
- pulmonary embolism in patients with or without venous thrombosis;
- Chronic Thromboembolic Pulmonary Hypertension (CTEPH);
- arterial thrombosis on ruptured atherosclerotic plaque, thrombosis on intra-arterial prosthesis or catheter and thrombosis in apparently normal arteries, this includes but not exclusively acute coronary syndromes, ST elevation myocardial infarction, non ST elevation myocardial infarction, unstable angina, stent thrombosis, thrombosis of any artificial surface in the arterial system and thrombosis of pulmonary arteries in subjects with or without pulmonary hypertension;

- thrombosis and thromboembolism in patients undergoing percutaneous coronary interventions (PCI);
- cardioembolic and cryptogenic strokes;
- thrombosis in patients with invasive and non-invasive cancer malignancies;
- thrombosis over an indwelling catheter;
- thrombosis and thromboembolism in severely ill patients;
- cardiac thrombosis and thromboembolism, this includes but not exclusively cardiac thrombosis after myocardial infarction, cardiac thrombosis related to condition such as cardiac aneurysm, myocardial fibrosis, cardiac enlargement and insufficiency, myocarditis and artificial surface in the heart;
- thromboembolism in patients with valvular heart disease with or without atrial fibrillation;
- thromboembolism over valvular mechanic or biologic prostheses;
- injuries or trauma in patients who had native or artificial cardiac patches, arterial or venous conduit tubes after heart repair of simple or complex cardiac malformations;
- venous thrombosis and thromboembolism after knee replacement surgery, hip replacement surgery, and orthopedic surgery, thoracic or abdominal surgery;
- arterial or venous thrombosis after neurosurgery including intracranial and spinal cord interventions;
- congenital or acquired thrombophilia including but not exclusively factor V Leiden, prothrombin mutation, antithrombin III, protein C and protein S deficiencies, factor XIII mutation, familial dysfibrinogenemia, congenital deficiency of plasminogen, increased levels of factor XI, sickle cell disease, antiphospholipid syndrome, autoimmune disease, chronic bowel disease, nephrotic syndrome, hemolytic uremia, myeloproliferative disease, disseminated intra vascular coagulation, paroxysmal nocturnal hemoglobinuria and heparin induced thrombopenia;
- thrombosis and thromboembolism in chronic kidney disease (e.g., stage 3 or 4);
- thrombosis and thromboembolism in end stage renal disease (ESRD);
- arterial and venous thrombosis and thromboembolism in patients with chronic kidney disease or ESRD undergoing hemodialysis including thrombosis or occlusion of the vascular access (e.g. catheter thrombosis, arteriovenous graft, thrombosis or arteriovenous fistula);
- prevention of major cardiac and cerebral events (e.g. cardiovascular death, myocardial infarction, stroke and urgent revascularization) in patients with ESRD undergoing hemodialysis.
- prevention of major adverse limb event (MALE) (e.g. revascularization therapies in critical limb ischemiainclusing amputation or any major vascular reintervention (thrombectomy, thrombolysis, or major surgical procedure [new bypass graft, jump/interposition graft revision]) in the index limb

The incidence and prevalence of atrial fibrillation (AF) in end stage renal disease (ESRD) patients are higher than in the general population and are associated with an increased risk of stroke and mortality. (Zimmerman et al., Nephrol Dial Transplant (2012) 27: 3816-3822). ESRD or end stage renal failure is the late stage of chronic kidney disease (e.g., stage 4), and is typically associated with poor kidney function. For example, the Glomerular Filtration Rate (GFR) of ESRD patients typically indicates less than 15% kidney function. Symptoms of ESRD may include, but are not limited to, anemia, easy bleeding and bruising, headache, fatigue and drowsy feeling, weakness, mental symptoms (such as lowered mental alertness, trouble concentrating, confusion, seizures), nausea, vomiting, generally less desire to eat, muscle cramps, muscle twitching, nocturia (night-time urination), numb sensation in the extremities, diarrhea, itchy skin, itchy eyes, skin color changes (grayish complexion, sometimes yellowish-brownish tone), swelling and puffiness, difficulty breathing (due to fluid in the lungs, anemia), high blood pressure, and poor digestion. A treatment option for ESRD is dialysis (e.g., hemodialysis or peritoneal dialysis).

Therefore, specifically provided herein is a method of preventing, treating or managing or reducing the risk of stroke or thromboembolism (e.g., systemic embolism) comprising administering to a subject afflicated with end stage renal disease (ESRD), an effective amount of a pharmaceutical composition comprising an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401. In specific aspects, the subject is receiving dialysis or extracorporeal membrane oxygenation. In certain aspects, the subject is diagnosed with stage 4 chronic kidney disease. In certain aspects, the subject is diagnosed with stage 3 or 4 chronic kidney disease. In particular aspects, the subject is determined to have a creatinine clearance rate of approximately 15 to 60 mL/min (Cr Cl 15 to 60 mL/min).

Therefore, specifically provided herein is a method of preventing, treating or managing or reducing the risk of stroke or thromboembolism (e.g., systemic embolism) comprising administering to a subject in need thereof who is afflicated with end stage renal disease (ESRD), an effective amount of a pharmaceutical composition comprising an anti-FXI/FXIa antibody comprising a VH and a VL comprising SEQ ID NOs: 29 and 39, respectively, or comprising a heavy chain comprising the amino acid sequence of SEQ ID NO: 31 and a light chain comprising the amino acid sequence of SEQ ID NO: 41. In specific aspects, the subject is receiving dialysis, such as hemodialysis, or extracorporeal membrane oxygenation. In certain aspects, the subject is diagnosed with stage 4 chronic kidney disease. In certain aspects, the subject is diagnosed with stage 3 or 4 chronic kidney disease. In particular aspects, the subject is determined to have a creatinine clearance rate of approximately 15 to 60 mL/min (Cr Cl 15 to 60 mL/min). In specific aspects, the subject also has atrial fibrillation (AF), such as, non-valvular AF.

In specific aspects, specifically provided herein is a method of preventing, treating or managing or reducing the risk of stroke or thromboembolism (e.g., systemic embolism) associated with hemodialysis for ESRD comprising administering to a subject in need thereof who is afflicated with end stage renal disease (ESRD), an effective amount of a pharmaceutical composition comprising an anti-FXI/FXIa antibody comprising a VH and a VL comprising SEQ ID NOs: 29 and 39, respectively, or comprising a heavy chain comprising the amino acid sequence of SEQ ID NO: 31 and a light chain comprising the amino acid sequence of SEQ ID NO: 41. In specific aspects, the subject is receiving dialysis, such as hemodialysis, or extracorporeal membrane oxygenation. In certain aspects, the subject is diagnosed with stage 4 chronic kidney disease. In certain aspects, the subject is diagnosed with stage 3 or 4 chronic kidney disease. In particular aspects, the subject is determined to have a creatinine clearance rate of approximately 15 to 60 mL/min (Cr Cl 15 to 60 mL/min). In specific aspects, the subject also has atrial fibrillation (AF), such as, non-valvular AF.

In another specific aspect, provided herein is a method of preventing or reducing the risk of stroke or thromboembolism comprising administering to a subject afflicated with atrial fibrillation (AF) and ESRD and who is undergoing dialysis, such as hemodialysis, or extracorporeal membrane oxygentation, an effective amount of a pharmaceutical composition comprising an anti-FXI/FXIa antibody described herein, for example, an anti-FXI/FXIa antibody described in Table 1, such as, NOV1401 or anti-FXI/FXIa antibodies comprising VL CDRs and VHCDRs of NOV1401. In particular aspects, the subject is afflicted with non-valvular AF. In further specific aspects, the subject is afflicted with non-valvular AF with a demonstrated high risk of bleeding. In certain aspects, the subject has a high bleeding risk as characterized by a $CHA_2DS_2VASc$ risk score $\geq$ 2.

In another specific aspect, provided herein is a method of preventing or reducing the risk of stroke or thromboembolism comprising administering to a subject afflicated with atrial fibrillation (AF) and ESRD and who is undergoing dialysis, such as hemodialysis, an effective amount of a pharmaceutical composition comprising an anti-FXI/FXIa antibody described herein, for example, an anti-FXI/FXIa antibody comprising a VH comprising the amino acid sequence of SEQ ID NO: 29 and a VL comprising the amino acid sequence of SEQ ID NO: 39, or a heavy chain comprising the amino acid sequence of SEQ ID NO: 31 and a light chain comprising the amino acid sequence of SEQ ID NO: 41. In particular aspects, the subject is afflicted with non-valvular AF. In further specific aspects, the subject is afflicted with non-valvular AF with a demonstrated high risk of bleeding. In certain aspects, the subject has a high bleeding risk as characterized by a $CHA_2DS_2VASc$ risk score $\geq$ 2.

In a specific aspect, provided herein is a method of preventing, treating or managing or reducing the risk of stroke or thromboembolism, such as systemic embolism, comprising administering to a subject in need thereof about 100 mg to about 250 mg of an anti-FXI/FXIa antibody (e.g. anti-FXI/FXIa antibody comprising a VH comprising the amino acid sequence of SEQ ID NO: 29 and a VL comprising the amino acid sequence of SEQ ID NO: 39, or a heavy chain comprising the amino acid sequence of SEQ ID NO: 31 and a light chain comprising the amino acid sequence of SEQ ID NO: 41), or antigen-binding fragment thereof that specifically binds within the catalytic domain of FXI, wherein the subject has one, two, or more, or all, of the following characteristics:

(i) Male or female subjects $\geq$ 55 and < 85 years old;

(ii) Body weight between 50 and 130 kg inclusive;

(iii) Atrial fibrillation (AF) or atrial flutter, as documented by Electrocardiography;

(iv) $CHA_2DS_2$-VASc risk score $\geq$ 2 for male and female subject;

(v) Male subject with $CHA_2DS_2VASc$ risk score of 1 and anticoagulation therapy is warranted; and

(vi) Either anticoagulant-naïve or receiving a stable treatment of a recommended dose of a new oral anticoagulant (NOAC) over the 8 weeks prior to treatment.

In particular aspects, subjects with moderate to high risk for stroke and systemic embolism have a $CHA_2DS_2VASc$ risk score $\geq$ 2. In further particular aspects, subjects with a HAS BLED risk score $\geq$ 3 is characterized as having a high risk of bleeding (see Gallego, et al., (2012) Carc Arrhythm Electrophysiol.; 5:312-318, and Friberg et al., (2012) Circulation.; 125:2298-2307).

The risk of thromboembolic events including stroke, systemic embolism, coronary or peripheral artery thrombosis, venous thrombosis and pulmonary embolism increases with presence of predisposing factors such as thrombophilia, vessel wall damage and stasis. Evaluation of medical history, familiar antecedents and associated co-morbidities can help to stratify patients according to their thromboembolic risks. In patients with AF, several scoring systems e.g., CHADS2 and $CHA_2DS_2$-VASc have been developed to assess stroke risk. Each was developed based on data from randomized trials, and clinical and epidemiologic cohort studies, and translated a weighted, multivariate formula of stroke risk factors to a simplified, easy-to-use mnemonic device, algorithm, calculator, or online tool. The CHADS2 risk score was used stratification tool to predict thromboembolic risk in AF patients (LIP 2011; Camm et al 2012); however, accumulated evidence shows that $CHA_2DS_2$-VASc is at least as good as or possibly better than, scores such as CHADS2 in identifying patients who develop stroke and thromboembolism and definitively better at identifying 'truly low-risk' patients with AF. The $CHA_2DS_2$-VASc score is presently recommended by Guidelines (Camm et al Eur Heart J (2012) 33, 2719-2747; January et al, AHA/ACC/HRS Atrial Fibrillation Guideline; J Am Coll Cardiol 2014;64:2246-80) to guide the decision with regard to patients who should benefit of anticoagulant therapy and also to identify low risk patients in whom anticoagulation therapy is not warranted.

Bleeding risk assessment tools specific to the AF patients e.g., HAS-BLED, ATRIA, HEMORR2HAGES; ORBIT and ABC risk score were developed to predict the bleeding risk in patients with AF. Unfortunately, as the bleeding risk is tightly correlated with the stroke risk, those risk score were of rather limited value to guide therapeutic decisions to use vitamin K antagonists such as warfarin or NOACS. However, bleeding risk scores may become of considerable help to identify patients who can benefit of a new therapy with a reduced bleeding risk e.g. anti-FXI/FXIa antibody (e.g., antibody NOV1401).

In certain aspects, subjects with demonstrated high risk of bleeding can be identified by previous medical history of bleeding, for example, bleeding during or after surgery or bleeding when treated with an anticoagulant (e.g. Warfarin). In addition, subjects with demonstrated high risk of bleeding can be identified by in vitro/ex vivo assays known in the art, for example, aPTT assay with a subject's plasma. Other non-limiting examples of bleeding risk factors include: history of previous stroke/TIA, previous major bleed or clinically relevant bleed, CKD stage 3 or 4 (Cr Cl 15 to 60 Ml/min), treatment with dual antiplatelet therapy, e.g., post-ACS/PCI, and history of falls associated with hematoma bruise or other manifestations.

In specific aspects, a subject being treated by the methods provided herein is at least 18 years old. In another aspect, a subject being treated by the methods provided herein is at least 50 years old. In another aspect, a subject being treated by the methods provided herein is at least 55 years old. In another aspect, a subject being treated by the methods provided herein is at least 60 years old. In another aspect, a subject being treated by the methods provided herein is at least 65 years old.

In specific aspects, a subject being treated by the methods provided herein has a body mass index (BMI) that is greater than or equal to 18 $kg/m^2$. In another aspect, a subject being treated by the methods provided herein has a BMI that is greater than or equal to 30 $kg/m^2$. In another aspect, a subject being treated by the methods provided herein has a BMI that is greater than or equal to 35 $kg/m^2$. In another aspect, a subject being treated by the methods provided herein has a BMI that is greater than or equal to 40 $kg/m^2$. In a specific aspect, a subject being treated by the methods provided herein is a human subject.

In specific aspects, methods provided herein results in one or more of the therapeutic benefits: (i) anticoagulation; (ii) reduction in hospital stay period, (iii) prolongation of clotting time, for example, as determined by aPTT; (iv) reduction in incidence of thrombosis or frequency of thrombosis; and (v) reduction in one or more symptoms associated with thromboembolic disorders (e.g., stroke, systemic embolism, myocardial infarction, deep venous thrombosis, pulmonary embolism, cardiovascular death, need for urgent revascularization) or reduction in severity of one or more symptoms associated with thromboembolic disorders. In particular aspects, methods provided herein results in prolongation of clotting time, for example, as determined by aPTT by 1.2 fold, 1.3 fold, 1.4 fold, 1.5 fold, 1.6 fold, 1.7 fold, 1.8 fold, 1.9 fold, or 2 fold.

In specific aspects, the present disclosure provides a method of treating, preventing, or managing or reducing the risk of thromboembolic disorders by administering to a subject in need thereof an effective amount of an anti-FXI/FXIa antibody described herein (e.g., NOV1401) in combination with another therapeutic active agent. Such combination therapies may be useful for treating thromboembolic disorders, such as, ischemic stroke (cardioembolic, thrombotic) or systemic embolism, AF, stroke prevention in AF (SPAF), deep vein thrombosis, venous thromboembolism, pulmonary embolism, acute coronary syndromes (ACS), acute limb ischemia, chronic thromboembolic pulmonary hypertension, or systemic embolism).

In particular aspects, non-limiting examples of therapeutic active agents suitable for use in combination with an anti-FXI/FXIa antibody described herein (e.g., NOV1401) include thromboxane inhibitors (e.g., aspirin), adenosine diphosphate receptor antagonists (or P2Y12 inhibitors) such as thienopyridines (e.g., clopidogrel and prasugrel) and nonthienopyridines (e.g., ticagrelor and cangrelor), protease-activated receptor-1 (PAR1) antagonists (e.g., vorapaxar and atopaxar), and proton pump inhibitors (PPIs) (e.g., omeprazole, diazepam, phenytoin, lansoprazole, dexlansoprazole, rabeprazole, pantoprazole, esomeprazole, and naproxen). The use of PPIs in combination therapy may be suitable in cases where a subject has or has a history of a GI disorder, such as previous GI bleed or antecedent of peptic ulcer.

In a specific aspect, provided herein are methods of managing bleeding in a patient being treated or administered an anti-FXI/FXIa antibody provided herein (e.g., an antibody described in Table 1, such as, an anti-FXI/FXIa antibody comprising VL CDRs and VHCDRs of NOV1401), for example, bleeding associated with trauma, surgery menstruation or post-delivery, said method comprises reversing of the anticoagulant effect. FXI deficiency is rarely associated with spontaneous bleeding manifestations; in specific aspects, bleeding is most typically associated with trauma, surgery, menstruation or post-delivery. Prolonged bleeding may occur after a major trauma or after surgery involving organs with high fibrinolytic area such as buccal, nasal, genital or urinary mucosa. Tooth extraction, tonsillectomy and ablation of the uterus or prostate are examples of surgeries that entail a high risk of bleeding. People with the disorder also have a strong tendency to develop epistaxis and ecchymoses, and more rarely, bleeding into the urine or intestines. Spontaneous muscle or joint and intracranial bleeding frequency is not increased in patients with FXI deficiency. Venous puncture is not usually associated with an extended bleeding. Other genetic mutations associated with FXI deficiency may contribute to the heterogeneous and unpredictable bleeding tendency in patients with severe FXI deficiency. Concomitant use of antiplatelets, other anticoagulants and fibrinolytic agents can increase the risk of bleeding.

In particular embodiments, provided herein is a method of managing bleeding in a patient (e.g., patient with ESRD and/or AF) being treated with an anti-FXI/FXIa antibody provided herein (*e.g.,* an antibody described in Table 1, such as, an anti-FXI/FXIa antibody comprising VL CDRs and VHCDRs of NOV1401), said method comprises temporarily reversing of the anticoagulant effect for a sufficient time to manage the bleeding. In specific embodiments, the step of reversing of the anticoagulant effect comprises (i) fluid replacement using colloids, crystalloids, human plasma or plasma proteins such as albumin; or (ii) transfusion with packed red blood or whole blood. In a particular embodiment, therapeutic agents for reversal of the effect of anticoagulants, for example, in cases of severe emergency, include, but are not limited to, prohemostasis blood components such as fresh frozen plasma (FFP), prothrombin complex concentrates (PCC) and activated PCC [(APCC); e.g. factor VIII inhibitor bypass activity (FEIBA)] and recombinant activated factor VII (rFVIIa). In one embodiment, a regimen comprising administration of rFVIIa at a dose of 30 $\mu$g/kg followed by administration of rFVIIa at a dose of 15-30 $\mu$g/kg every 2-4 hours for 24-48 hours in addition to tranexamic acid 1 g every 6 hours for 5 to 7 days may have potential to recover hemostasis and stop bleeding in subjects treated with an anti-FXI/FXIa antibody provided herein (e.g., NOV1401 or an antibody comprising VL CDRs and VH CDRs of NOV1401) who are undergoing major surgery and in patients with an active non-accessible bleeding site. For instance, Riddell et al reported experience in 4 patients with severe FXI deficiency without inhibitor undergoing surgery (Riddell et al., 2011, Thromb. Haemost.; 106: 521-527); patients were administered rFVIIa 30 $\mu$g/kg and tranexamic acid 1 g i.v. at induction of anesthesia. Subsequent bolus doses of rFVIIa 15-30 $\mu$g/kg were administered at 2 to 4 hourly intervals as guided by rotational thromboelastometry (ROTEM) results. Patients were treated with rFVIIa at above mentioned doses for 24-48 hours. Tranexamic acid 1 g every six-hourly was continued for five days. In this small series, rFVIIa at doses as low as 15-30 $\mu$g/kg in combination with tranexamic acid was safe and effective in correcting the hemostatic defect in severe FXI deficiency in this study. In another study comprising 4 patients with severe FXI deficiency with inhibitor (autologous neutralizing FXI antibodies usually acquired after transfusion or administration of blood products to patients with severe FXI deficiency) who experienced 5 surgeries, the authors (Livnat et al., 2009, Thromb. Haemost.; 102: 487-492) applied the following protocol: 1 g of tranexamic acid orally two hours before surgery, then patients received immediately prior to the interventions another 1 g tranexamic acid i.v. Recombinant FVIIa at doses ranging from 15 to 30 $\mu$g/kg was infused at the completion of surgery. Subsequently, oral tranexamic acid 1 g was given every 6 hour for at least 7 days. Fibrin glue was sprayed at the bed of the extirpated gallbladder in one patient. This protocol secured normal hemostasis in patients with severe FXI deficiency with inhibitor.

In one aspect, fibrin glue can be used to restore local hemostasis during dental surgery in patients with FXI deficiency (Bolton-Maggs (2000) Haemophilia; 6 (S1):100-9). In a certain embodiment with respect to methods to manage bleeding in patients being treated with an anti-FXI/FXIa antibody provided herein (*e.g.,* NOV1401), a regimen consisting of tranexamic acid 1 g every 6 hours for 5 to 7 days associated with the use of fibrin glue could be used to establish local hemostasis in subjects undergoing minor surgery and in subjects with accessible bleeding site, including oral and nasal bleeding events.

In a particular aspect, provided herein are methods of managing bleeding or bleeding risk in a patient (e.g., patient with ESRD and/or AF) treated or administered an anti-FXI antibody described herein *(e.g.,* antibody described in Table 1 such as NOV1401 or an anti-FXI antibody comprising HCDRs and LCDRs of NOV1401), comprising the step of administering to the patient in need thereof, an anti-idiotype antibody, or antigen binding fragment thereof (*e.g.,* Fab), of the anti-FXI antibody, wherein the anti-idiotype or antigen binding fragment thereof (*e.g.,* Fab) specifically binds to the anti-FXI antibody and blocks the anti-FXI antibody from binding to FXI. In specific embodiments, an anti-idiotype antibody or antigen binding fragment thereof (*e.g.,* Fab) reverses the effects of an anti-FXI antibody described herein to mitigate bleeding risks, for example during urgent major surgery or trauma.

In specific aspects, an anti-idiotype antibody or antigen binding fragment thereof (e.g., Fab) reverses or inhibits an anti-FXI antibody's anti-coagulant effects. In particular aspects, the anti-idiotype antibody or antigen binding fragment thereof (*e.g.*, Fab) is administered to a patient in need thereof to temporarily reverse the anti-coagulant effect of an anti-FXI antibody described herein (*e.g.*, antibody described in Table 1 such as NOV1401 or an anti-FXI antibody comprising HCDRs and LCDRs of NOV1401).

In a particular aspect, provided herein are methods of managing bleeding or bleeding risk in a patient treated or administered an anti-FXI antibody such as NOV1401 (*e.g.,* SEQ ID NOs: 31 and 41), comprising the step of administering to the patient in need thereof, an anti-idiotype antibody, or antigen binding fragment thereof (*e.g.,* Fab), of the anti-FXI antibody such as NOV1401 (*e.g.,* SEQ ID NOs: 31 and 41), wherein the anti-idiotype, or antigen binding fragment thereof (*e.g.,* Fab), specifically binds to the antigen-binding region of an anti-FXI antibody such as NOV1401 (*e.g.,* SEQ ID NOs: 31 and 41) and blocks the anti-FXI antibody from binding to FXI and/or FXIa. In a specific embodiment, the anti-idiotype antibody, or antigen binding fragment thereof (*e.g.,* Fab), of an anti-FXI antibody such as NOV1401 (*e.g.,* SEQ ID NOs: 31 and 41) reverses or inhibits one or more of the anti-coagulant effects of the anti-FXI antibody (*e.g.,* NOV1401). In certain embodiments, a temporary reversal or inhibition of one or more of the anti-coagulant effects of the anti-FXI antibody (e.g., NOV1401) is achieved. In specific embodiments, following the temporary reversal or inhibition of the anti-FXI antibody *(e.g.,* NOV1401), the anti-FXI antibody *(e.g.,* NOV1401) is again administered to the patient.

Anti-Idiotype antibodies can be produced by various methods described previously, see, *e.g.,* Pan et al., 1995, FASEB J. 9:43-49. Anti-idiotype antibodies are elicited by an antibody molecule (*e.g.,* NOV1401) and are directed against antigenic determinants in or close to the antibody's combining site (idiotope). Anti-idiotype antibodies recognize antigenic determinants that overlap a portion of the combining site that is in contact with the original antigen and they can mimic the eliciting antigen.

**Pharmaceutical Compositions**

The present disclosure provides pharmaceutical compositions comprising FXIa-binding antibodies (intact or binding fragments), e.g., anti-FXI/FXIa antibodies described in Table 1, formulated together with a pharmaceutically acceptable carrier, for use in the methods described herein (e.g., methods of reducing the risk of stroke and/or systemic embolism). The compositions can additionally contain one or more other therapeutic agents that are suitable for treating or preventing, for example, thromboembolic disorders (*e.g.,* thrombotic disorders). Pharmaceutically acceptable carriers enhance or stabilize the composition, or can be used to facilitate preparation of the composition. Pharmaceutically acceptable carriers include solvents, dispersion media, coatings, antibacterial and antifungal agents, isotonic and absorption delaying agents, and the like that are physiologically compatible.

A pharmaceutical composition for use the present disclosure can be administered by a variety of methods known in the art. The route and/or mode of administration vary depending upon the desired results. It is preferred that administration be intravenous (i.v.), intramuscular (i.m.), intraperitoneal (i.p.), or subcutaneous (s.c.), or administered proximal to the site of the target. The pharmaceutically acceptable carrier should be suitable for intravenous, intramuscular, subcutaneous, parenteral, spinal or epidermal administration (*e.g.,* by injection or infusion). Depending on the route of administration, the active compound, *i.e.,* antibody, bispecific and multispecific molecule, may be coated in a material to protect the compound from the action of acids and other natural conditions that may inactivate the compound.

In particular aspects, anti-FXI/FXIa antibodies described herein (*e.g.,* antibodies described in Table 1, such as NOV1401 or antibodies comprising LCDRs and HCDRs of NOV1401) are formulated at approximately 75 mg/1 mL to approximately 200 mg/1 mL concentration, in liquid vials for subcutaneous injections. In particular embodiments, the pharmaceutical composition comprises a pharmaceutical carrier or excipient, for example, sucrose, and polysorbate 20. In particular embodiments, the pharmaceutical composition comprises L-histidine and/or histidine HCl monohydrate. In certain embodiments, the pharmaceutical composition has a pH of approximately 4 to 7, or 5 to 6.

In particular aspects, anti-FXI/FXIa antibodies described herein (e.g., antibodies described in Table 1, such as NOV1401 or antibodies comprising LCDRs and HCDRs of NOV1401) are formulated at 150 mg/1 mL concentration, in liquid vials for subcutaneous injections. In one embodiment, the 150 mg/mL liquid formulation contains 150 mg anti-FXI/FXIa antibody, L-histidine, histidine HCl monohydrate, sucrose, and polysorbate 20, with a pH = 5.5 $\pm$ 0.5. The composition should be sterile and fluid. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

Pharmaceutical compositions for use the present disclosure can be prepared in accordance with methods well known and routinely practiced in the art. See, *e.g.,* Remington: The Science and Practice of Pharmacy, Mack Publishing Co., 20th ed., 2000; and Sustained and Controlled Release Drug Delivery Systems, J.R. Robinson, ed., Marcel Dekker, Inc., New York, 1978. Pharmaceutical compositions are preferably manufactured under GMP conditions. Typically, a therapeutically effective dose or efficacious dose of the FXIa-binding antibody is employed in the pharmaceutical compositions of the present disclosure. The FXIa-binding antibodies are formulated into pharmaceutically acceptable dosage forms by conventional methods known to those of skill in the art. Dosage regimens are adjusted to provide the optimum desired response (*e.g.,* a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the present disclosure can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present disclosure employed, thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

A physician can start doses of the antibodies of the present disclosure employed in the pharmaceutical composition at levels lower than that required to achieve the desired therapeutic effect and gradually increase the dosage until the desired effect is achieved. In general, effective doses of the compositions of the present disclosure, for the treatment of a thrombotic and/or thromboembolic disorders described herein vary depending upon many different factors, including means of administration, target site, physiological state of the patient, other medications administered, and whether treatment is prophylactic or therapeutic. Treatment dosages need to be titrated to optimize safety and efficacy. For systemic administration with an antibody, the dosage ranges from about 0.01 to 15 mg/kg of the host body weight. For administration (*e.g.,* subcutaneous administration) with an antibody, the dosage may range from 0.1 mg to 5 mg or from 1 mg to 600 mg. For example, an anti-FXI/FXIa antibody described herein can be administered at a dose of 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg, 1.0 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2.0 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4.0 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, or 5.0 mg/kg. An exemplary treatment regime entails systemic administration once per every two weeks or once a month or once every 3 to 6 months. An exemplary treatment regime entails systemic administration once per week, once per every two weeks, once per every three weeks, once a month, or once every 3 to 6 months, or as needed (PRN).

In a certain embodiment, an anti-FXI/FXIa antibody described herein (e.g., an antibody described in Table 1, such as NOV1401 or an antibody comprising VL CDRs and VH CDRs of NOV1401) is administered, for example by i.v. or s.c., at a dose of 3 mg/kg. In a specific aspect, such anti-FXI/FXIa antibody is administered once every week, once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401) is administered, for example by i.v. or s.c., at a dose of 10 mg/kg. In a specific aspect, such anti-FXI/FXIa antibody is administered once every week, once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401) is administered, for example by i.v. or s.c., at a dose of 30 mg/kg. In a specific aspect, such anti-FXI/FXIa antibody is administered once every week, once every two weeks, once every 3 weeks, or once every 4 weeks (or once every month).

In a certain embodiment, an anti-FXI/FXIa antibody described herein (*e.g.,* an antibody described in Table 1, such as NOV1401 or an antibody comprising VL CDRs and VH CDRs of NOV1401) is administered, for example by i.v. or s.c., at a dose of 50 mg/kg. In a specific aspect, such anti-FXI/FXIa antibody is administered once every week, once every two weeks, once every 3 weeks, or once every 4 weeks (or once every month).

In a certain embodiment, an anti-FXI/FXIa antibody described herein (*e.g.,* an antibody described in Table 1, such as NOV1401 or an antibody comprising VL CDRs and VH CDRs of NOV1401) is administered, for example by i.v. or s.c., at a dose of 100 mg/kg. In a specific aspect, such anti-FXI/FXIa antibody is administered once every week, once every two weeks, once every 3 weeks, or once every 4 weeks (or once every month).

In a certain embodiment, an anti-FXI/FXIa antibody described herein (*e.g.,* an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered, for example by i.v. or s.c. route, at a dose in the range of 5 mg to 600 mg. In a certain embodiment, an anti-FXI/FXIa antibody described herein (*e.g.,* an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered, for example by i.v. or s.c. route, at a dose in the range of 100 mg to 200 mg, for example, once every week, once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered, for example by i.v. or s.c. route, to a subject in need thereof (e.g., subject with atrial fibrillation and/or with ESRD) at a dose of approximately 5 mg, 10 mg, 15 mg, 20 mg, 30 mg, 40 mg, 50 mg, 60 mg, 90 mg, 100 mg, 120 mg, 150 mg, 180 mg, 200 mg, 210 mg, 240 mg, 250 mg, 270 mg, 300 mg, 330 mg, 350 mg, 360 mg, 390 mg, 400 mg, 420 mg, 450 mg, 480 mg, 500 mg, 510 mg, 540 mg, 550 mg, 570 mg, or 600 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 5 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 15 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 50 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 120 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 150 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 180 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (*e.g.,* an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 210 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (*e.g.,* an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 300 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 600 mg. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 5 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein *(e.g.,* an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 15 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 50 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 120 mg or greater. In a specific aspect, such anti-FXI/FXIa antibody is administered once every week, once every two weeks, once every 3 weeks, or once every 4 weeks (or once every month). In a particular aspect,

such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 150 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 180 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 210 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401), for use in the methods provided herein (e.g., methods for preventing, treating or managing or reducing the risk of stroke or thromboembolism such as systemic embolism), is administered to a subject in need thereof (e.g., subject with atrial fibrillation and/or with ESRD), for example by s.c. route, at a dose of 300 mg or greater. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month).

In a certain embodiment, an anti-FXI/FXIa antibody described herein (e.g., an antibody described in Table 1, such as NOV1401 or an antibody comprising VL CDRs and VH CDRs of NOV1401) is administered, for example by i.v. or s.c. route, at a dose sufficient to achieve a mean duration of aPTT prolongation of 2-fold or greater for a period no longer than 30 days, 35 days, 36 days, 37 days, 38 days, 39 days, 40 days, 41 days, 42 days, 43 days, 44 days, 45 days, or 50 days.

In a certain embodiment, an anti-FXI/FXIa antibody described herein (e.g., an antibody described in Table 1, such as NOV1401 or an antibody comprising VL CDRs and VH CDRs of NOV1401) is administered, for example by i.v. or s.c. route, at a dose sufficient to achieve a mean duration of aPTT prolongation of 2-fold or greater for a period no longer than 42 days.

In a certain embodiment, antibody NOV1401 (e.g., antibody comprising a heavy chain comprising the amino acid sequence of SEQ ID NO: 31 and a light chain comprising the amino acid sequence of SEQ ID NO: 41) or a pharmaceutical composition comprising NOV1401 and a pharmaceutically acceptable carrier is for use in preventing, treating, or managing or reducing the risk of stroke or thromboembolism, such as systemic embolism, associated with treatment for ESRD, such as hemodialysis, wherein the antibody is administered subcutaneously, at a dose in the range of 100 mg to 300 mg.

In a specific aspect, antibody NOV1401 (e.g., antibody comprising a heavy chain comprising the amino acid sequence of SEQ ID NO: 31 and a light chain comprising the amino acid sequence of SEQ ID NO: 41) or a pharmaceutical composition comprising NOV1401 and a pharmaceutically acceptable carrier is for use in preventing, treating, or managing or reducing the risk of stroke or thromboembolism, such as systemic embolism, associated with treatment for ESRD, such as hemodialysis, in a subject, wherein the antibody is administered subcutaneously, at a dose of approximately 100 mg, 120 mg, 150 mg, 180 mg, 210 mg, or 240 mg, once a month. In specific aspects, the subject is afflicted with AF and ESRD.

In a certain embodiment, antibody NOV1401 (e.g., antibody comprising a heavy chain comprising the amino acid sequence of SEQ ID NO: 31 and a light chain comprising the amino acid sequence of SEQ ID NO: 41), or a pharmaceutical composition comprising NOV1401 or an antigen-binding fragment thereof and a pharmaceutically acceptable carrier, is for use in preventing, treating, or managing or reducing the risk of stroke or thromboembolism, such as systemic embolism, in a subject afflicted with AF, such as non-vavular AF, wherein the antibody is administered subcutaneously, at a dose in the range of 100 mg to 300 mg., once a month. In specific aspects, the subject has non-valvular AF with a demonstrated high risk of bleeding, for example, as determined by a $CHA_2DS_2VASc$ risk score $\geq 2$, and wherein the subject is 55 years old or older.

In a specific aspect, antibody NOV1401 (e.g., antibody comprising a heavy chain comprising the amino acid sequence of SEQ ID NO: 31 and a light chain comprising the amino acid sequence of SEQ ID NO: 41), or a pharmaceutical composition comprising NOV1401 or an antigen-binding fragment thereof and a pharmaceutically acceptable carrier, is for use in preventing, treating, or managing or reducing the risk of stroke or thromboembolism, such as systemic embolism, in a subject afflicted with AF, such as non-valvular AF, wherein the antibody is administered subcutaneously, at a dose of approximately 100 mg, 120 mg, 150 mg, 180 mg, 210 mg, 240 mg, or 300 mg, once a month. In specific aspects, the subject has non-valvular AF with a demonstrated high risk of bleeding, for example, as determined by a $CHA_2DS_2VASc$ risk score $\geq 2$, and wherein the subject is 55 years old or older. In a specific aspect, such anti-FXI/FXIa antibody is administered once every week, once every two weeks, once every 3 weeks, or once every 4 weeks (or once every month). In a particular aspect, such anti-FXI/FXIa antibody is administered once every two weeks, once every 3 weeks, or once every 4 weeks (or once every month). In a specific aspect, such anti-FXI/FXIa antibody is administered at a dose of approximately 100 mg, 110 mg, 120 mg, 130 mg, or 140 mg once every week or once every two weeks. In a specific aspect, such anti-FXI/FXIa antibody is administered at a dose of approximately 140 mg, 150 mg, 160 mg, 170 mg, or 180 mg, once every 3 weeks or once every 4 weeks (or once every month). In a particular aspect, such anti-FXI/FXIa antibody is administered at a dose of 150 mg or 1 80 mg once every 3 weeks or once every 4 weeks (or once every month).

In a specific aspect, antibody NOV1401 (e.g., antibody comprising a heavy chain comprising the amino acid sequence of SEQ ID NO: 31 and a light chain comprising the amino acid sequence of SEQ ID NO: 41), or a pharmaceutical composition comprising NOV1401 or an antigen-binding fragment thereof and a pharmaceutically acceptable carrier, is for use in preventing, treating, or managing or reducing the risk of stroke or thromboembolism, such as systemic embolism, in a subject afflicted with AF, such as non-valvular AF, wherein the antibody is administered subcutaneously, at a dose of 120 mg, 150 mg, or 180 mg, once a month. In specific aspects, the subject has non-valvular AF with a demonstrated high risk of bleeding, for example, as determined by a $CHA_2DS_2VASc$ risk score $\geq 2$, and wherein the subject is 55 years old or older.

Antibody is usually administered on multiple occasions. Intervals between single dosages can be weekly, biweekly, monthly or yearly. Intervals can also be irregular as indicated by measuring blood levels of FXI- and/or FXIa-binding antibody in the patient. In addition alternative dosing intervals can be determined by a physician and administered monthly or as necessary to be efficacious. In some methods of systemic administration, dosage is adjusted to achieve a plasma antibody concentration of 1-1000 $\mu$g/mL or 1-1200 $\mu$g/mL, and in some methods 25-500 $\mu$g/mL. Alternatively, antibody can be administered as a sustained release formulation, in which case less frequent administration is required. Dosage and frequency vary depending on the half-life of the antibody and its target in the patient. In general, human and humanized antibodies show longer half-life, in humans, than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regimen.

EXAMPLES

The following examples are provided to further illustrate the present disclosure but not to limit its scope. Other variants of the present disclosure will be readily apparent to one of ordinary skill in the art and are encompassed by the appended claims.

**Example 1**

**Example 1**

**Binding Data**

**Surface plasmon resonance (SPR) analysis for the FXI catalytic domain.**

The SPR measurements were performed on a BIACORE™ T200 surface plasmon resonance based optical biosensor (BIACORE™, GE Healthcare, Uppsala). Series S sensor chips (CM5), immobilization kits and regeneration buffer were purchased from GE Healthcare (Uppsala). Two different assay setups were performed depending on the ligand format, IgG or Fab. First, the surface was activated by *N*-hydroxysuccinimide (NHS) and *N*-(3-dimethylaminopropyl)-*N*-ethylcarbodiimide hydrochloride (EDC). The NOV1401-Fab was covalently attached to the activated dextran matrix on a CM5 chip by the standard amine-coupling method (GE Healthcare, Uppsala). For the NOV1401-IgG a capture assay was performed and a goat anti-human IgG-Fc antibody (JIR) was immobilized on the chip at 14000 RUs. Remaining active surface groups were inactivated with Ethanolamine (EA). A reference cell without immobilized ligand was prepared and the system equilibrated with 1x HBS-EP+ buffer (10 mM HEPES, 150 mM NaCl, 3 mM EDTA, 0.05% P20, pH 7.4, Teknova H8022).

All binding experiments were performed at 25 °C at a flow rate of 50 $\mu$L/min using HBS-EP+ buffer. For the capture assay NOV1401-IgG was captured until reaching an RU level of 80. For kinetic studies a dilution series of the FXI catalytic domain with concentrations ranging from 0-200 nM in HBS-EP+ buffer was used. Association time was 120 s and the dissociation time 180 s. The surface was regenerated with a single injection of 10 mM Glycine pH 1.5 (contact time 60 s, stabilization time 120 s). Data processing as well as $k_{on}$, $k_{off}$, and $K_D$ determination were accomplished with the T200 BiaEvaluation software version 1.0. Double referencing (subtraction of reference and blank injection) was applied to correct for bulk effects and other systematic artefacts. Sensograms were fitted by applying a 1:1 binding model ($R_{max}$ set at global).

**Solution Equilibrium Titration (SET) for FXI and FXIa**

22 serial 1.6n dilutions of the antigens were prepared in sample buffer (PBS pH 7.4 containing 0.5 % BSA and 0.02 % Tween 20) and a constant concentration of NOV1401-Fab (200 pM for huFXI and 500 pM for huFXIa) or NOV1401-Antibody (10 pM for huFXI and huFXIa) was added to each antigen concentration. Starting concentrations for the antigen dilution series were 100 nM for huFXIa and 20 nM huFXI (Fab assay) or 1 nM (IgG assay). 60 $\mu$l/well of each dilution mix was distributed in duplicates to a 384-well polypropylene MTP. Sample buffer served as negative control and a sample containing no antigen as positive control (Bmax). The plate was sealed and incubated overnight at RT on a shaker. A standard 384-well MSD array MTP was coated with 30 $\mu$l/well of 0.1 $\mu$g/ml huFXIa (for huFXIa and huFXI) diluted in PBS, sealed and incubated overnight at 4°C.

After incubation and three times washing with TBST (TBS containing 0.05 % Tween 20) the antigen-coated MSD plate was blocked with 50 $\mu$l/well blocking buffer (PBS containing 5 % BSA) and incubated for 1 h at RT on a shaker. The wash step was repeated and 30 $\mu$l/well of the Fab-/IgG-antigen preparation was transferred from the polypropylene MTP to the antigen coated MSD plate and incubated for 20 min at RT on a shaker. After an additional wash step, 30 $\mu$l of 0.5 $\mu$g/ml ECL-labeled goat anti-human-IgG/Fab detection antibody (MSD) diluted in sample buffer was added to each well and incubated for 30 min at RT with shaking. After washing the plate again three times, 35 $\mu$l of read buffer (MSD) was added to each well. Electrochemiluminescence (ECL) signals were generated and detected with the MSD Sector Imager 6000. Average ECL-signals were calculated from duplicate measurements within each assay. Data were baseline adjusted by subtracting the lowest value from all data points and plotted against the corresponding antigen concentration. $K_D$ values were determined by fitting the plot with the following 1:1 (for Fab) or 1:2 (for IgG) fit model (according to Piehler *et al,.* 1997).

**Results**

The results are summarized in **Tables 3 and 4.** For both NOV1401 formats, Fab and IgG, $K_D$ values of approximately 20 nM were obtained for the FXI catalytic domain as determined by BIACORE™. Affinities of the Fab to both the activated and zymogen FXI were in the pM range and were 66 and 300 times higher than the affinity to the catalytic domain, respectively. Based on their high affinities these interactions were measured by SET assays. The NOV1401-Fab exhibited a five-fold higher affinity to the zymogen FXI (62 pM) than to the activated FXI (305 pM). Affinities of the NOV1401-IgG to both the dimeric zymogen and activated FXI are marked as apparent $K_D$ values since the interaction might influenced by avidity effects.

To confirm that NOV2401 also binds to cynomolgus monkey FXI, SET experiments were performed with activated cynomolgus monkey FXI and cynomolgus monkey FXI zymogen resulting in apparent $K_D$ values of 12.5 $\pm$ 6.6 pM (N=2) and 5.0 $\pm$ 0.7 pM (N=2), respectively. Hence, the affinities of NOV1401 to cynomolgus monkey FXI proteins (active form and zymogen) are comparable to those for binding to human FXI (Table 3).

**Table 2.** $K_D$ values and kinetic binding parameters of NOV1401-Fab/IgG for human FXI catalytic domain as determined by BIACORE[TM].

| Catalytic domain | $k_a$ (1/Ms) | $k_d$ (1/s) | $K_D$ (nM) | n |
|---|---|---|---|---|
| NOV1401-Fab | 3.2 ± 0.5 E+4 | 6.1 ± 1.8 E-4 | 19 ± 6 | 3 |
| NOV1401-IgG | 4.2 ± 1.6 E+4 | 8.8 ± 2.2 E-4 | 21 ± 3 | 2 |

**Table 3.** $K_D$ values of NOV1401-Fab/IgG for human FXI activated and zymogen determined by the in solution equilibrium titration (SET). * Only apparent $K_D$ values reported, since the interaction might be influenced by avidity effects.

| NOV1401-Fab | $K_D$ [pM] | N |
|---|---|---|
| Human FXI activated | 305 ± 8 | 3 |
| Human FXI zymogen | 62 ± 18 | 3 |
| NOV1401-IgG | | |
| Human FXI activated | 4.7 ± 2.1* | 3 |
| Human FXI zymogen | 1.3 ± 0.3* | 3 |

Reference: Piehler et al. Assessment of affinity constants by rapid solid phase detection of equilibrium binding in a flow system, J.Immunol. Meth. 1997. 189-206

**Example 2**

**Biochemical assay: Inhibition of FXIa in activity assay**

**using fluorescent peptide as substrate**

The activity of human FXIa (Kordia Life Science NL, catalogue number HFXIa 1111a) is determined by monitoring the cleavage of a fluorescently labelled peptide with the sequence D-Leu-Pro-Arg*Rh110-D-Pro (product number BS-2494; Biosyntan GmbH, Berlin, Germany). In the substrate sequence written above, * indicates the scissile bond, D-Leu: D-leucine, Pro: proline, Arg: arginine, Rh110: rhodamine 110, D-Pro: D-proline). FXIa mediated cleavage of the scissile bond of the peptide substrate leads to an increase of fluorescence intensity of the rhodamine 110 when using excitation and emission wavelengths of 485 nm and 535 nm, respectively. Fluorescence intensity is continuously measured using the microtiter plate reader Safire2 (TECAN, Maennedorf, Switzerland) at room temperature (RT). The assay buffer contains 50 mM HEPES at pH 7.4, 125 mM NaCl, 5 mM $CaCl_2$ and 0.05% (w/v) CHAPS. In the final activity assay, human FXIa and the substrate BS-2494 have assay concentrations of 0.1 nM and 0.5 $\mu$M, respectively. Under these conditions, the increase of fluorescence intensity over time is linear for at least 60 minutes.

For testing the inhibitory activity of antibodies, serial dilutions of antibodies are prepared in PBS buffer (137 mM NaCl, 2.7 mM KCl, 10 mM $Na_2HPO_4$, 1.8 mM $KH_2PO_4$) containing 0.05% (w/v) CHAPS. Two $\mu$L of antibody solution are pre-incubated with 10 $\mu$L FXIa solution (in assay buffer) for 60 minutes at RT. After the pre-incubation step, 10 $\mu$L of substrate BS-2494 (diluted in assay buffer) is added, and the enzymatic reaction is allowed to proceed for 60 minutes, after which the fluorescence intensity is measured. The fluorescence intensity values are converted into percent inhibition by using control reactions (signal of uninhibited reactions is equivalent to 0 % inhibition, and a reaction containing no enzyme is equivalent to 100 % inhibition) and the following formula for transferring values:

$$y = 100\% - [FI(x)-FI(min)]/[FI(max)-FI(min)],$$

where y is the %-inhibition at the antibody concentration x, FI(x) is the fluorescence intensity measured at antibody concentration x, FI(min) is the fluorescence intensity measured in the control reaction in absence of antibody and FI(max) is the fluorescence intensity measured in the uninhibited control reaction. Data are analyzed using the program Origin 7.5SR6 (OriginLab Corporation, USA). IC50 values from averaged data are calculated using the logistics function:

$$y = A2+(A1–A2)/(1+(x/IC50)^p),$$

where y is the %-inhibition at the antibody concentration x, A1 is the lowest inhibition value, and A2 the maximum inhibition value. The exponent, p, is the Hill coefficient.

**Figure 4A** shows a representative compound response curve of antibody NOV1401 inhibiting the enzymatic activity of full length human FXIa. The results show that NOV1401 inhibits the enzymatic activity of human full length FXIa in a concentration dependent manner (Figure 4A). Fitting with the logistic fit model leads to an $IC_{50}$ value of approximately 160 pM.

## Example 3

### Anticoagulant Activity of anti-FXIa Abs

The antithrombotic activity of the antibodies NOV1401 and NOV1090 were tested by using the activated partial thromboplastin time (aPTT) assay and the thrombin generation assay (TGA).

### aPTT Assay:

Lyophilized normal human plasma 'Coagulation Control N' (reference no 5020050) was purchased from Technoclone GmbH (Vienna, Austria). It was pooled from citrated plasma of selected healthy donors. The clotting time obtained with this normal plasma reflects normal concentrations of the coagulation factors involved in clotting. The lyophilized plasma was stored at 4°C. Prior to its use, the plasma was re-suspended in 1 mL of distilled water by carefully rotating the vial and then keeping it for 10 minutes at RT.

The intrinsic pathway triggering reagent 'aPTT-s' (reference no TE0350) was purchased from SYCOmed (Lemgo, Germany) and contains phospholipid and silicate (colloidal) in a buffered solution (sodium chloride, polyethylene glycol 20000; sucrose, sodium azide). The solution was stored at 4 °C.

Calcium Chloride (reference no C1016-500G; Sigma-Aldrich Chemie GmbH, Steinheim, Germany) was prepared in bi-distilled water at a stock concentration of 25 mM.

UltraPure Tris/HCl buffer at pH 7.5 (reference no 15567-027; Life Technologies Corporation, NY, USA) and Phosphate Buffered Saline (PBS, reference no P4417-100TAB; Sigma-Aldrich Chemie GmbH, Steinheim, Germany) were compound dilution.

3-[(3-Cholamidopropyl)dimethylammonio]-1-propanesulfonate hydrate (CHAPS, reference no C3023-25G) and anhydrous Dimethyl sulfoxide (DMSO, reference no 276855-100ML) were purchased from Sigma-Aldrich Chemie GmbH (Steinheim, Germany).

The measurements of the clotting time were performed in an Amelung ball coagulometer model KC4A (purchased through SYCOmed, Lemgo, Germany), which is a semi-automated mechanical clot detection system. The system utilizes a special cuvette (reference no AI4000; SYCOmed) in which a stainless steel ball (reference no AI5000; SYCOmed) was placed.

The sample is added to the cuvette. After an appropriate incubation period, the cuvette is placed into the measuring well of the ball coagulometer. The measuring well rotates slowly causing the cuvette to rotate along its longitudinal axis. Because the cuvette is positioned at a slight angle, gravity and inertia always position the ball at the lowest point of the cuvette. Exactly opposite the ball-position is a magnetic sensor. With the addition of the trigger reagent, a timer is started. As coagulation takes place fibrin strands form in the reaction mixture. The fibrin strands pull the ball away from its inertia position that triggers an impulse in the magnetic sensor. This impulse electronically stops the timer. The pipetting scheme was as follows **(Table 4a):**

**Table '4a**

| Assay step | Solution | aPTT assay Volume [$\mu$L] |
|---|---|---|
| 1 | compound dilution or diluent | 50 |
| 2 | human blood plasma | 50 |
| 3 | aPTT-s reagent | 50 |

(continued)

| Assay step | Solution | aPTT assay Volume [$\mu$L] |
|---|---|---|
| 4 | | Incubate 3 minutes at 37 °C under rotation |
| 5 | 25 mM Calcium Chloride | 50 |
| 6 | | Immediately start the timer |
| 7 | | The timer stops when the clot is formed |

The samples were measured in duplicates at a temperature of 37 °C in the Amelung ball coagulometer.

**Figure 4B** shows a representative compound response curve of antibody NOV1401, leading to the concentration-dependent prolongation of aPTT clotting times. The results suggest that NOV1401 leads to the prolongation of aPTT clotting times of human plasma in a concentration dependent manner. The aPTT clotting time is doubled compared to baseline at a NOV1401 concentration of approximately 14 nM. The $IC_{50}$ value was calculated to be approximately 13 nM.

## Thrombin Generation Assay (TGA):

For the TGA lyophilized normal human plasma (Coagulation control N) was purchased from Technoclone GmbH, (reference number 5020040, Lot# 1P37B00) and reconstituted in distilled water in a the volume suggested by the manufacturer.

The substrate solution was prepared using the fluorogenic substrate Z-Gly-Gly-Arg-AMC from Technoclone GmbH (reference number 5006230, Lot# 8F41B00). Aliquots of the lyophilized substrate were kept at 4° C. The substrate was dissolved freshly in the volume of distilled water indicated on the vial 20 minutes prior its use in the assay. The reconstituted substrate solution contains the fluorogenic peptide at a concentration of 1 mM and $CaCl_2$ at a concentration of 15 mM.

Two different reagents 'TGA RD' (reference no 500622) and 'TGA RC low' (reference no 5006213) for triggering the intrinsic and the extrinsic pathway, respectively, were purchased from Technoclone GmbH (Vienna, Austria). The trigger reagent 'platelet poor plasma (PPP)-reagent low' was purchased from Thrombinoscope (TS31.00, Lot# PPL1409/01) and reconstituted in distilled water as indicated on the vial. 'PPP-reagent low' contains a mixture of phospholipids and tissue factor at very low concentration. The reagent was 8-fold diluted in 80 mM Tris/HCl at pH7.4, 0.05% (w/v) CHAPS immediately before use.

The samples were aliquoted and measured in 96 well black/clear bottom plates purchased from Costar (product no 3603). For automation transfer samples were placed in V-bottom 96 well plate (Costar, 3894) and transferred using a CyBio automation system (Analytik Jena US, Woburn, MA, USA).

The reconstituted human blood plasma, trigger reagent 'PPP-reagent low' and substrate were pre-warmed for 10 minutes in a water bath at 37 °C. Serial 1:3 antibody dilutions in PBS were prepared in a 96 well plate starting with a NOV1401 concentration of 5 $\mu$M (5x the highest final concentration of 1 $\mu$M) for a total of 8 dilutions. 222 $\mu$l of trigger reagent was mixed with 1108 $\mu$l of substrate solution to generate the 10+50 trigger reagent substrate mix. 80 $\mu$l per well was added into a V-bottom 96 well plate for later transfer using an automation system. The plate was kept at 37 °C. The reagents were added according to the scheme given in Table 4b.

**Table 4b**

| Assay step | Solution | Volume [ul] |
|---|---|---|
| 1 | Antibody solutions (8 dilutions) | 20 |
| 2 | Plasma stock solution | 20 |
| | 5 minutes incubation at 37°C in a thermomixer at 300 rpm. | |
| 3 | Trigger reagent/substrate mixture | 10 + 50 |

Trigger/substrate mixtures were transferred using automation. After adding the mixtures, excitation and emission at 360 nm at 460 nm, respectively, were recorded immediately using a Synergy Neo instrument (BioTek Instrument Inc., Winooski, VT, USA). The samples were measured in duplicates at a temperature of 37 °C in the plate reader for 90 minutes at intervals of 55 seconds.

To generate peak thrombin concentration values data were processed using the TGA evaluation software file provided by Technoclone. To generate plots for peak thrombin concentration vs antibody concentration data were fit using GraphPad software. These data were fit to a non-linear regression model in the GraphPad Prism5 software (GraphPad Software Inc., La Jolla, CA, USA). The $IC_{50}$ value was determined using the built-in four-parameter dose-response curve equation (variable slope): y = Bottom + (Top - Bottom) / (1 + 10^((LogIC50- x)*HillSlope)) where y is the maximal concentration of thrombin formed at the inhibitor concentration, x, and top and bottom represent the concentration of thrombin without inhibitor and at the highest concentration of inhibitor, respectively.

**Figure 4C** shows a representative compound response curve of antibody NOV1401, displaying the concentration-dependent inhibition of thrombin generation in the TGA. An $IC_{50}$ value of 24 nM and a residual thrombin concentration of 159 nM (dotted line) were calculated for this compound response curve.

. **Example 5**

**Effect of FXI antibody on FeCl$_3$-induced thrombosis in mice**

Mice deficient in FXI (FXI[-/-] mice) on a C57Bl background were bred at Novartis (E. Hanover, NJ) and used to assess the anti-thrombotic efficacy of NOV1401. When reconstituted intravenously with human FXI (hFXI), these mice acquire a wild-type thrombophilic phenotype when exposed to a thrombogenic stimulus. In the studies herein, thrombosis was induced in the carotid artery by applying ferric chloride (FeCl$_3$) to the surface of the artery.

NOV1401 was injected as a bolus through the jugular vein of anesthetized mice 15 minutes prior to the induction of thrombosis. Doses of antibody ranged from 0.24 mg/kg - 0.47 mg/kg. The FXI[-/-] mice were reconstituted with human FXI by injecting 0.47 mg/kg human FXI via the jugular vein 10 minutes prior to the FeCl$_3$ challenge. Two 1 mm x 1.5 mm pieces of filter paper saturated with 3.5% FeCl$_3$ were then applied to opposite sides of the carotid artery, in contact with its adventitial surface, and removed 3 minutes afterward, followed by thorough washing with saline. Blood flow through the carotid artery was measured with a Transonic flow probe. Baseline blood flow was obtained for 5 minutes prior to FeCl$_3$ application and then for 30 minutes after application of FeCl$_3$ (*i.e.,* during the thrombogenic period). At the end of the experiment blood was sampled from the vena cava into syringes containing 3.8% sodium citrate, plasma was prepared and subjected to an aPTT assay.

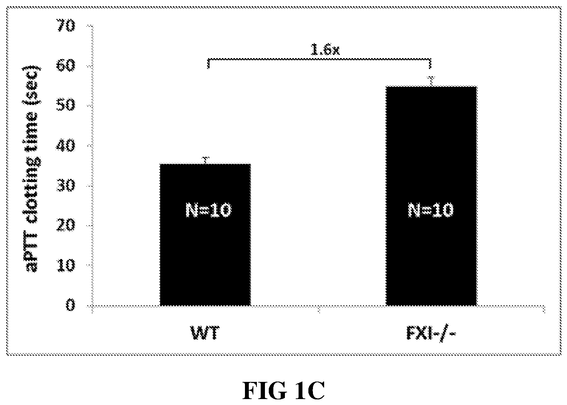

**Figure 1A** shows the effect of NOV1401 on FeCl$_3$-induced thrombosis in FXI[-/-]mice reconstituted with human FXI (humanized FXI mouse model). **Figure 1B** shows the effect of NOV1401 on aPTT in the same mouse model. **Figure 1C** shows aPTT prolongation in FXI[-/-]mice in comparison to wild-type mice.

NOV1401 fully inhibited FeCl$_3$-induced thrombus formation in hFXI-reconstituted FXI[-/-]mice **(Figure 1A)** starting at 0.24 mg/kg. A steep dose response was observed, likely reflecting a stoichiometric all-or-none antithrombotic response. The aPTT was prolonged to 1.6 fold above vehicle controls in the high dose group **(Figure 1 B)**, corresponding to the same level of prolongation by genetic depletion of FXI **(Figure 1 C),** *i.e.,* maximal effect. These results show that NOV1401 has anti-thrombotic activity in mouse FeCl$_3$ thrombosis model.

**Example 6**

**Effects of FXI antibody on free FXI and aPTT in cynomolgus monkeys**

To evaluate the pharmacokinetic (PK) profile and pharmacological effects of an anti-FXI/FXIa antibody, such as NOV1401, the antibody was administered via subcutaneous (s.c.) or intravenous (i.v.) injections to cynomolgus monkeys in a rising dose study.

The anticoagulant effect of NOV1401 was characterized in cynomolgus monkeys by testing the antibody's ability to prolong aPTT and reduce free FXI (FXI$_f$) levels after a single intravenous (N=2) or subcutaneous (N=2) dose of 3 mg/kg. A second dose of 10 mg/kg was administered to all animals followed by a third dose of 30 mg/kg to determine if the effects observed at 3 mg/kg can be potentiated by higher doses. These results show that NOV1401 has sustained anticoagulant activity in cynomolgus monkeys. The pharmacodynamics (PD) of NOV1401, characterized by its anticoagulant effect as determined by aPTT and FXI$_f$ levels, were then compared to the PK profile. The comparison indicates that there is a good PK/PD correlation.

Animals were dosed either i.v. (N=2) or s.c. (N=2) with NOV1401 on study day 1 at 3 mg/kg, day 85 at 10 mg/kg and day 114 at 30 mg/kg. Blood samples were collected into sodium citrate coagulation tubes at 15 min and 2 hours post-dose for i.v. dosed animals, and for all animals at pretest, 6, 24, 48, 72 and 96 hours post-dose (days 1, 85 and 114) and at 8, 11, 15, 18, 22, 25, 29, 32, 36, 39, 43, 46, 50, 53, 57, 60, 64, 66, 71, 75 and 78 days post-dose (day 1 only Blood was also collected on days 92, 95, 99, 102, 107, 110, 121, 124, 128 and prior to dosing on day 114. All blood samples were centrifuged; plasma samples were obtained and frozen at approximately -70 °C or below.

Total NOV1401 plasma concentrations were measured by standard methods for human IgG detection by ELISA using a sandwich immunoassay with a mouse anti-human-IgG monoclonal antibody as capture antibody and a goat anti-human-IgG with an HRP label as detection antibody.

For free FXI measurements in plasma samples that contain both FXI and NOV1401, unbound FXI was captured with immobilized NOV1401 and FXI already complexed with NOV1401 was washed away. Plate-bound FXI was then detected with a mouse Fc containing antibody 14E11, a monoclonal antibody that binds to the A2 domain of FXI and has been described in the literature (Cheng, et al. Blood, 116:3981-3989, 2010). The very high affinity of NOV1401 to both FXI and FXIa and the different binding sites for NOV1401 and the detection antibody 14E11 allowed an accurate determination of free FXI.

ELISA plates (384-Well LUMITRAC™ 600 HB) were coated with NOV1401 (5 μg/mL in PBS) for binding of free FXI. After blocking (milk blocker: KPL #50-82-01, 1:20 dilution) and washing the plates with wash buffer (PBS; 0.05% Tween 20), plasma samples diluted 1:40 in assay buffer (50 mM HEPES at pH 7.4, 125 mM NaCl, 5 mM $CaCl_2$, 5 mM EDTA and 0.05% (w/v) CHAPS) were incubated at RT for 30 min. and washed 3X with wash buffer. The detection antibody 14E11 was added at 1 μg/mL in dilution buffer (1.7 mM sodium phosphate monobasic, 8.1 mM sodium phosphate dibasic heptahydrate, 0.15 M sodium chloride, 0.7% Triton X-100, and 0.1% sodium azide, pH 7) containing 0.7% casein. After washing the plates with wash buffer, a secondary detection antibody, peroxidase-labeled anti-mouse IgG (Sigma #A5278), was added at 0.5 μg/mL in dilution buffer containing 0.4% casein. After washing the plates in wash buffer, 50 μL peroxidase chemiluminescent substrate solution (LumiGLO, KPL #54-61-01) was added and the luminescence signal was read immediately on multi-mode microplate reader (SPECTRAMAX M5E). The free FXI concentration in each sample was determined using a standard curve generated with human FXI (zymogen) from Enzyme Research Laboratories (Catalog #HFXI 1111) starting from 100 nM FXI with a dilution factor of 2 and 22 dilution steps. The lower limit of quantification (LLOQ) was 0.24 nM FXI taking into account the 1:40 dilution before measurement.

Plasma samples from all time points were subjected to aPTT analysis and aPTT results were compared to total plasma NOV1401 concentration and free FXI levels. **Figures 2A and 2B** show changes of aPTT clotting times in relationship to total plasma NOV1401 levels for i.v.- and s.c.-dosed animals. **Figures 3A and 3B** show changes of aPTT clotting times in relationship to free FXI levels for i.v.- and s.c.-dosed animals.

For i.v.-administered NOV1401, the highest plasma total NOV1401 levels were observed at 15 min. post-dose **(Figure 2A).** At this time the aPTT was approximately doubled versus baseline in both animals and remained at this level for an average of 5-6 weeks. The mean aPTT prolongations from 15 min. post-dose over the measurements preceding the decline toward baseline were 2.0 ± 0.02 times and 1.9 ± 0.03 times for each animal.

By day 85, prior to administration of a second dose, aPTT had reached baseline levels and NOV1401 plasma concentrations had fallen below 10 nM. A second dose of 10 mg/kg was administered on day 85 increasing the plasma concentration of total NOV1401 by about at least 3-fold and resulting in aPTT prolongation similar to what was observed after the first dose. A third dose of 30 mg/kg was administered on day 114 while aPTT was still prolonged, and did not result in any significant additional aPTT prolongation, despite another at least 3-fold increase in total NOV1401 plasma concentration **(Figure 2A).** Therefore, NOV1401 doses higher than 3 mg/kg achieved comparable aPTT prolongation as the 3 mg/kg dose, and did not seem to increase the magnitude of aPTT prolongation. As expected, s.c. administration of NOV1401 resulted in a slower rise in aPTT than with i.v. administration, but the extent of prolongation was comparable to that in the i.v. group **(Figure 2B).** The aPTT was prolonged versus baseline for an average of 5-6 weeks in the two animals. Mean aPTT fold prolongations were similar to those of i.v.-treated animals: 2.0 ± 0.03 and 1.8 ± 0.02 from 6 hrs. post-dose through the measurements preceding the decline toward baseline. As in the i.v, administering higher doses did not lead to higher aPTT responses despite higher NOV1401 plasma exposures.

The results in Figures 2A-2B demonstrate that NOV1401 prolongs aPTT in cynomolgus monkeys.

The mean baseline plasma $FXI_f$ concentration was 10.9 ± 0.3 nM in the i.v. group and fell rapidly (by 15 min.) upon injection of NOV1401 **(Figure 3A).** Plasma $FXI_f$ levels remained low until total NOV1401 plasma levels dropped to between 15 nM-25 nM **(Figure 2A, Figure 3A).** In the s.c. group, the mean baseline $FXI_f$ concentration was 14.3 ± 1.0 nM. $FXI_f$ was sharply lower vs baseline by 6 hrs. post-treatment **(Figure 3B),** and remained low until plasma NOV1401 levels declined to between 15 nM-25 nM **(Figure 2B, Figure 3B).** $FXI_f$ dropped again sharply after the second dose at 10 mg/kg in all animals and remained low until the end of the study. The two higher doses did not further reduce $FXI_f$ relative to baseline.

In all treated animals, the drop and recovery of $FXI_f$ levels were temporally and inversely related to NOV1401-induced prolongation of aPTT, confirming that NOV1401 inhibits the function of the intrinsic coagulation pathway (prolongs aPTT) by lowering $FXI_f$.

These results (e.g., Figures 3A and 3B) demonstrate that NOV1401 lowers plasma $FXI_f$ levels in cynomolgus monkeys. In the cynomolgus monkey studies, no evidence of excessive bleeding was observed at the venipuncture sites or by gross observations at necropsy. Moreover, occult blood was not detected in stools throughout the study.

A sustained anticoagulant effect of NOV1401 was also observed in a 13-week s.c./4-week i.v. repeat dose toxicity study in cynomolgus monkeys. In this study, NOV1401 was administered weekly at doses of 10 mg/kg (N=3, male and female combined) and 100 mg/kg (N=5, male and female combined) s.c. for 13 weeks (14 doses) or at 50 mg/kg (N=3, male and female combined) i.v. for 4 weeks (5 doses). The control group (N=5, male and female combined) received vehicle for 13 and 4 weeks s.c. and i.v., respectively. FXI:C was assessed by measuring clotting time of cynomolgus monkey plasma samples in the presence of human FXI deficient plasma (one-stage aPTT). aPTT and FXI:C were measured on study days 2, 23, and 79 for the s.c. groups and on days 2 and 23 for the i.v. group. Across all animals and all treatment groups, an aPTT prolongation of 2.1- to 3-fold was observed **(Figure 5A).** The effect was sustained throughout the dosing phase of the study and no dose-dependency was observed similar to the observation in the previous rising dose study. FXI:C was reduced across animals and treatment groups by 88-95% and remained at these levels throughout the dosing phase of the study **(Figure 5B).** The effect on FXI:C was also dose-independent over these doses.

No evidence of macroscopic or microscopic indications of bleeding, including excessive bleeding, was observed at the venipuncture sites (including s.c. and i.v. injection and blood sampling sites) or by gross observations at necropsy. Moreover, occult blood was not detected in stools at the end of the study. In addition, no mortality occurred and there were no test article-related effects on clinical signs, body weight, food consumption, ophthalmologic and electrocardiographic parameters, hematology, clinical chemistry, or urinalysis. No target organs of toxicity were identified.

Increased thyroid weights were observed in males at 100 mg/kg s.c. However, the toxicological significance of this finding is inconclusive, since there were no histologic correlates. There was large variability of thyroid weights amongst the animals, and the finding was present only in one sex. Microscopically, dose-dependent fibrosis at s.c. injection sites in both sexes was observed at 10 and 100 mg/kg/week s.c. These findings were not considered adverse.

No significant toxicity findings were observed in single rising dose or repeat dose general toxicity studies in cynomolgus monkeys up to 13 weeks. Therefore, the highest s.c. dose level administered in the 13-week GLP toxicity study (100 mg/kg/week) was defined as the NOAEL.

## Example 7

### Pharmacokinetics in cynomolgus monkey - single dose

Cynomolgus monkeys (female, N=2) were administered a single 3 mg/kg dose of NOV1401 either i.v. or s.c. and observed until plasma $FXI_f$ concentrations and aPTT returned to pre-dose values. The animals were then administered a single 10 mg/kg dose of NOV1401 either i.v. or s.c. followed 2 weeks later by a 30 mg/kg dose either i.v. or s.c. and another 2-week observation period. The PK of NOV1401 was assessed by measuring total NOV1401. The exposure to total NOV1401, as measured by either the maximum observed total NOV1401 concentration ($C_{max}$) or the area under the total NOV1401 concentration-time curve ($AUC_{0-14d}$), was comparable between the individual animals in each group. Exposure ($C_{max}$ or $AUC_{0-14d}$) was approximately dose-proportional for each dosing route (Table 9). $C_{max}$ was approximately 3-fold higher in the i.v. group than in the s.c. group. However, plasma total NOV1401 concentrations were similar in both groups following the initial distribution phase. The terminal elimination half-life ($t_{1/2}$) was estimated for each animal using a two-compartment model following administration of the 3 mg/kg dose. The $t_{1/2}$ ranged from 14-15 days (N=2). The absolute bioavailability following s.c. injection ranged from 61-66% (3 dose levels). Anti-NOV1401 antibodies were not detected after either i.v. or s.c. administration in any animals.

**Table 9:** Mean pharmacokinetic parameters following single (rising) dose administration in female cynomolgus monkeys

| Dose (mg/kg) | Route | $t_{max}$ (hr)* | $C_{max}$ (μg/mL) | $AUC_{0-14d}$ (μg·d/mL) |
|---|---|---|---|---|
| 3 | i.v. | 0.25 | 96.0 | 544 |
| 3 | s.c. | 168 | 36.0 | 360 |
| 10 | i.v. | 0.25 | 325 | 1,810 |
| 10 | s.c. | 132 | 101 | 1,160 |
| 30 | i.v. | 1.08 | 1,170 | 6,770 |
| 30 | s.c. | 132 | 344 | 4,140 |
| * $t_{max}$ is reported as median value. | | | | |

## Example 8

### Toxicokinetics in cynomolgus monkey - repeat dose

Cynomolgus monkeys were administered weekly doses of 10 or 100 mg/kg NOV1401 s.c. for 13 weeks (14 doses) or doses of 50 mg/kg NOV1401 i.v. for 4 weeks (5 doses). Animals treated with NOV1401 were exposed to NOV1401 during the dosing phase of the study; no exposure was noted in control animals. No gender-related differences in exposure to plasma total NOV1401 were observed. The increase in exposure (both $C_{max}$ and $AUC_{0-7d}$) was dose-proportional in both male and female animals (Table 10). Anti-NOV1401 antibodies were detected in 5 of 6 animals at 10 mg/kg/week s.c., in 1 of 10 animals at 100 mg/kg/week s.c., and in 1 of 6 animals at 50 mg/kg/week i.v. Exposure to total NOV1401 was not compromised in any of the s.c. dose groups. Only one anti-drug antibody (ADA)-positive animal had an

$AUC_{0-7d}$ on Study Day 22 that was lower than the other animals in the same group (50 mg/kg/week i.v.). There was no impact on aPTT prolongation in this animal and no toxicity was observed.

**Table 10:** Mean toxicokinetic parameters for the penultimate dose (Study Day 85 for the s.c. arms, Study Day 22 for the i.v. arm) of 13-week/4-week GLP-compliant toxicity study in cynomolgus monkeys (male + female combined)

| Dose (mg/kg/week) | Route | $t_{max}$ (hr)* | $C_{max}$ (µg/mL) | $AUC_{0-14d}$ (µg·d/mL) |
|---|---|---|---|---|
| 10 | s.c. | 24-120 | 719 | 3,100 |
| 100 | s.c. | 72-120 | 5,630 | 23,400 |
| 50 | i.v. | 0.25-96 | 1,990 | 10,700 |
| * $t_{max}$ is reported as the range of values observed. | | | | |

**Example 9**

**Dose escalation study 1 in humans**

Human studies are carried out to assess the safety and tolerability of anti-FXI/FXIa antibodies, such as NOV1401, following single dose administration in healthy subjects. A total of approximately 60 healthy male and post-menopausal/surgically sterile female subjects, between 18 and 55 years of age, are entered into this study. Good health is determined by past medical history, physical examination, neurological examination, vital signs, electrocardiogram (ECG), and laboratory tests at screening. Selected subjects weigh at least 50 kg, and have a body mass index (BMI) within the range of 18-35 kg/m$^2$. BMI = body weight (kg) / [height (m)]$^2$.

Six s.c. dose levels of 5, 15, 50, 150, 300 and 600 mg are to be tested in a human study, provided that the predicted mean duration of aPTT prolongation ≥ 2-fold does not persist for ≥ 42 days at any tested dose. Two interim analyses (IA) are conducted to confirm dose selection for the 2 highest dose levels. If the model-predicted mean duration of aPTT prolongation is ≥ 2-fold for longer than 42 days at the 300 mg or the 600 mg dose, the dose can be lowered based, for example, based on model simulations, to ensure that the mean duration of aPTT prolongation does not exceed 2-fold for ≥ 42 days. Non-limiting exemplary dose adjustments may involve lowering a dose using decrements of 10 mg, 20 mg, 30 mg, 40 mg, or 50 mg.

The first three dose escalation steps occur at ≈1/2 log increments. The last 2 dose escalation steps are ≤ 2-fold increments to mitigate the risk of prolonged target saturation and extended aPTT prolongation.

The maximum duration of 2-fold aPTT prolongation for a certain number of days (e.g., 30 days, 35 days, 40 days, 42 days, etc.) with a therapy targeting FXI can be assessed based on genetic data showing mild bleeding phenotype in patients with severe FXI deficiency, data from patients with FXI deficiency with acquired inhibitor, and also data from human studies, for instance, FXI-ASO (see, *e.g.,* Liu et al., (2011) "ISIS-FXIRx, a novel and specific antisense inhibitor of factor XI, caused significant reduction in FXI antigen and activity and increased aPTT without causing bleeding in healthy volunteers." Presented at the 53rd American Society of Hematology annual meeting and exposition, San Diego, California. Blood; 118: Abstract 209), where multiple dose administration of FXI-ASO over 6 weeks resulted in a robust and sustained FXI depletion over > 6 weeks (42 days) with no bleeding events. In certain embodiments, a model-based analysis predicts that maximum aPTT prolongation of ≈ 2.7-fold (relative to pre-dose) can be achieved transiently at a 50 mg s.c. dose of NOV1401 (60-kg subject). In certain embodiments, higher doses are predicted to extend the duration of this maximum aPTT prolongation of 2.7 fold.

Subjects are monitored throughout the study for safety parameters and/or end points, such as, physical exam, neurological exam, vital signs, electrocardiogram (ECG), safety laboratories, and adverse events (AEs) including serious AEs (SAEs) up until and including Day 106 post-dose.

The effect of anti-FXI/FXIa antibody (e.g., NOV1401) on aPTT is assessed based on relative changes from baseline. Plasma concentrations of total anti-FXI/FXIa antibody (e.g., NOV1401) are measured to assess the PK of single doses in these subjects.

To assess immunogenicity (IG) of anti-FXI/FXIa antibodies (e.g., NOV1401), screening and confirmation for ADA are conducted.

Free and total FXI and FXI coagulation activity (FXI:C) are measured to assess the effects of anti-FXI/FXIa antibodies (*e.g.,* NOV1401) on target engagement and target-related PD parameters.

D-dimer, prothrombin fragments 1.2 (F1.2) and prothrombin-antithrombin complex (TAT) are assessed to determine the effects of anti-FXI/FXIa antibodies (e.g., NOV1401) on thrombogenesis parameters.

To study the effects of anti-FXI/FXIa antibodies (e.g., NOV1401) on other coagulation parameters, the following can be assessed: prothrombin time (PT), thrombin time (TT), and exploratory coagulation laboratory parameters such as thrombin activatable fibrinolysis inhibitor, fibrinogen, tissue plasminogen activator (tPA) and TGA in the subjects.

Biomarkers studied may include, but are not be limited to: D-Dimer, FXI activity, PT/INR, TT, F1.2, fibrinogen, TGA, TAFI activity, TAT, PAI-1 antigen, TFPi activity, tPA activity, and vWF activity.

**Dose escalation study 2 in humans**

NOV1401 was evaluated in a first-in-human (FIH) study to characterize its safety/tolerability, pharmacokinetics (PK) and pharmacodynamics (PD) in healthy subjects following single s.c. administration. A total of 60 subjects were enrolled in 6 consecutive cohorts of 10 subjects each (8 NOV1401: 2 Placebo), and received doses from 5 mg to 240 mg (e.g., 5 mg, 15 mg, 50 mg, 150 mg, 240 mg) subcutaneously of NOV1401 or matching placebo.

In this study, NOV1401 was safe and well tolerated, and the incidences of adverse events (AEs) were comparable across dose groups and to placebo. No bleeding events, hypersensitivity reactions or injection site reactions were reported. Exposure increased with increasing dose of s.c. NOV1401; the median maximum observed concentration ($C_{max}$) occurred on Day 7 to 21 and the mean terminal elimination half-life ($t_{1/2}$) ranged from 20 to 28 days. A dose and time-dependent prolongation of aPTT occurred with NOV1401 after single s.c. administration; the 150 mg dose resulted in a mean aPTT prolongation $\geq$ 2-fold at Day 29 and the 240 mg dose extended the duration of aPTT prolongation. Robust and sustained reductions of free FXI $\geq$ 90% were observed in all patients with 150 mg NOV1401 through Day 29 and through Day 43 with 240 mg. The NOV1401 240 mg dose resulted in a FXI inhibition of $\geq$ 80% for $\geq$ 43 days in the subjects with stage 2/3 obesity and $\geq$ 57 days in other subjects.

Robust reductions in free FXI and in FXI coagulation activity and relevant aPTT prolongation are predicted to occur within 12 to 24 hours after NOV1401 s.c. administration with relevant clinical doses.

The results of this study demonstrate the anticoagulant activity NOV1401 in humans and support using NOV1401 in clinical settings where anticoagulation is therapeutically beneficial in the context of inhibiting FXIa activity.

**Example 10**

**Study 1 in humans with AF**

Human studies are carried out to assess the PK, PD, safety and tolerability of NOV1401 in patients with atrial fibrillation (AF). Inclusion criteria include AF patients with a demonstrated high risk of bleeding characterized as having a $CHA_2DS_2VASc$ risk score $\geq$ 2 and one, two or more of the following risk factors:

    (a) 65 years old or older;
    (b) history of previous stroke or transient ischemic attack;
    (c) previous major bleed or clinically relevant bleed;
    (d) chronic kidney disease (CKD) stage 3 to 4;
    (e) treatment with single or dual antiplatelet therapy; and
    (f) history of falls associated with hematoma bruise or other manifestations.

NOV1401 s.c. dose levels of 90 mg, 120 mg, 150 mg, 180 mg, 210 mg, and 240 mg, once a month, are to be tested over a 3-month period.

Subjects are monitored throughout the study for safety parameters and/or end points, such as, physical exam, neurological exam, vital signs, electrocardiogram (ECG), safety laboratories, and adverse events (AEs) including serious AEs (SAEs). The primary objective of this study is to evaluate the proportion of patients achieving FXI inhibition $\geq$ 80% at trough after monthly dosing of NOV1401.

The effect of NOV1401 on aPTT is assessed, and plasma concentrations of total NOV1401 are measured to assess the PK.

Free and total FXI and FXI coagulation activity (FXI:C) are measured to assess the effects of NOV1401 on target engagement and target-related PD parameters.

D-dimer, prothrombin fragments 1.2 (F1.2) and prothrombin-antithrombin complex (TAT) are assessed to determine the effects of NOV1401 on thrombogenesis parameters.

To study the effects of NOV1401 on other coagulation parameters, the following can be assessed: prothrombin time (PT), thrombin time (TT), and exploratory coagulation laboratory parameters such as thrombin activatable fibrinolysis inhibitor, fibrinogen, tissue plasminogen activator (tPA) and TGA in the subjects.

Biomarkers studied may include, but are not be limited to: D-Dimer, FXI activity, PT/INR, TT, F1.2, fibrinogen, TGA, TAFI activity, TAT, PAI-1 antigen, TFPi activity, tPA activity, and vWF activity.

**Study 2 in humans with AF**

Study purpose/objectives: The purpose of this study is to evaluate the pharmacokinetics (PK), pharmacodynamics (PD), safety and tolerability of NOV1401 in patients with atrial fibrillation at low to medium risk of stroke. The trial will evaluate the effects of three dosing regimens of NOV1401 on Factor XI inhibition, indices of coagulation, thrombogenesis biomarkers, bleeding, and major cardiovascular (CV) and cerebrovascular events. PK/PD relationship, hypersensitivity reactions, injection site reactions and immunogenicity will also be assessed. A comparative study with a NOAC can be conducted in parallel.

Primary Objective(s): The primary objective of this study is to evaluate the proportion of patients achieving FXI inhibition $\geq$ 80% at trough (Day 91) after monthly dosing at 3 dose levels of NOV1401.

Secondary Objectives:

1: To evaluate the proportion of patients achieving FXI inhibition $\geq$ 80% at trough after the first and second dose (Day 31 and Day 61) at 3 dose levels of NOV1401;
2: To evaluate the incidence of major bleeding events, clinically relevant non-major bleeding events and total bleeding with NOV1401 relative to an NOAC during the treatment period;
3: To assess the effect of NOV1401 on D-dimer and other thrombogenesis biomarkers as indicators of efficacy at Day 31, Day 61 and Day 91 compared to a NOAC; and
4: To evaluate the safety and tolerability of NOV1401 compared to a NOAC.

Study design: This is a randomized, open-label, blinded endpoint evaluation, active-controlled, dose-range finding study. After a screening period of 1 to 2 weeks, patients will be randomized to 1 of 4 treatment groups below (low, medium or high dose NOV1401 or a NOAC) in a 1:1:1:1 ratio and followed during a 90-day treatment period:

1. NOV1401 120 mg subcutaneous administration once a month;
2. NOV1401 150 mg subcutaneous administration once a month;
3. NOV1401 180 mg subcutaneous administration once a month; and
4. a NOAC.

Randomization will be stratified by country and whether patients are anticoagulant naïve (Yes/No) at screening. Patients will be transitioned to a NOAC and/or other standard of care therapy at a doctor's discretion on Day 91 and followed up to Day 170.

Population: 600 male and female patients age $\geq$ 55 to < 85 with atrial fibrillation will be randomized in the study.

Key Inclusion criteria:

- Male and female patients $\geq$ 55 and < 85 years old;
- Body weight between 50 and 130 kg inclusive;
- Atrial fibrillation or atrial flutter, as documented by electrocardiography;
- $CHA_2DS_2$-VASc risk score $\geq$ 2 for male and female patients, male patients with $CHA_2DS_2VASc$ risk score of 1 can be included if anticoagulation therapy is warranted; and
- Either anticoagulant-naïve or receiving a stable treatment of a recommended dose of a new oral anticoagulant (NOAC) over the 8 weeks prior to screening.

Efficacy assessments:

- Inhibition of free factor XI at Days 31, 61 and 91; and
- Change from baseline to Days 31, 61 and 91 in D-dimer, prothrombin fragment 1.2, thrombin-antithrombin III-complexes and fibrinogen.

Key safety assessments:

- Confirmed major bleeding events, clinically relevant non-major bleeding events and total bleeding events;
- Adverse event monitoring;
- Occurrence of major cardiovascular and cerebral events (stroke, transient ischemic attack, systemic embolism, myocardial infarction, venous thromboembolism and cardiovascular death;
- Physical examinations;
- Monitoring of laboratory parameters in blood;
- ECGs;

- Hypersensitivity reactions;
- Injection site reactions; and
- Development of anti-drug antibodies;

Other assessments: Factor XI coagulation activity, activated partial thromboplastin time (aPTT), and pharmacokinetics.

Interim analyses: Four interim analyses are planned. The interim analyses will be implemented when approximately 80, 160 and 300 patients have completed at least 1 month of treatment, and when all patients have completed the treatment period and data collected at the Day 91 visit are available. Available PD parameters (e.g., relevant coagulation parameters), safety and tolerability data will be assessed.

## Incorporation By Reference

All references cited herein, including patents, patent applications, papers, publications, text books, and the like, and the references cited therein, to the extent that they are not already, are hereby incorporated herein by reference in their entirety.

## Equivalents

The foregoing written specification is considered to be sufficient to enable one skilled in the art to practice the invention. The foregoing description and examples detail certain preferred embodiments of the invention and describe the best mode contemplated by the inventors. It will be appreciated, however, that no matter how detailed the foregoing may appear in text, the invention may be practiced in many ways and the invention should be construed in accordance with the appended claims and any equivalents thereof.

The following numbered clauses, describing aspects of the invention, are part of the description.

1. A method of preventing, treating or managing or reducing the risk of stroke or thromboembolism comprising administering to a subject afflicated with end stage renal disease, an effective amount of a pharmaceutical composition comprising an antibody or antigen-binding fragment that binds within the catalytic domain of Factor XI ("FXI") and/or activated FXI ("FXIa").

2. The method of clause 1, wherein the subject is receiving dialysis.

3. The method of clause 1 or 2, wherein the subject is receiving hemodialysis or other types of dialysis, such as, peritoneal dialysis.

4. The method of clause 1, 2, or 3, wherein the subject is afflicted with atrial fibrillation.

5. The method of clause 4, wherein the subject is afflicted with non-valvular atrial fibrillation.

6. A method of preventing or reducing the risk of stroke or thromboembolism comprising administering to a subject afflicated with atrial fibrillation and end stage renal disease and who is undergoing dialysis, such as hemodialysis, an effective amount of a pharmaceutical composition comprising an antibody or antigen-binding fragment that binds within the catalytic domain of FXI and/or FXIa.

7. The method of clause 6, wherein the subject is afflicted with non-valvular atrial fibrillation.

8. A method of preventing, treating or managing or reducing the risk of catheter-related thrombosis comprising administering to a subject afflicated with end stage renal disease initiated on hemodialysis with inserted tunnelled catheter, an effective amount of a pharmaceutical composition comprising an antibody or antigen-binding fragment that binds within the catalytic domain of FXI and/or FXIa.

9. The method of any one of the preceding clauses, wherein the subject has a demonstrated high risk of bleeding.

10. A method of preventing or reducing the risk of stroke or thromboembolism, such as systemic embolism, comprising administering to a subject afflicated with atrial fibrillation, an effective amount of a pharmaceutical composition comprising an antibody or antigen-binding fragment that binds within the catalytic domain of FXI and/or FXIa, wherein the subject has a demonstrated high risk of bleeding which is characterized by a $CHA_2DS_2VASc$ risk score $\geq 2$.

11. The method of any one of clauses 1-10 wherein the subject is at least 55 years old.

12. The method of any one of clauses 9-11, wherein the subject has a demonstrated high risk of bleeding characterized as having a $CHA_2DS_2VASc$ risk score $\geq 2$ and one, two or more of the following risk factors:

(a) over 65 years old;

(b) history of previous stroke or transient ischemic attack;

(c) previous major bleed or clinically relevant bleed;

(d) chronic kidney disease (CKD) stage 3 to 4;

(e) treatment with single or dual antiplatelet therapy; and

(f) history of falls associated with hematoma bruise or other manifestations.

13. The method of any one of clauses 1-12, wherein the antibody or fragment comprises a VH selected from the group consisting of SEQ ID NO: 9 and 29 or an amino acid sequence with at least 90% identity thereof; and a VL selected from the group consisting of SEQ ID NO: 19 and 39 or an amino acid sequence with at least 90% identity thereof.

14. The method of clause 13, wherein the antibody or fragment comprises a VH selected from the group consisting of SEQ ID NO: 9 and 29 or an amino acid sequence with at least 95% identity thereof; and a VL selected from the group consisting of SEQ ID NO: 19 and 39 or an amino acid sequence with at least 95% identity thereof.

15. The method of clause 13, wherein the antibody or fragment comprises a VH selected from the group consisting of SEQ ID NO: 9 and 29 or an amino acid sequence with at least 97% identity thereof; and a VL selected from the group consisting of SEQ ID NO: 19 and 39 or an amino acid sequence with at least 97% identity thereof.

16. The method of clause 13, wherein the antibody or fragment comprises a variable heavy chain selected from the group consisting of SEQ ID NO: 9 and 29; and variable light chain sequence selected from the group consisting of SEQ ID NO: 19 and 39.

17. The method of any one of the preceding clauses, wherein the antibody or fragment comprises a heavy chain comprising the amino acid sequence of SEQ ID NO: 31 and a light chain comprising the amino acid sequence of SEQ ID NO: 41.

18. The method of clause 13, wherein the antibody or fragment is: (i) an antibody or fragment comprising a variable heavy chain of SEQ ID NO: 9 and a variable light chain sequence of SEQ ID NO: 19; (ii) an antibody or fragment comprising a variable heavy chain of SEQ ID NO: 29 and a variable light chain sequence of SEQ ID NO: 39; (iii) an antibody comprising a heavy chain comprising the amino acid sequence of SEQ ID NO: 31 and a light chain comprising the amino acid sequence of SEQ ID NO: 41; or (iv) an antibody comprising a heavy chain comprising the amino acid sequence of SEQ ID NO: 11 and a light chain comprising the amino acid sequence of SEQ ID NO: 21.

19. The method of any one of clauses 1-18, wherein the antibody or fragment comprises (i) a heavy chain variable region CDR1 comprising the amino acid sequence of SEQ ID NO: 46; CDR2 comprising the amino acid sequence of SEQ ID NO: 4; and CDR3 comprising the amino acid sequence of SEQ ID NO: 5; and (ii) a light chain variable region CDR1 comprising the amino acid sequence of SEQ ID NO: 33; CDR2 comprising the amino acid sequence of SEQ ID NO: 14; and CDR3 comprising the amino acid sequence of SEQ ID NO: 15.

20. The method of any one of clauses 1-18, wherein the antibody or fragment comprises (i) a heavy chain variable region CDR1 selected from the group consisting of SEQ ID NO: 3 and 23; CDR2 selected from the group consisting of SEQ ID NO: 4 and 24; and CDR3 selected from the group consisting of 5 and 25; and (ii) a light chain variable region CDR1 selected from the group consisting of SEQ ID NO: 13 and 33; CDR2 selected from the group consisting of SEQ ID NO: 14 and 34; and CDR3 selected from the group consisting of SEQ ID NO: 15 and 35.

21. The method of any one of clauses 1-18, wherein the antibody or fragment comprises a heavy chain variable region CDR1 selected from the group consisting of SEQ ID NO: 6 and 26; CDR2 selected from the group consisting of SEQ ID NO: 7 and 27; CDR3 selected from the group consisting of 8 and 28; a light chain variable region CDR1 selected from the group consisting of SEQ ID NO: 16 and 36; CDR2 selected from the group consisting of SEQ ID NO: 17 and 37; and CDR3 selected from the group consisting of SEQ ID NO: 18 and 38.

22. The method of any one of clauses 1-18, wherein the antibody or fragment comprises (i) a heavy chain variable region CDR1 of SEQ ID NO: 3; a heavy chain variable region CDR2 of SEQ ID NO: 4; a heavy chain variable region CDR3 of SEQ ID NO: 5; a light chain variable region CDR1 of SEQ ID NO: 13; a light chain variable region CDR2 of SEQ ID NO: 14; and a light chain variable region CDR3 of SEQ ID NO: 15;

(ii) a heavy chain variable region CDR1 of SEQ ID NO: 23; a heavy chain variable region CDR2 of SEQ ID NO: 24; a heavy chain variable region CDR3 of SEQ ID NO: 25; a light chain variable region CDR1 of SEQ ID NO: 33; a light chain variable region CDR2 of SEQ ID NO: 34; and a light chain variable region CDR3 of SEQ ID NO: 35;

(iii) a heavy chain variable region CDR1 of SEQ ID NO: 6; a heavy chain variable region CDR2 of SEQ ID NO: 7; a heavy chain variable region CDR3 of SEQ ID NO: 8; a light chain variable region CDR1 of SEQ ID NO: 16; a light chain variable region CDR2 of SEQ ID NO: 17; and a light chain variable region CDR3 of SEQ ID NO: 18; or

(iv) a heavy chain variable region CDR1 of SEQ ID NO: 26; a heavy chain variable region CDR2 of SEQ ID NO: 27; a heavy chain variable region CDR3 of SEQ ID NO: 28; a light chain variable region CDR1 of SEQ ID NO: 36; a light chain variable region CDR2 of SEQ ID NO: 37; and a light chain variable region CDR3 of SEQ ID NO: 38.

23. The method of any one of clauses 1-22, wherein the antibody or fragment binds to the same epitope as an antibody comprising a variable heavy chain of SEQ ID NO: 9 and a variable light chain sequence of SEQ ID NO: 19 or an antibody comprising a variable heavy chain of SEQ ID NO: 29 and a variable light chain sequence of SEQ ID NO: 39.

24. The method of any one of clauses 1-22, wherein the antibody or fragment competes for binding to a human FXI and/or FXIa protein with a reference antibody comprising a variable heavy chain of SEQ ID NO: 9 and a variable light chain sequence of SEQ ID NO: 19 or a reference antibody comprising a variable heavy chain of SEQ ID NO: 29 and a variable light chain sequence of SEQ ID NO: 39; and wherein the antibody or fragment contacts one, two, three, four, five, six, seven, eight, nine, or ten, or more residues selected from the following: Pro410, Arg413, Leu415, Cys416, His431, Cys432, Tyr434, Gly435, Glu437, Tyr472, Lys473, Met474, Ala475, Glu476, Tyr521, Arg522, Lys523, Leu524, Arg525, Asp526, Lys527, Arg548, His552, Ser575, Ser594, Trp595, Gly596, Glu597, Arg602, Glu603, and Arg604.

25. The method of any one of clauses 1-24, wherein the antibody or fragment is selected from the group consisting of NOV1090, NOV1401, or an antibody comprising the 3 VH CDRs of SEQ ID NO: 29 and 3 VL CDRs of SEQ ID NO: 39 of NOV1401.

26. A method of managing or reducing bleeding or bleeding risk in a subject afflicted with end stage renal disease who has been treated or administered an anti-FXI/FXIa antibody or antigen-biniding fragment that binds within the catalytic domain of FXI and/or FXIa, comprising the step of administering to the subject in need thereof, an anti-idiotype antibody or fragment thereof that specifically binds to the anti-FXI/FXIa antibody and blocks the anti-FXI/FXIa antibody from binding to FXI and/or FXIa, and wherein the anti-idiotype antibody or fragment thereof reverses the anticoagulant activity of the anti-FXI/FXIa antibody.

27. The method of clause 26, wherein the anti-idiotype antibody or fragment thereof is administered to the subject once or a few times (e.g. twice, three times or four times) to reverse the anticoagulant effect of the anti-FXI/FXIa antibody.

28. A method of managing or reducing bleeding or bleeding risk in a subject afflicted with end stage renal disease who has been treated or administered an anti-FXI/FXIa antibody or antigen-biniding fragment that binds within the catalytic domain of FXI and/or FXIa, said method comprises temporarily reversing of the anticoagulant effect for a sufficient time to manage the bleeding by one of the following: (i) fluid replacement using colloids, crystalloids, human plasma or plasma proteins such as albumin; (ii) transfusion with packed red blood or whole blood; or (iii) administration of fresh frozen plasma (FFP), prothrombin complex concentrates (PCC), activated PCC (APCC), such as, factor VIII inhibitor, and/or recombinant activated factor VII.

29. The method of any one of clauses 26-28, wherein the subject is receiving dialysis, such as hemodialysis, or extracorporeal membrane oxygenation.

30. The method of any one of clauses 26-28, wherein the subject is afflicted with atrial fibrillation.

31. The method of clause 30, wherein the subject is afflicted with non-valvular atrial fibrillation.

32. The method of clause 30 or 31, wherein the subject has a demonstrated high risk of bleeding.

33. The method of any one of clauses 26-32, wherein the anti-FXI/FXIa antibody or antigen-biniding fragment comprises a VH selected from the group consisting of SEQ ID NO: 9 and 29 or an amino acid sequence with at least 90% identity thereof; and a VL selected from the group consisting of SEQ ID NO: 19 and 39 or an amino acid sequence with at least 90% identity thereof.

34. The method of any one of clauses 26-32, wherein the anti-FXI/FXIa antibody or antigen-biniding fragment competes, in a concentration-dependent manner, for binding to a human FXI and/or FXIa protein with an antibody comprising a variable heavy chain of SEQ ID NO: 9 and a variable light chain sequence of SEQ ID NO: 19 or an antibody comprising a variable heavy chain of SEQ ID NO: 29 and a variable light chain sequence of SEQ ID NO: 39.

35. The method of any one of clauses 1-34, wherein the anti-FXI/FXIa antibody comprises a heavy chain comprising the amino acid sequence of SEQ ID NO: 31 and a light chain comprising the amino acid sequence of SEQ ID NO: 41.

36. The method of any one of clauses 1-35 wherein the anti-FXI/FXIa antibody or antigen-binding fragment thereof is administered to the subject subcutaneously.

37. An antibody or antigen-binding fragment thereof that binds within the catalytic domain of FXI and/or FXIa, or a pharmaceutical composition comprising the antibody or antigen-binding fragment thereof and a pharmaceutically acceptable carrier, for use in preventing, treating or managing or reducing the risk of stroke or thromboembolism in a subject afflicated with end stage renal disease, wherein the antibody comprises a heavy chain comprising the amino acid sequence of SEQ ID NO: 31 and a light chain comprising the amino acid sequence of SEQ ID NO: 41.

38. The antibody or antigen-binding fragment thereof or a pharmaceutical composition according to clause 37, wherein the subject is receiving dialysis.

39. The antibody or antigen-binding fragment thereof or a pharmaceutical composition according to clause 37 or 38, wherein the subject is receiving hemodialysis or other types of dialysis, such as, peritoneal dialysis.

40. The antibody or antigen-binding fragment thereof or a pharmaceutical composition according to any one of clauses 37-39, wherein the subject is afflicted with atrial fibrillation, such as non-valvular atrial fibrillation.

41. An antibody or antigen-binding fragment thereof that binds within the catalytic domain of FXI and/or FXIa, or a pharmaceutical composition comprising the antibody or antigen-binding fragment thereof and a pharmaceutically acceptable carrier, for use in preventing or reducing the risk of stroke or thromboembolism in a subject afflicated with atrial fibrillation and end stage renal disease and who is undergoing dialysis, such as hemodialysis, wherein the antibody comprises a heavy chain comprising the amino acid sequence of SEQ ID NO: 31 and a light chain comprising the amino acid sequence of SEQ ID NO: 41.

42. The antibody or antigen-binding fragment thereof or a pharmaceutical composition according to any one of clauses 37-41, wherein the subject is afflicted with non-valvular atrial fibrillation.

43. An antibody or antigen-binding fragment thereof that binds within the catalytic domain of FXI and/or FXIa, or a pharmaceutical composition comprising the antibody or antigen-binding fragment thereof, for use in preventing, treating or managing or reducing the risk of catheter-related thrombosis in a subject afflicated with end stage renal disease and initiated on hemodialysis with a catheter, wherein the antibody comprises a heavy chain comprising the amino acid sequence of SEQ ID NO: 31 and a light chain comprising the amino acid sequence of SEQ ID NO: 41.

44. The antibody or antigen-binding fragment thereof or a pharmaceutical composition according to any one of clauses 37-43, wherein the subject has a demonstrated high risk of bleeding.

45. An antibody or antigen-binding fragment thereof that binds within the catalytic domain of FXI and/or FXIa, or a pharmaceutical composition comprising the antibody or antigen-binding fragment thereof and a pharmaceutically acceptable carrier, for use in preventing or reducing the risk of stroke or thromboembolism, such as systemic embolism, in a subject afflicated with atrial fibrillation, wherein the subject has a demonstrated high risk of bleeding which is characterized by a $CHA_2DS_2VASc$ risk score $\geq 2$, and wherein the antibody comprises a heavy chain comprising the amino acid sequence of SEQ ID NO: 31 and a light chain comprising the amino acid sequence of SEQ ID NO: 41.

46. The antibody or antigen-binding fragment thereof or a pharmaceutical composition according to any one of clauses 37-45, wherein the subject is at least 55 years old.

47. The antibody or antigen-binding fragment thereof or a pharmaceutical composition according to any one of clauses 37-46, wherein the subject has a demonstrated high risk of bleeding characterized as having (i) a $CHA_2DS_2VASc$ risk score $\geq 2$ and (ii) one, two or more of the following risk factors:

(a) over 65 years old;

(b) history of previous stroke or transient ischemic attack;

(c) previous major bleed or clinically relevant bleed;

(d) chronic kidney disease (CKD) stage 3 to 4;

(e) treatment with single or dual antiplatelet therapy; and

(f) history of falls associated with hematoma bruise or other manifestations.

48. The antibody or antigen-binding fragment thereof or a pharmaceutical composition according to any one of clauses 37-47, wherein the antibody or antigen-binding fragment thereof is administered to the subject subcutaneously.

49. The antibody or antigen-binding fragment thereof or a pharmaceutical composition according to any one of clauses 37-48, wherein the antibody or antigen-binding fragment thereof is administered at a dose of at least 90 mg, 120 mg, 150 mg, 180 mg, or 210 mg.

50. The antibody or antigen-binding fragment thereof or a pharmaceutical composition according to any one of clauses 37-48, wherein the antibody or antigen-binding fragment thereof is administered at a dose of 120 mg, 150 mg, or 180 mg.

51. The antibody or antigen-binding fragment thereof or a pharmaceutical composition according to any one of clauses 37-50, wherein the antibody or antigen-binding fragment thereof is administered once a month.

52. Use of antibody or antigen-binding fragment thereof that binds within the catalytic domain of FXI and/or FXIa for the manufacture of a medicament for preventing, treating or managing or reducing the risk of stroke or thromboembolism in a subject afflicated with end stage renal disease, wherein the antibody comprises a heavy chain comprising the amino acid sequence of SEQ ID NO: 31 and a light chain comprising the amino acid sequence of SEQ ID NO: 41.

53. The use according to clause 52, wherein the subject is receiving dialysis.

54. The use according to clause 52 or 53, wherein the subject is receiving hemodialysis or other types of dialysis, such as, peritoneal dialysis.

55. The use according to any one of clauses 52-54, wherein the subject is afflicted with atrial fibrillation, such as non-valvular atrial fibrillation.

56. Use of an antibody or antigen-binding fragment thereof that binds within the catalytic domain of FXI and/or FXIa for the manufacture of a medicament for preventing or reducing the risk of stroke or thromboembolism in a subject afflicated with atrial fibrillation and end stage renal disease and who is undergoing dialysis, such as hemodialysis, wherein the antibody comprises a heavy chain comprising the amino acid sequence of SEQ ID NO: 31 and a light chain comprising the amino acid sequence of SEQ ID NO: 41.

57. The use according to any one of clauses 52-56, wherein the subject is afflicted with non-valvular atrial fibrillation.

58. Use of an antibody or antigen-binding fragment thereof that binds within the catalytic domain of FXI and/or FXIa for the manufacture of a medicament for preventing, treating or managing or reducing the risk of catheter-related thrombosis in a subject afflicated with end stage renal disease and initiated on hemodialysis with a catheter, wherein the antibody comprises a heavy chain comprising the amino acid sequence of SEQ ID NO: 31 and a light chain comprising the amino acid sequence of SEQ ID NO: 41.

59. The use according to any one of clauses 52-58, wherein the subject has a demonstrated high risk of bleeding.

60. Use of an antibody or antigen-binding fragment thereof that binds within the catalytic domain of FXI and/or FXIa for the manufacture of a medicament for preventing or reducing the risk of stroke or thromboembolism, such as systemic embolism, in a subject afflicated with atrial fibrillation, wherein the subject has a demonstrated high risk of bleeding which is characterized by a $CHA_2DS_2VASc$ risk score $\geq 2$, and wherein the antibody comprises a heavy chain comprising the amino acid sequence of SEQ ID NO: 31 and a light chain comprising the amino acid sequence of SEQ ID NO: 41.

61. The use according to any one of clauses 52-60, wherein the subject is at least 55 years old.

62. The use according to any one of clauses 52-60, wherein the subject has a demonstrated high risk of bleeding characterized as having (i) a $CHA_2DS_2VASc$ risk score $\geq 2$ and (ii) one, two or more of the following risk factors:

(a) over 65 years old;

(b) history of previous stroke or transient ischemic attack;

(c) previous major bleed or clinically relevant bleed;

(d) chronic kidney disease (CKD) stage 3 to 4;

(e) treatment with single or dual antiplatelet therapy; and

(f) history of falls associated with hematoma bruise or other manifestations.

63. The use according to any one of clauses 52-62, wherein the the medicament is suitable for administration to the subject subcutaneously.

64. The use according to any one of clauses 52-63, wherein the medicament is suitable for administration to the subject at a dose of at least 90 mg, 120 mg, 150 mg, 180 mg, or 210 mg.

65. The use according to any one of clauses 52-63, wherein the medicament is suitable for administration to the subject at a dose of 120 mg, 150 mg, or 180 mg.

66. The use according to any one of clauses 52-65, wherein the medicament is suitable for administration to the subject once a month.

## Claims

1. An antibody or antigen-binding fragment thereof that binds FXI and FXIa, or a pharmaceutical composition comprising the antibody or antigen-binding fragment thereof and a pharmaceutically acceptable carrier, for use in preventing, treating or managing, or reducing the risk of stroke or thromboembolism in a subject afflicted with end stage renal disease, wherein the antibody comprises:

i. a heavy chain variable region CDR1 of SEQ ID NO: 23; a heavy chain variable region CDR2 of SEQ ID NO: 24; a heavy chain variable region CDR3 of SEQ ID NO: 25; a light chain variable region CDR1 of SEQ ID NO: 33; a light chain variable region CDR2 of SEQ ID NO: 34; and a light chain variable region CDR3 of SEQ ID NO: 35;

ii. a heavy chain variable region CDR1 of SEQ ID NO: 26; a heavy chain variable region CDR2 of SEQ ID NO: 27; a heavy chain variable region CDR3 of SEQ ID NO: 28; a light chain variable region CDR1 of SEQ ID NO: 36; a light chain variable region CDR2 of SEQ ID NO: 37; and a light chain variable region CDR3 of SEQ ID NO: 38;

iii. a heavy chain variable region CDR1 of SEQ ID NO: 43; a heavy chain variable region CDR2 of SEQ ID NO: 44; a heavy chain variable region CDR3 of SEQ ID NO: 45; a light chain variable region CDR1 of SEQ ID NO: 47; a light chain variable region CDR2 of SEQ ID NO: 37; and a light chain variable region CDR3 of SEQ ID NO: 15; or

iv. a heavy chain variable region CDR1 of SEQ ID NO: 46; a heavy chain variable region CDR2 of SEQ ID NO: 4; a heavy chain variable region CDR3 of SEQ ID NO: 5; a light chain variable region CDR1 of SEQ ID NO: 33; a light chain variable region CDR2 of SEQ ID NO: 14; and a light chain variable region CDR3 of SEQ ID NO: 15.

2. The antibody or antigen-binding fragment thereof or the pharmaceutical composition for use according to claim 1, wherein the subject is receiving dialysis or hemodialysis.

3. The antibody or antigen-binding fragment thereof or the pharmaceutical composition for use according to claim 2, wherein the subject is receiving peritoneal dialysis.

4. The antibody or antigen-binding fragment thereof or the pharmaceutical composition for use according to any one of claims 1 to 3, wherein the subject is afflicted with atrial fibrillation.

5. The antibody or antigen-binding fragment thereof or the pharmaceutical composition for use according to any one of claims 1 to 4, wherein the subject is afflicted with non-valvular atrial fibrillation.

6. The antibody or antigen-binding fragment thereof or the pharmaceutical composition for use according to any one of claims 1 to 5, wherein the subject is obese.

7. The antibody or antigen-binding fragment thereof or the pharmaceutical composition for use according to any one of claims 1 to 5, wherein the subject is afflicted with heart failure.

8. The antibody or antigen-binding fragment thereof or the pharmaceutical composition for use according to any one of claims 1 to 5, wherein the subject has a history of previous stroke or transient ischemic attack.

9. The antibody or antigen-binding fragment thereof or the pharmaceutical composition for use according to any one of claims 1 to 5, wherein the subject has a left ventricular thrombus.

10. The antibody or antigen-binding fragment thereof or the pharmaceutical composition for use according to any one of claims 1 to 5, wherein the stroke or thromboembolism is a catheter-related thrombosis.

11. The antibody or antigen-binding fragment thereof or the pharmaceutical composition for use according to any one of claims 1 to 2 or 4 to 10, wherein the subject is receiving hemodialysis with a catheter.

12. The antibody or antigen-binding fragment thereof or the pharmaceutical composition for use according to any one of claims 1 to 2 or 4 to 10, wherein the subject is receiving hemodialysis with an arteriovenous fistula or an arteriovenous graft.

13. The antibody or antigen-binding fragment thereof or the pharmaceutical composition for use according to any one of claims 1 to 12, wherein the subject has a demonstrated high risk of bleeding.

14. The antibody or antigen-binding fragment thereof or the pharmaceutical composition for use according to any one of claims 1 to 13, wherein the subject has a demonstrated high risk of bleeding characterized as having (i) a $CHA_2DS_2$-VASc risk score > 2 and (ii) one, two or more of the following risk factors:

(a) over 65 years old;
(b) history of previous stroke or transient ischemic attack;
(c) previous major bleed or clinically relevant bleed;
(d) chronic kidney disease (CKD) stage 3 to 4;
(e) treatment with single or dual antiplatelet therapy; and

(f) history of falls associated with hematoma, bruise or other manifestations.

15. The antibody or antigen-binding fragment thereof or the pharmaceutical composition for use according to any one of claims 1 to 13, wherein the subject has a HAS-BLED bleeding risk score of at least 3.

16. The antibody or antigen-binding fragment thereof or the pharmaceutical composition for use according to any one of claims 1 to 13, wherein the subject has a history of previous major bleed or clinically relevant bleed.

17. The antibody or antigen-binding fragment thereof or the pharmaceutical composition for use according to any one of claims 1 to 13, wherein the subject is over 65 years old.

18. The antibody or antigen-binding fragment thereof or the pharmaceutical composition for use according to any one of claims 1 to 17, wherein the antibody or antigen-binding fragment thereof is administered to the subject subcutaneously.

19. The antibody or antigen-binding fragment thereof or the pharmaceutical composition for use according to any one of claims 1 to 17, wherein the antibody or antigen-binding fragment thereof is administered to the subject intravenously.

20. The antibody or antigen-binding fragment thereof or the pharmaceutical composition for use according to any one of claims 1 to 19, wherein the antibody or antigen-binding fragment thereof is administered at a dose of at least 90 mg, 120 mg, 150 mg, 180 mg, or 210 mg.

21. The antibody or antigen-binding fragment thereof or the pharmaceutical composition for use according to any one of claims 1 to 20, wherein the antibody or antigen-binding fragment thereof is administered once a month.

22. The antibody or antigen-binding fragment thereof or the pharmaceutical composition for use according to any one of claims 1 to 21, wherein the isolated anti-FXI and/or anti-FXIa antibody or antigen-binding fragment thereof comprises a VH with at least 90% identity to SEQ ID NO: 29 and a VL with at least 90% identity to SEQ ID NO: 39.

23. The antibody or antigen-binding fragment thereof or the pharmaceutical composition for use according to any one of claims 1 to 22, wherein the isolated anti-FXI and/or anti-FXIa antibody or antigen-binding fragment thereof comprises a heavy chain of SEQ ID NO: 31 or a heavy chain with at least 90% identity thereto, and a light chain of SEQ ID NO: 41 or a light chain with at least 90% identity thereto.

**FIG 1A**

**FIG 1B**

**FIG 1C**

FIG 2A

FIG 2B

FIG 3A

FIG 3B

FIG 4A

FIG 4B

FIG 4C

FIG 5A

FIG 5B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62438654
- US 62589851
- US 6703199 B
- US 5641870 A
- US 5766886 A
- WO IB2016053790 A
- WO 2016207858 A
- US 5225539 A
- US 5530101 A
- US 5585089 A
- US 5693762 A
- US 6180370 B, Queen
- EP 0154316 A, Nishimura
- EP 0401384 A, Ishikawa
- WO 9823289 A
- WO 9734631 A
- US 6277375 B
- WO 9315199 A
- WO 9315200 A
- WO 0177137 A
- EP 413622 A
- US 5336603 A
- US 5622929 A
- US 5359046 A
- US 5349053 A
- US 5447851 A
- US 5112946 A
- EP 307434 A
- EP 367166 A
- WO 9604388 A
- WO 9106570 A
- US 5605793 A
- US 5811238 A
- US 5830721 A
- US 5834252 A
- US 5837458 A
- US 4458066 A

### Non-patent literature cited in the description

- **WEITZ J.I.** *Thromb. Haemost.*, 2010, vol. 103, 62
- **LASSEN, M.R. et al.** *N. Engl. J. Med.*, 2009, vol. 361, 594
- **CONNOLLY, S.J. et al.** *N. Engl. J. Med.*, 2009, vol. 361, 1139
- **KANNEL ; BENJAMIN.** *Med Clin North Am.*, 2008, vol. 92, 17-40
- **BUNCH et al.** *J Innovations of Card Rhythm Manag*, 2012, vol. 3, 855-63
- **MARZONA et al.** *CMAJ*, 2012, vol. 184, 329-36
- **CAMM et al.** *Eur Heart J*, 2012, vol. 33, 2719-2747
- **CONNOLLY et al.** *N Engl J Med*, 2009, vol. 361, 1139-51
- **CONNOLLY et al.** *N Engl J Med*, 2011, vol. 364, 806-17
- **PATEL et al.** *N Engl J Med*, 2011, vol. 365, 883-91
- **GALLEGO et al.** *Carc Arrhythm Electrophysiol*, 2012, vol. 5, 312-318
- **PICCINI et al.** *Eur Heart J*, 2014, vol. 35, 1873-80
- **NIEUWLAAT et al.** *Eur Heart J*, 2005, vol. 26, 2422-2434
- **ZIMMERMAN et al.** *Nephrol Dial Transplant*, 2012, vol. 27, 3816-3822
- **MANDLE RJ JR et al.** *Blood*, 1979, vol. 54 (4), 850
- **BANE ; GAILANI.** *Drug Disc*, 2014, vol. 19 (9)
- **WARD et al.** *Nature*, 1989, vol. 341, 544-546
- **BIRD et al.** *Science*, 1988, vol. 242, 423-426
- **HUSTON et al.** *Proc. Natl. Acad. Sci.*, 1988, vol. 85, 5879-5883
- **HOLLINGER ; HUDSON.** *Nature Biotechnology*, 2005, vol. 23 (9), 1126-1136
- **ZAPATA et al.** *Protein Eng.*, 1995, vol. 8 (10), 1057-1062
- **HOFFMAN M.M. ; MONROE D.M.** *Curr Hematol Rep.*, 2005, vol. 4, 391-396
- **JOHNE J et al.** *Biol Chem.*, 2006, vol. 387, 173-178
- **CREIGHTON.** *Proteins*, 1984
- **MORRISON et al.** *Proc. Natl. Acad. Sci. USA*, 1984, vol. 81, 6851-6855
- **MORRISON ; OI.** *Adv. Immunol.*, 1988, vol. 44, 65-92
- **VERHOEYEN et al.** *Science*, 1988, vol. 239, 1534-1536
- **PADLAN.** *Molec. Immun.*, 1991, vol. 28, 489-498

- **PADLAN**. *Molec. Immun.*, 1994, vol. 31, 169-217
- **SMITH** ; **WATERMAN**. *Adv. Appl. Math*, 1970, vol. 2, 482c
- **NEEDLEMAN** ; **WUNSCH**. *J. Mol. Biol.*, 1970, vol. 48, 443
- **PEARSON** ; **LIPMAN**. *Proc. Nat'l. Acad. Sci. USA*, 1988, vol. 85, 2444
- **BRENT et al.** Current Protocols in Molecular Biology. John Wiley & Sons, Inc., 2003
- **ALTSCHUL et al.** *Nuc. Acids Res.*, 1977, vol. 25, 3389-3402
- **ALTSCHUL et al.** *J. Mol. Biol.*, 1990, vol. 215, 403-410
- **HENIKOFF** ; **HENIKOFF**. *Proc. Natl. Acad. Sci. USA*, 1989, vol. 89, 10915
- **KARLIN** ; **ALTSCHUL**. *Proc. Natl. Acad. Sci. USA*, 1993, vol. 90, 5873-5787
- **E. MEYERS** ; **W. MILLER**. *Comput. Appl. Biosci.*, 1988, vol. 4, 11-17
- **NEEDLEMAN** ; **WUNSCH**. *J. Mol, Biol.*, 1970, vol. 48, 444-453
- **BATZER et al.** *Nucleic Acid Res.*, 1991, vol. 19, 5081
- **OHTSUKA et al.** *J. Biol. Chem.*, 1985, vol. 260, 2605-2608
- **ROSSOLINI et al.** *Mol. Cell. Probes*, 1994, vol. 8, 91-98
- **SALOMON et al.** *Thromb Haemost.*, 2011, vol. 105, 269-73
- **GAILANI** ; **RENNÉ**. *Arterioscler Thromb Vasc Biol.*, 2007, vol. 27 (12), 2507-13
- **WANG et al.** *J Thromb Haemost*, 2006, vol. 4, 1982-8
- **WANG et al.** *J Thromb Haemost;*, 2005, vol. 3, 695-702
- **CHENG et al.** *Blood*, 2010, vol. 116, 3981-9
- **LEUNG et al.** *Transl Stroke Res*, 2012, vol. 3, 381-9
- **TUCKER et al.** *Blood*, 2009, vol. 113, 936-44
- **ZHANG et al.** *Blood*, 2010, vol. 116, 4684-92
- **YOUNIS et al.** *Blood*, 2012, vol. 119, 2401-8
- **CROSBY et al.** *Arterioscler Thromb Vasc Biol*, 2013, vol. 33, 1670-8
- **SCHUMACHER et al.** *Eur J Pharmacol*, 2007, vol. 570, 167-74
- **WONG et al.** *J Thromb Thrombolysis*, 2011, vol. 32, 129-37
- **SALOMON et al.** *Blood*, 2008, vol. 111, 4113-17
- **MEIJERS et al.** *N Engl J Med.*, 2000, vol. 342, 696-701
- **BÜLLER et al.** *N Engl J Med.*, 07 December 2014, vol. 372 (3), 232-40
- Sequences of Proteins of Immunological Interest. **KABAT et al.** 5th Ed. Public Health Service. National Institutes of Health, 1991
- **AL-LAZIKANI et al.** *JMB*, 1997, vol. 273, 927-948
- **LEFRANC et al.** *Dev. Comp. Immunol.*, 2003, vol. 27, 55-77
- **ALTSCHUL et al.** *J. Mol. Biol.*, 1990, vol. 215, 403-10
- **RIECHMANN, L. et al.** *Nature*, 1998, vol. 332, 323-327
- **JONES, P. et al.** *Nature*, 1986, vol. 321, 522-525
- **QUEEN, C. et al.** *Proc. Natl. Acad., U.S.A.*, 1989, vol. 86, 10029-10033
- Sequences of Proteins of Immunological Interest. **KABAT, E. A. et al.** U.S. Department of Health and Human Services. NIH Publication, 1991
- **TOMLINSON, I. M. et al.** *J. Mol. Biol.*, 1992, vol. 227, 776-798
- **COX, J. P. L.** ; **1994 et al.** *Eur. J Immunol.*, vol. 24, 827-836
- **ASHKENAZI et al.** *Proc. Natl. Acad. Sci. USA*, 1991, vol. 88, 10535-10539
- **ZHENG et al.** *J. Immunol.*, 1995, vol. 154, 5590-5600
- **VIL et al.** *Proc. Natl. Acad. Sci. USA*, 1992, vol. 89, 11337-11341
- **PATTEN et al.** *Curr. Opinion Biotechnol.*, 1997, vol. 8, 724-33
- **HARAYAMA**. *Trends Biotechnol.*, 1998, vol. 16 (2), 76-82
- **HANSSON et al.** *J. Mol. Biol.*, 1999, vol. 287, 265-76
- **LORENZO** ; **BLASCO**. *Biotechniques*, 1998, vol. 24 (2), 308-313
- **GENTZ et al.** *Proc. Natl. Acad. Sci. USA*, 1989, vol. 86, 821-824
- **WILSON et al.** *Cell*, 1984, vol. 37, 767
- **DENARDO et al.** *Clin Cancer Res.*, 1998, vol. 4 (10), 2483-90
- **PETERSON et al.** *Bioconjug. Chem.*, 1999, vol. 10 (4), 553-7
- **ZIMMERMAN et al.** *Nucl. Med. Biol.*, 1999, vol. 26 (8), 943-50
- Monoclonal Antibodies For Immunotargeting Of Drugs In Cancer Therapy. **ARNON et al.** Monoclonal Antibodies And Cancer Therapy. Alan R. Liss, Inc., 1985, 243-56
- **HELLSTROM et al.** Antibodies For Drug Delivery'', in Controlled Drug Delivery. Marcel Dekker, Inc, 1987, 623-53
- Antibody Carriers Of Cytotoxic Agents In Cancer Therapy: A Review. **THORPE et al.** Monoclonal Antibodies 84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16

- **THORPE et al.** *Immunol. Rev.*, 1982, vol. 62, 119-58
- **NARANG et al.** *Meth. Enzymol.*, 1979, vol. 68, 90
- **BROWN et al.** *Meth. Enzymol.*, 1979, vol. 68, 109
- **BEAUCAGE et al.** *Tetra. Lett.*, 1981, vol. 22, 1859
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.*, 1991, vol. 19, 967
- **ECKERT et al.** *PCR Methods and Applications*, 1991, vol. 1, 17
- **HARRINGTON et al.** *Nat Genet*, 1997, vol. 15, 345
- **SMITH**. *Annu. Rev. Microbiol.*, 1995, vol. 49, 807
- **ROSENFELD et al.** *Cell*, 1992, vol. 68, 143
- **WINNACKER**. FROM GENES TO CLONES. VCH Publishers, 1987
- **QUEEN et al.** *Immunol. Rev*, 1986, vol. 89, 49-68
- **ELLIOT** ; **O'HARE**. *Cell*, 1997, vol. 88, 223

- **FRIBERG et al.** *Circulation*, 2012, vol. 125, 2298-2307
- **JANUARY et al.** AHA/ACC/HRS Atrial Fibrillation Guideline. *J Am Coll Cardiol*, 2014, vol. 64, 2246-80
- **RIDDELL et al.** *Thromb. Haemost.*, 2011, vol. 106, 521-527
- **LIVNAT et al.** *Thromb. Haemost*, 2009, vol. 102, 487-492
- **PAN et al.** *FASEB J.*, 1995, vol. 9, 43-49
- Remington: The Science and Practice of Pharmacy. Mack Publishing Co, 2000
- Sustained and Controlled Release Drug Delivery Systems. Marcel Dekker, Inc., 1978
- **PIEHLER et al.** Assessment of affinity constants by rapid solid phase detection of equilibrium binding in a flow system. *J. Immunol. Meth.*, 1997, 189-206
- **CHENG et al.** *Blood*, 2010, vol. 116, 3981-3989
- **LIU et al.** ISIS-FXIRx, a novel and specific antisense inhibitor of factor XI, caused significant reduction in FXI antigen and activity and increased aPTT without causing bleeding in healthy volunteers. *Presented at the 53rd American Society of Hematology annual meeting and exposition, San Diego, California. Blood*, 2011, vol. 118, 209